# EUROPEAN PATENT SPECIFICATION

(11) **EP 2 947 071 B1**
(45) Date of publication and mention of the grant of the patent: **03.04.2019**
(21) Application number: 15153639.8
(22) Date of filing: 03.02.2015
(51) Int. Cl.: C09K 11/02, C09K 11/06, H01L 51/00, H01L 51/50, C07D 239/70, C07D 209/82

(54) **Organic compound and composition and organic optoelectric device and display device**
Organische Verbindung und Zusammensetzung und organische optoelektrische Vorrichtung und Anzeigevorrichtung
Composé et composition organique et dispositif optoélectronique organique et dispositif d'affichage

(30) Priority: 20.05.2014 KR 20140060527
(43) Date of publication of application: 25.11.2015
(73) Proprietor: Samsung SDI Co., Ltd., Gyeonggi-do 446-902 (KR)
(72) Inventor: Lee, Han-Ill, 443-803 Gyeonggi-do (KR); Shin, Chang-Ju, 443-803 Gyeonggi-do (KR); Han, Su-Jin, 443-803 Gyeonggi-do (KR); Kim, Young-Kwon, 443-803 Gyeonggi-do (KR); Min, Soo-Hyun, 443-803 Gyeonggi-do (KR); Yu, Eun-Sun, 443-803 Gyeonggi-do (KR); Jung, Ho-Kuk, 443-803 Gyeonggi-do (KR)
(74) Representative: Michalski Hüttermann & Partner Patentanwälte mbB

(56) References cited:
- WO-A1-2013/180376
- KR-A- 20120 117 693
- KR-A- 20140 004 005
- HERRERA A ET AL: "The reaction of tetralones with nitriles: a simple approach to the synthesis of new substituted benzo[h]quinazolines, benzo[f]quinazolines and dibenzo[a,i]phenanthridines", TETRAHEDRON, ELSEVIER SCIENCE PUBLISHERS, AMSTERDAM, NL, vol. 62, no. 12, 20 March 2006 (2006-03-20), pages 2799-2811, XP025001688, ISSN: 0040-4020, DOI: 10.1016/J.TET.2006.01.010 [retrieved on 2006-03-20]
- DATABASE CA [Online] CHEMICAL ABSTRACTS SERVICE, COLUMBUS, OHIO, US; 1 February 1984 (1984-02-01), ROBEV, S.: "2,4-Disubstituted benzo[h]quinazolines from N-(1-naphthyl)-amidines", XP002738377, retrieved from STN Database accession no. 1984:510858
- DATABASE CA [Online] CHEMICAL ABSTRACTS SERVICE, COLUMBUS, OHIO, US; 1 February 1989 (1989-02-01), ROBEV, S.: "Synthesis of dihydrobenzoquinazolines", XP002738378, retrieved from STN Database accession no. 1989:574053

## Description

### BACKGROUND OF THE INVENTION

### (a) Field of the Invention

An organic compound, composition, an organic optoelectric device and a display device are disclosed.

### (b) Description of the Related Art

An organic optoelectric device is a device that converts electrical energy into photoenergy, and vice versa.

An organic optoelectric device may be classified as follows in accordance with its driving principles. One is an electronic device where excitons generated by photoenergy are separated into electrons and holes and the electrons and holes are transferred to different electrodes respectively and electrical energy is generated, and the other is a light emitting device to generate photoenergy from electrical energy by supplying a voltage or a current to electrodes.

Examples of the organic optoelectric device include an organic photoelectric device, an organic light emitting diode, an organic solar cell, and an organic photo-conductor drum, and the like.

Among them, the organic light emitting diode (OLED) has recently drawn attention due to an increase in demand for flat panel displays. The organic light emitting diode converts electrical energy into light by applying current to an organic light emitting material, and has a structure in which an organic is interposed between an anode and a cathode.

Performance of an organic light emitting diode may be affected by characteristics of the organic layer, and among them, may be mainly affected by characteristics of an organic material of the organic layer. Particularly, development for an organic material being capable of increasing hole and electron mobility and simultaneously increasing electrochemical stability is needed so that the organic light emitting diode may be applied to a large-size flat panel display.

### SUMMARY OF THE INVENTION

One embodiment provides an organic compound being capable of realizing an organic optoelectric device having high efficiency and long life-span.

Another embodiment provides a composition for an organic optoelectric device including the organic compound.

Yet another embodiment provides an organic optoelectric device including the organic compound or the composition for an organic optoelectric device.

Compounds for organic optoelectric devices are inter alia taught in the KR 2012 0117 692, KR 2014 0004005, WO 2013/180376, in Herrera et al., Tetrahedron 2006, 2799-2811, in Robev et al, Doklady Bolgarskoi Akademii Nauk, 1988, 71-74.

Still another embodiment provides a display device including the organic optoelectric device.

According to one embodiment, an organic compound represented by the following Chemical Formula 1 is provided. 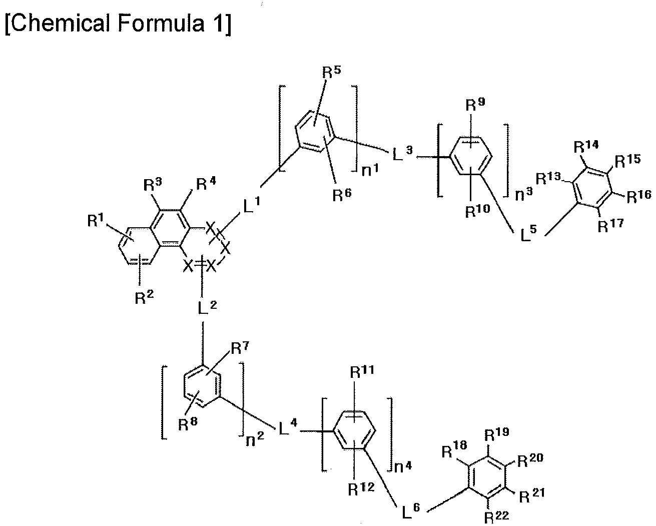 In the Chemical Formula 1,
two of X's are N and two of X's are C,
R¹ to R⁴ are independently hydrogen, deuterium, unsubstituted C1 to C10 alkyl group, unsubstituted C6 to C12 aryl group, unsubstituted C3 to C12 heterocycle, or a combination thereof,
R⁵ to R¹² are independently hydrogen, deuterium, unsubstituted C1 to C10 alkyl group, unsubstituted C6 to C12 aryl group, or a combination thereof,
R¹³ to R²² are independently hydrogen, deuterium, unsubstituted C1 to C10 alkyl group, unsubstituted C6 to C12 aryl group, unsubstituted C3 to C12 heterocycle, or a combination thereof,
R¹³ to R²² are independently present or adjacent two are linked to each other to form a fused ring,
L¹ to L⁶ are independently a single bond, unsubstituted phenylene group, unsubstituted biphenylene group, unsubstituted terphenylene group or unsubstituted quaterphenylene group,
n¹ to n⁴ are independently integers ranging from 0 to 5, and
the sum of n¹ to n⁴ is an integer of 2 or more, preferably 6 or less.

According to another embodiment, a composition for an organic optoelectric device includes a first organic compound and at least one second organic compound having a carbazole moiety.

According to yet another embodiment, an organic optoelectric device includes an anode and a cathode facing each other and at least one organic layer interposed between the anode and the cathode, wherein the organic layer includes the organic compound or the composition for an organic optoelectric device.

According to still another embodiment, a display device including the organic optoelectric device is provided.

An organic optoelectric device having high efficiency and long life-span may be realized.

### BRIEF DESCRIPTION OF THE DRAWINGS

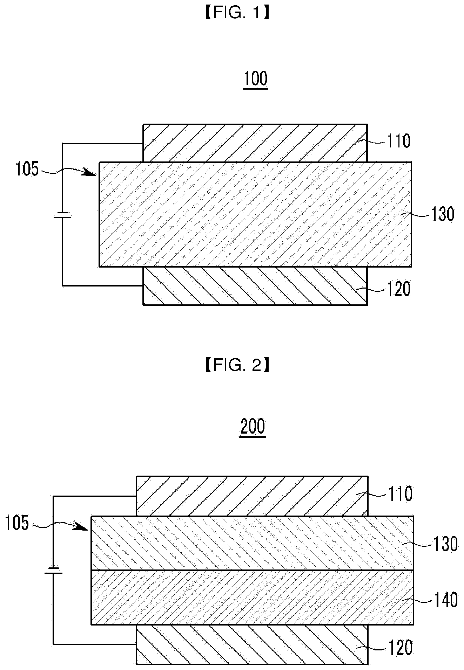

FIGS. 1 and 2 are cross-sectional views of each organic light emitting diode according to one embodiment.

### DETAILED DESCRIPTION

As used herein, when a definition is not otherwise provided, the term "substituted" refers to one substituted with a deuterium, a halogen, a hydroxy group, an amino group, a substituted or unsubstituted C1 to C30 amine group, a nitro group, a substituted or unsubstituted C1 to C40 silyl group, a C1 to C30 alkyl group, a C1 to C10 alkylsilyl group, a C3 to C30 cycloalkyl group, a C3 to C30 heterocycloalkyl group, a C6 to C30 aryl group, a C6 to C30 heterocycle, a C1 to C20 alkoxy group, a fluoro group, a C1 to C10 trifluoroalkyl group such as a trifluoromethyl group, and the like, or a cyano group, instead of at least one hydrogen of a substituent or a compound.

In addition, two adjacent substituents of the substituted halogen, hydroxy group, amino group, substituted or unsubstituted C1 to C30 amine group, nitro group, substituted or unsubstituted C1 to C40 silyl group, C1 to C30 alkyl group, C1 to C10 alkylsilyl group, C3 to C30 cycloalkyl group, C3 to C30 heterocycloalkyl group, C6 to C30 aryl group, C6 to C30 heterocyclic, C1 to C20 alkoxy group, fluoro group, C1 to C10 trifluoroalkyl group such as a trifluoromethyl group, and the like, or cyano group may be fused with each other to provide a ring. For example, the substituted C6 to C30 aryl group may be fused with another adjacent substituted C6 to C30 aryl group to form a substituted or unsubstituted fluorene ring.

In the present specification, when specific definition is not otherwise provided, the term "hetero" refers to one including 1 to 3 hetero atoms selected from N, O, S, P, and Si, and remaining carbons in one functional group.

As used herein, the term "aryl group" refers to a substituent including all element of the cycle having p-orbitals which form conjugation, and may be monocyclic, polycyclic or fused ring polycyclic (i.e., rings sharing adjacent pairs of carbon atoms) functional group.

As used herein, the term "heterocycle" may refer to a cyclic compound such as an aryl group or a cycloalkyl group including 1 to 3 hetero atoms selected from N, O, S, P, and Si and remaining carbons in one functional group. When the heterocycle is a fused ring, the entire ring or each ring of the heterocycle may include one or more hetero atom.

More specifically, the substituted or unsubstituted C6 to C30 aryl group and/or the substituted or unsubstituted C2 to C30 heterocycle may be a substituted or unsubstituted phenyl group, a substituted or unsubstituted naphthyl group, a substituted or unsubstituted anthracenyl group, a substituted or unsubstituted phenanthryl group, a substituted or unsubstituted naphthacenyl group, a substituted or unsubstituted pyrenyl group, a substituted or unsubstituted biphenyl group, a substituted or unsubstituted p-terphenyl group, a substituted or unsubstituted m-terphenyl group, a substituted or unsubstituted chrysenyl group, a substituted or unsubstituted triphenylenyl group, a substituted or unsubstituted perylenyl group, a substituted or unsubstituted indenyl group, a substituted or unsubstituted furanyl group, a substituted or unsubstituted thiophenyl group, a substituted or unsubstituted pyrrolyl group, a substituted or unsubstituted pyrazolyl group, a substituted or unsubstituted imidazolyl group, a substituted or unsubstituted triazolyl group, a substituted or unsubstituted oxazolyl group, a substituted or unsubstituted thiazolyl group, a substituted or unsubstituted oxadiazolyl group, a substituted or unsubstituted thiadiazolyl group, a substituted or unsubstituted pyridyl group, a substituted or unsubstituted pyrimidinyl group, a substituted or unsubstituted pyrazinyl group, a substituted or unsubstituted triazinyl group, a substituted or unsubstituted benzofuranyl group, a substituted or unsubstituted benzothiophenyl group, a substituted or unsubstituted benzimidazolyl group, a substituted or unsubstituted indolyl group, a substituted or unsubstituted quinolinyl group, a substituted or unsubstituted isoquinolinyl group, a substituted or unsubstituted quinazolinyl group, a substituted or unsubstituted quinoxalinyl group, a substituted or unsubstituted naphthyridinyl group, a substituted or unsubstituted benzoxazinyl group, a substituted or unsubstituted benzthiazinyl group, a substituted or unsubstituted acridinyl group, a substituted or unsubstituted phenazinyl group, a substituted or unsubstituted phenothiazinyl group, a substituted or unsubstituted phenoxazinyl group, a substituted or unsubstituted fluorenyl group, a substituted or unsubstituted dibenzofuranyl group, a substituted or unsubstituted dibenzothiophenyl group, a substituted or unsubstituted carbazole group, a combination thereof or a fused combination thereof.

In the specification, hole characteristics refer to characteristics capable of donating an electron to form a hole when electric field is applied, and characteristics that hole formed in the anode is easily injected into the emission layer and transported in the emission layer due to conductive characteristics according to HOMO level.

In addition, electron characteristics refer to characteristics capable of accepting an electron to form a hole when electric field is applied, and characteristics that electron formed in the cathode is easily injected into the emission layer and transported in the emission layer due to conductive characteristics according to LUMO level.

Hereinafter, an organic compound according to one embodiment is described.

The organic compound according to one embodiment is represented by the following Chemical Formula 1. 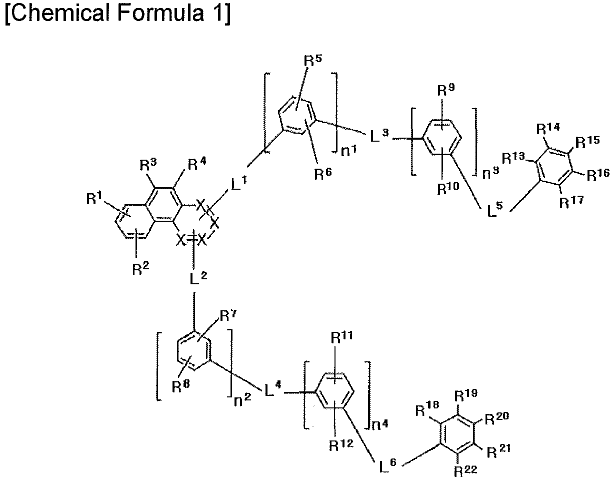 In the Chemical Formula 1,
two of X's are N and two of X's are C,
R¹ to R⁴ are independently hydrogen, deuterium, unsubstituted C1 to C10 alkyl group, unsubstituted C6 to C12 aryl group, unsubstituted C3 to C12 heterocycle, or a combination thereof,
R⁵ to R¹² are independently hydrogen, deuterium, unsubstituted C1 to C10 alkyl group, unsubstituted C6 to C12 aryl group, or a combination thereof,
R¹³ to R²² are independently hydrogen, deuterium, unsubstituted C1 to C10 alkyl group, unsubstituted C6 to C12 aryl group, unsubstituted C3 to C12 heterocycle, or a combination thereof,
R¹³ to R²² are independently present or adjacent two are linked to each other to form a fused ring,
L¹ to L⁶ are independently a single bond, unsubstituted phenylene group, unsubstituted biphenylene group, unsubstituted terphenylene group or unsubstituted quaterphenylene group,
n¹ to n⁴ are independently integers ranging from 0 to 5, and
the sum of n¹ to n⁴ is an integer of 2 or more, preferably 6 or less

The organic compound represented by the Chemical Formula 1 includes a substituted or unsubstituted benzoquinazoline including two nitrogens and at least two meta-bonded substituted or unsubstituted phenylene groups.

The nitrogen moiety of the substituted or unsubstituted benzoquinazoline has polarity and thus, may interact with an electrode and accordingly, inject a charge easily and increase charge mobility by three fused rings.

Furthermore, the substituted or unsubstituted benzoquinazoline has a relatively low LUMO energy level and thus, may easily inject electrons and improve thermal stability and electrical stability. The substituted or unsubstituted benzoquinazoline may have, for example, a LUMO energy level ranging from about 1.7 to about 2.1 eV.

The at least two meta-bonded substituted or unsubstituted phenylene groups may control a charge stream flowing toward the benzoquinazoline and thus, increase stability of the benzoquinazoline. In particular, the at least two meta-bonded substituted or unsubstituted phenylene groups may decrease oxidation of cyclic carbons neighboring a nitrogen-containing cycle of the benzoquinazoline, that is, carbons bonded with R³ or R⁴ and thus, increase stability of the organic compound. Accordingly, life-span of the organic compound may be improved.

For example, the R³ or R⁴ may be independently hydrogen.

The at least two meta-bonded substituted or unsubstituted phenylene groups may be positioned at one side or both sides of the benzoquinazoline. For example, the at least two meta-bonded substituted or unsubstituted phenylene groups may be positioned at both sides of the benzoquinazoline, for example, n1 and n2 of the above Chemical Formula 1 may be independently 1 to 5.

In addition, the organic compound structurally has steric hinderance characteristics and is suppressed from interaction with a neighboring molecule and thus, may decrease crystallization and improve efficiency and life-span characteristics.

The organic compound may have, for example, a molecular weight of greater than or equal to about 500. The organic compound has the molecular weight and thus, may show an increased glass transition temperature (Tg) and thus, increased stability but may be suppressed from degradation during the manufacuturing process when applied to a device.

The glass transition temperature (Tg) may be related to thermal stability of an organic compound and a device manufactured by applying the same. In other words, when an organic compound having a high glass transition temperature (Tg) is applied as a thin film to an organic light emitting diode, the organic compound and the organic light emitting diode using the compound are prevented from degradation of by a temperature in a subsequent process, for example, an encapsulation process after deposition of the organic compound, securing life-span characteristics of the organic compound and the organic light emitting diode.

The organic compound may have a glass transition temperature (Tg) of for example greater than or equal to about 70° C and effectively, greater than or equal to about 90° C within the range. The glass transition temperature (Tg) may be for example in a range of about 70° C or more to about 150° C or less and specifically about 90° C or more to about 130° C or less within the range.

The organic compound may be represented by the following Chemical Formula 2 or 3 according to the position of nitrogen (N).

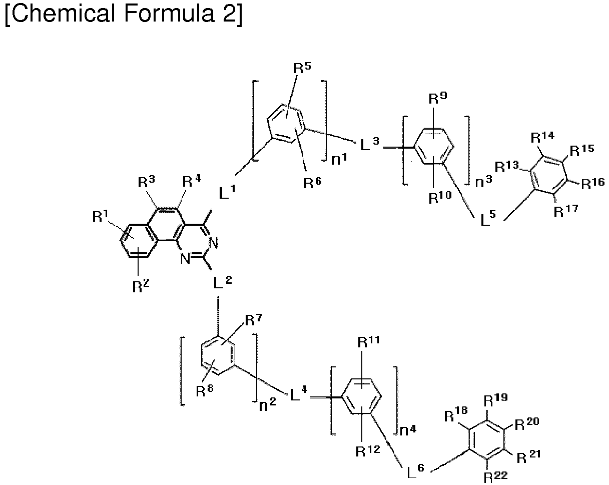

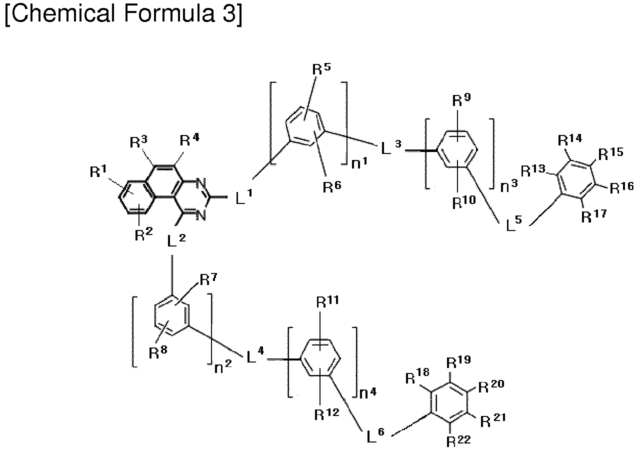

In Chemical Formula 2 or 3, R¹ to R²², L¹ to L⁶ and n¹ to n⁴ are the same as described above.

The Chemical Formula 2 may be, for example represented by the following Chemical Formula 2A.

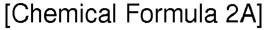

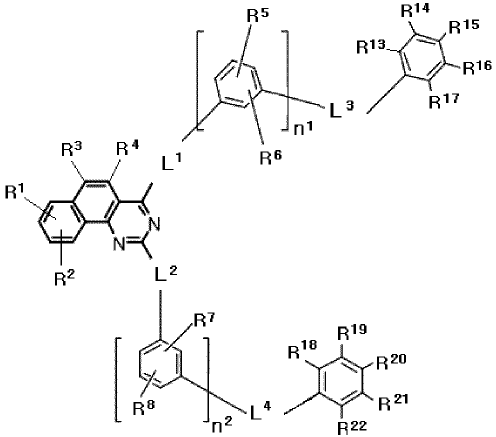

In the Chemical Formulae 2A or 3A, R¹ to R⁸, R¹³ to R²², L¹ to L⁴ and n¹ and n² are each is the same as described above.

The Chemical Formula 3 may be, for example represented by the following Chemical Formula 3A.

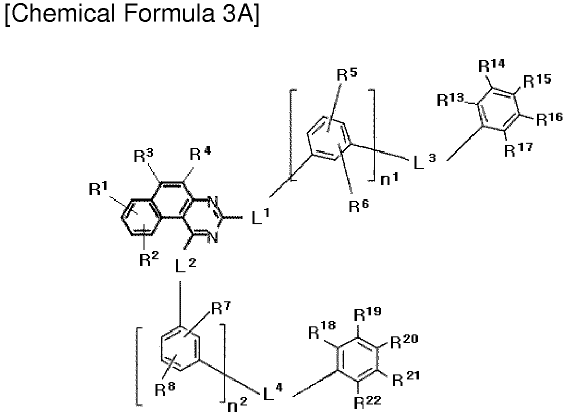

In the Chemical Formula 3A, R¹ to R⁸, R¹³ to R²², L¹ to L⁴ and n¹ and n² are the same as described above.

In the Chemical Formulae 1 to 3, 2A and 3A, the L¹ to L⁶ may be, for example a single bond or one of groups listed in the following Group 1. 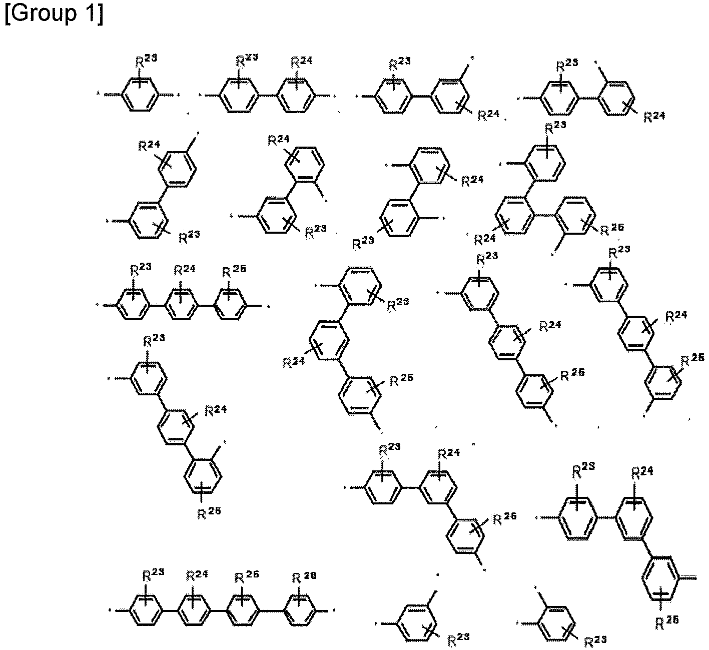 In the Group 1,
R²³ to R²⁶ are independently hydrogen, deuterium, unsubstituted C1 to C10 alkyl group, unsubstituted C6 to C12 aryl group, unsubstituted C3 to C12 heterocycle, or a combination thereof, and
* is a linking point.
For example, in the Group 1, R²³ to R²⁶ may be independently hydrogen.

The organic compound may be, for example one of compounds listed in the following Group 2, but is not limited thereto.

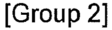

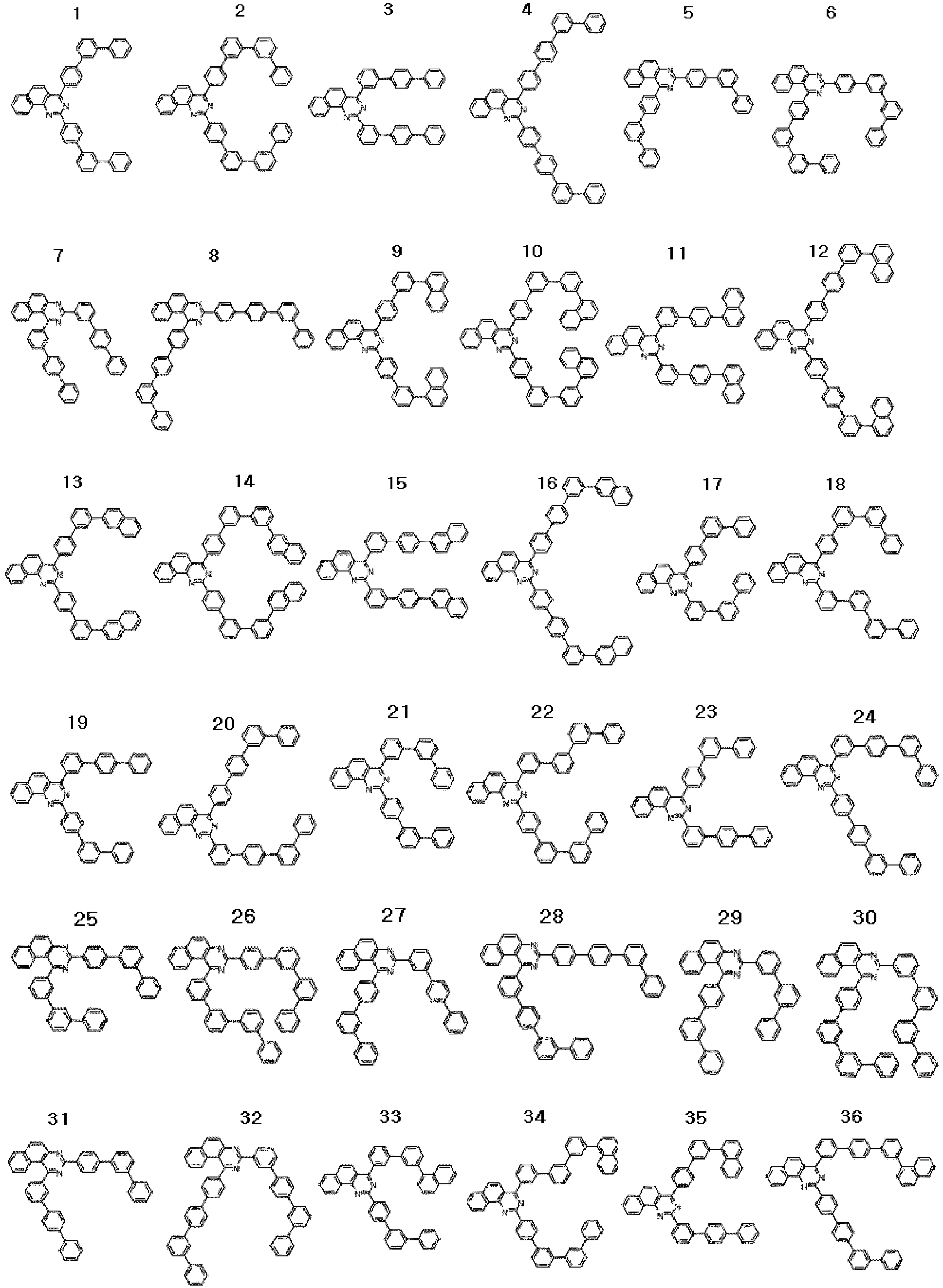

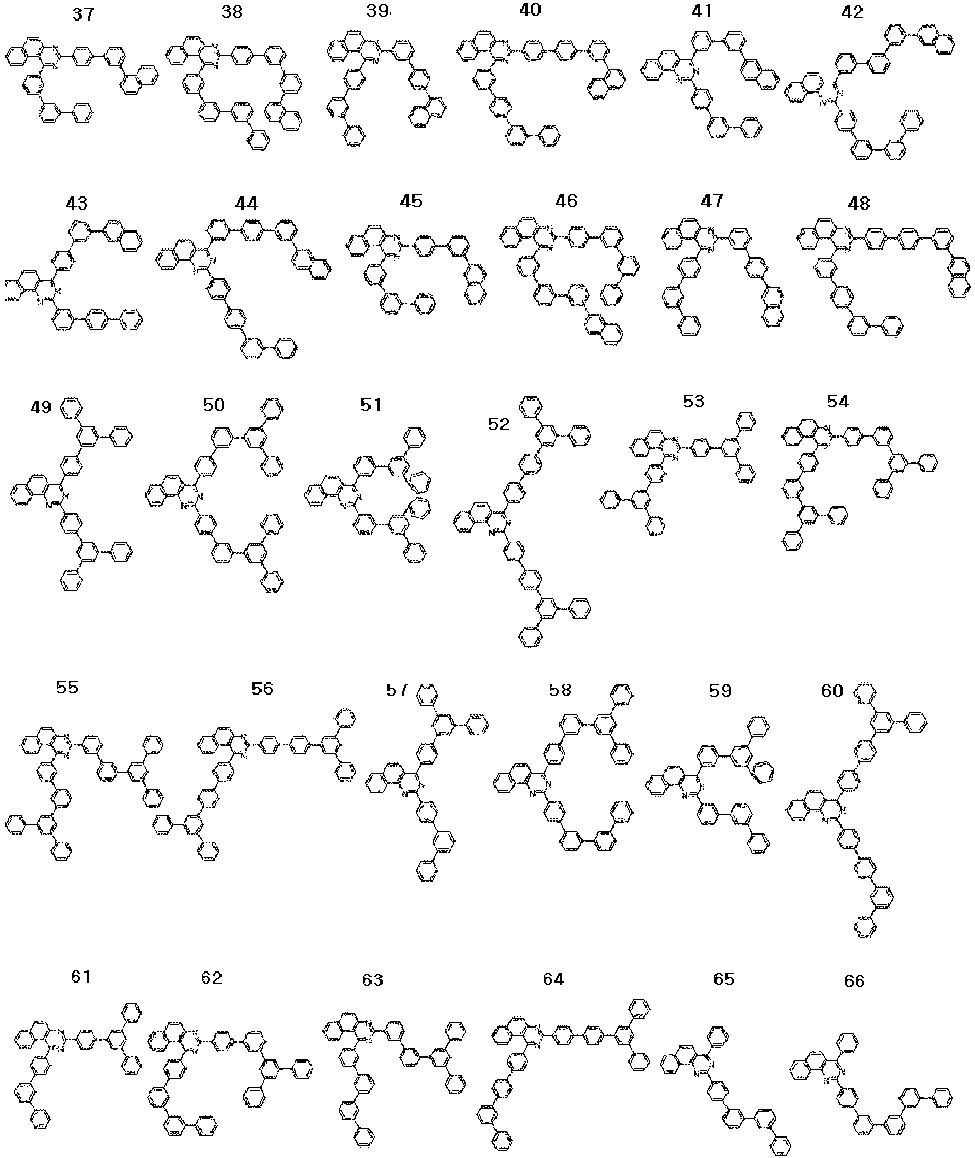

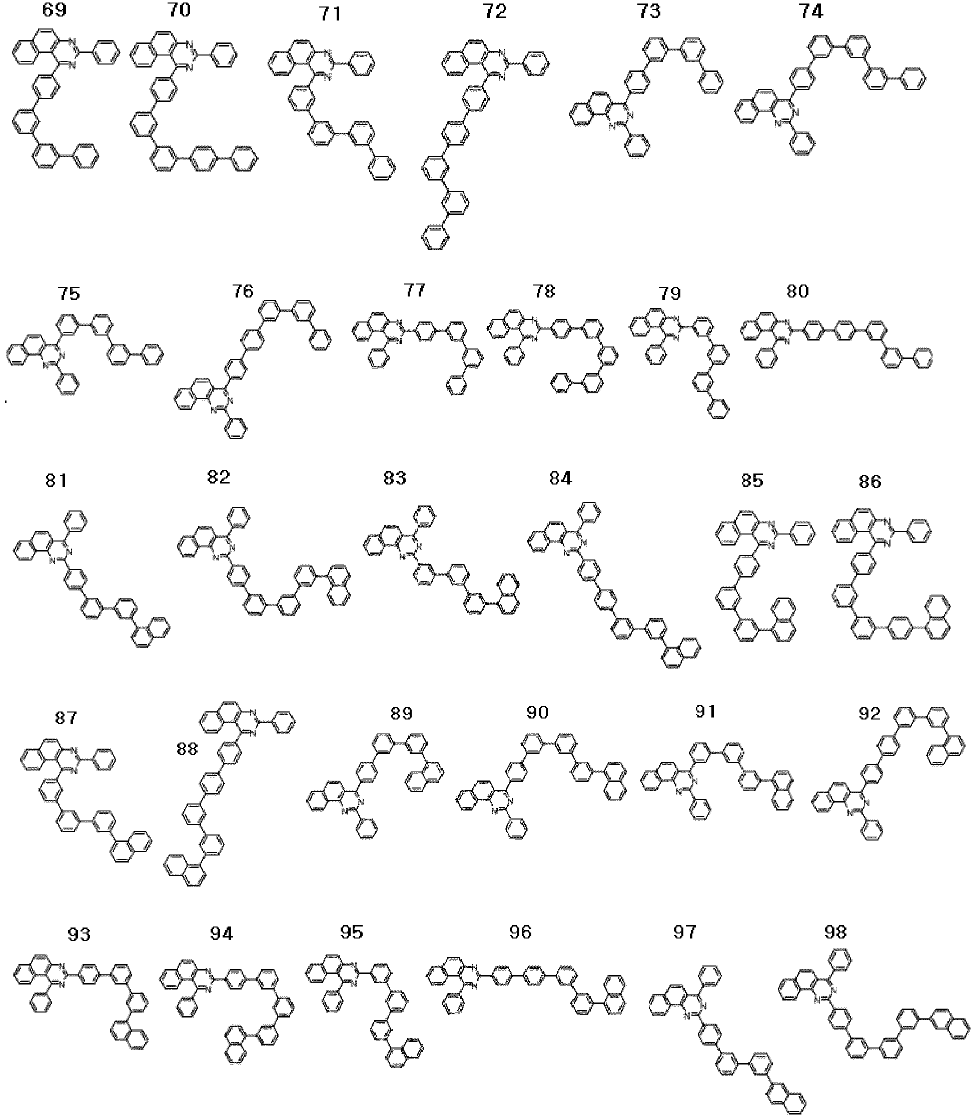

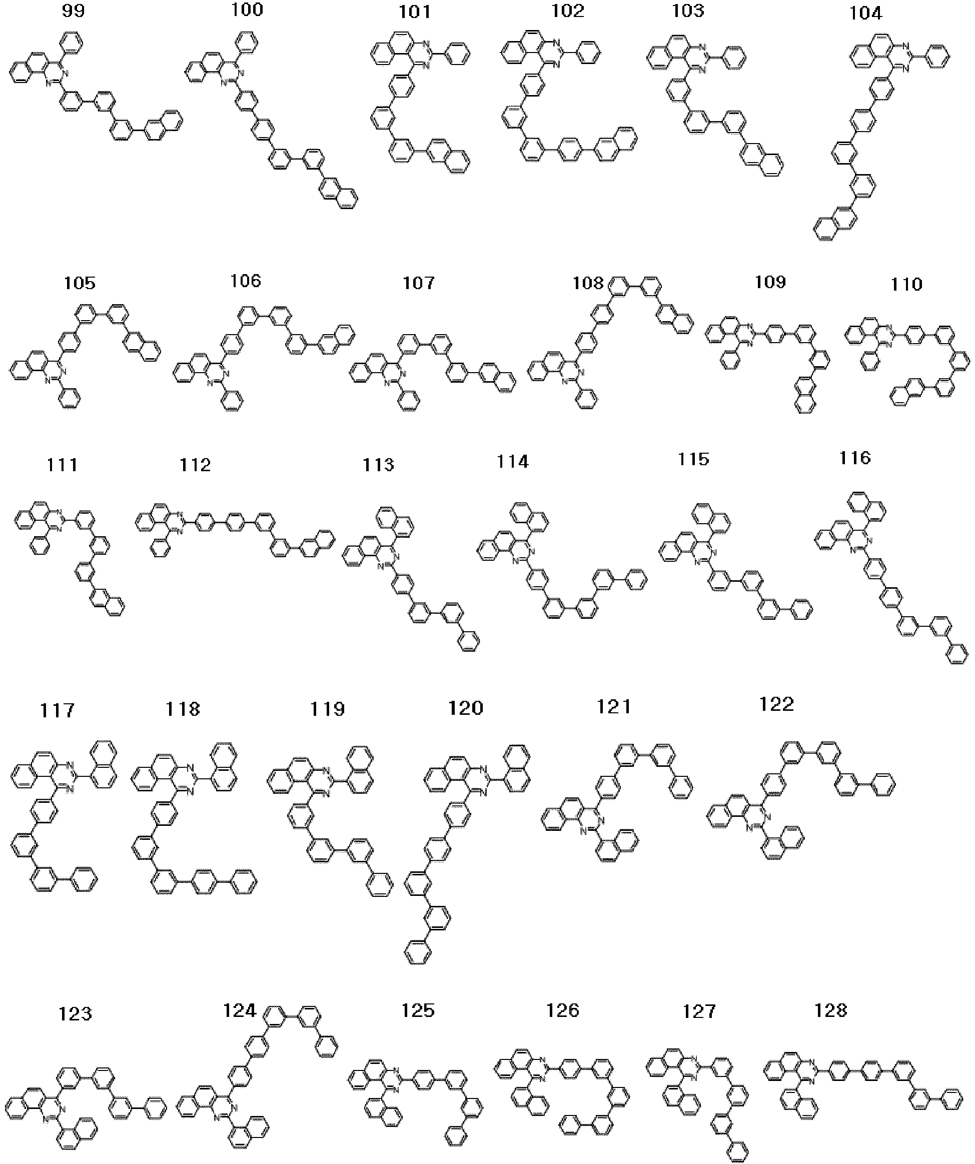

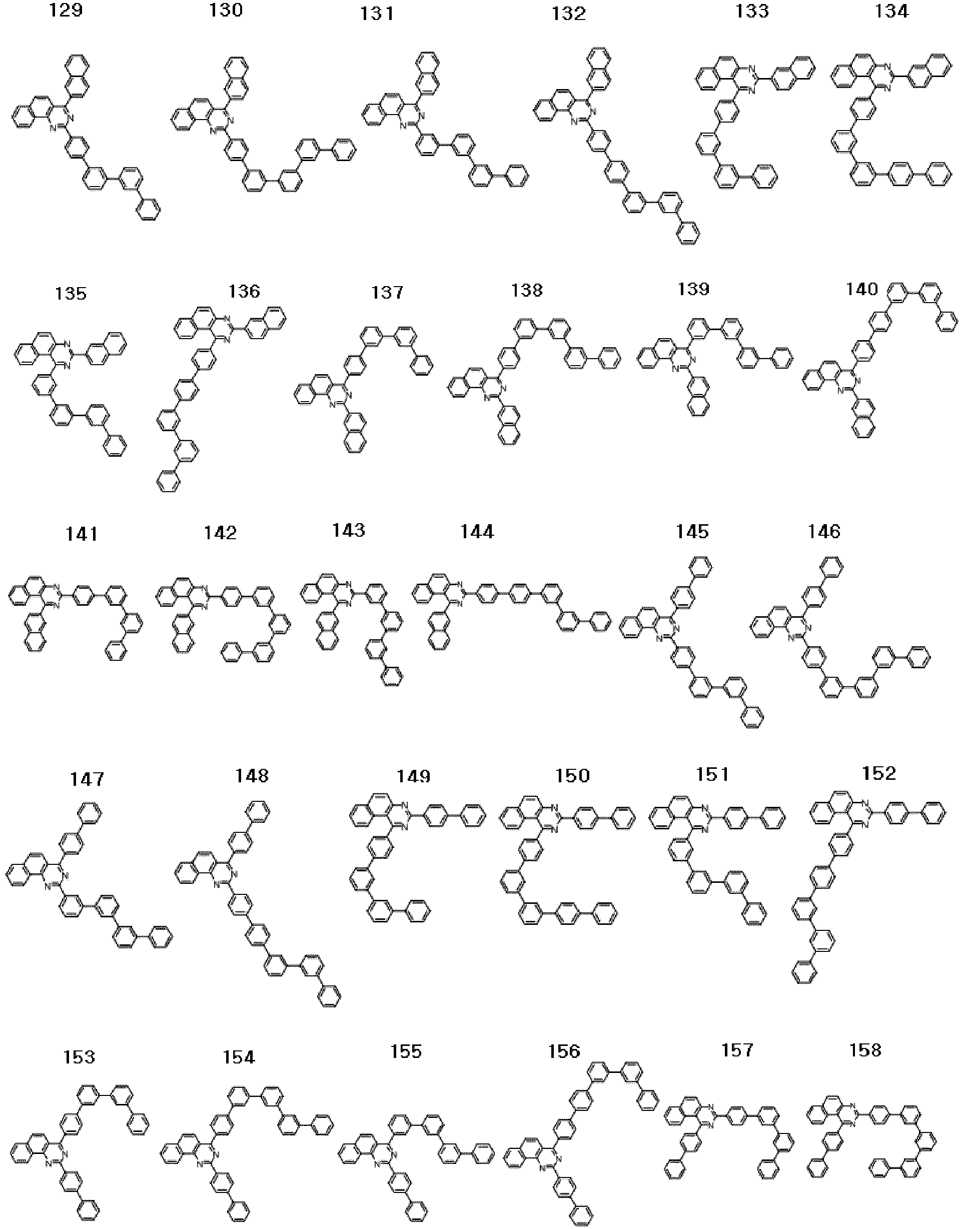

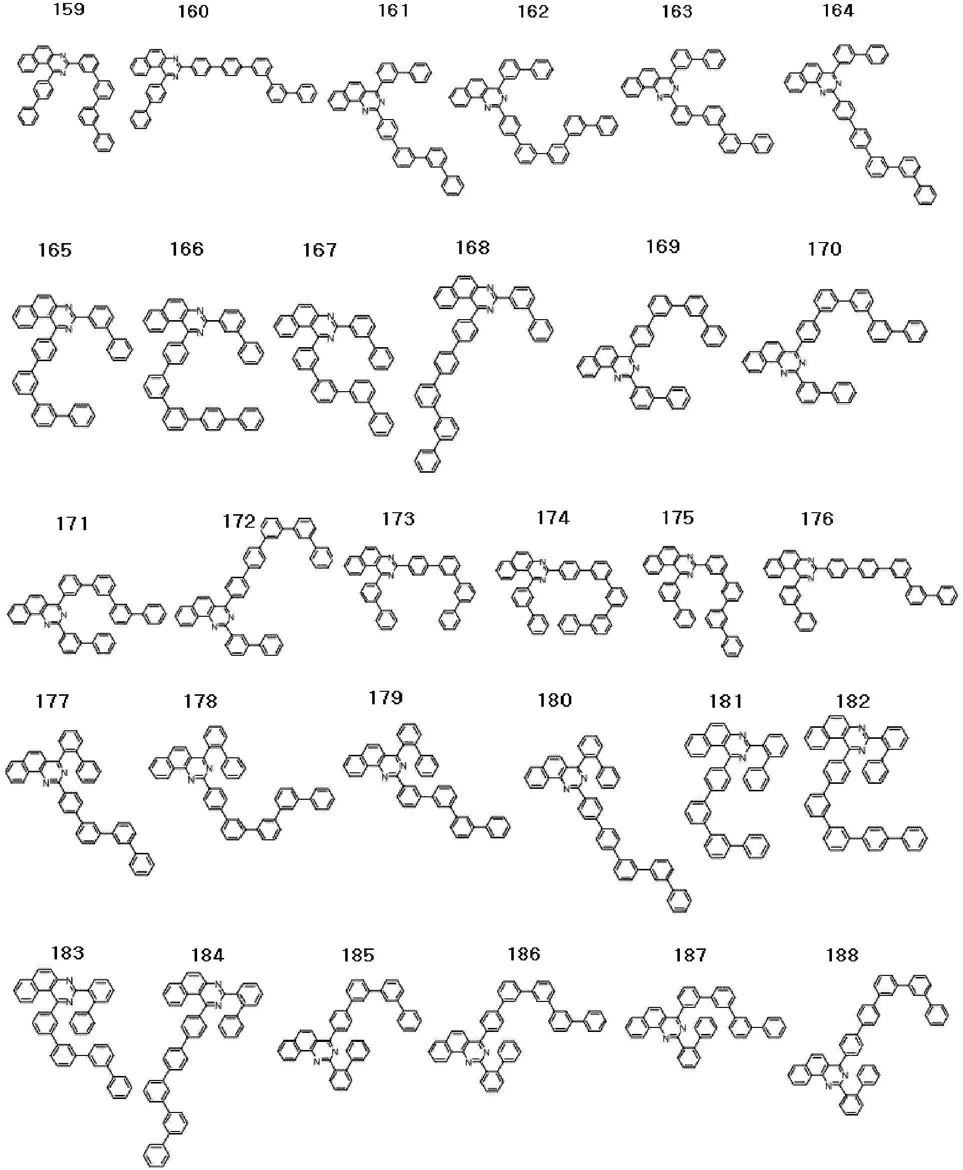

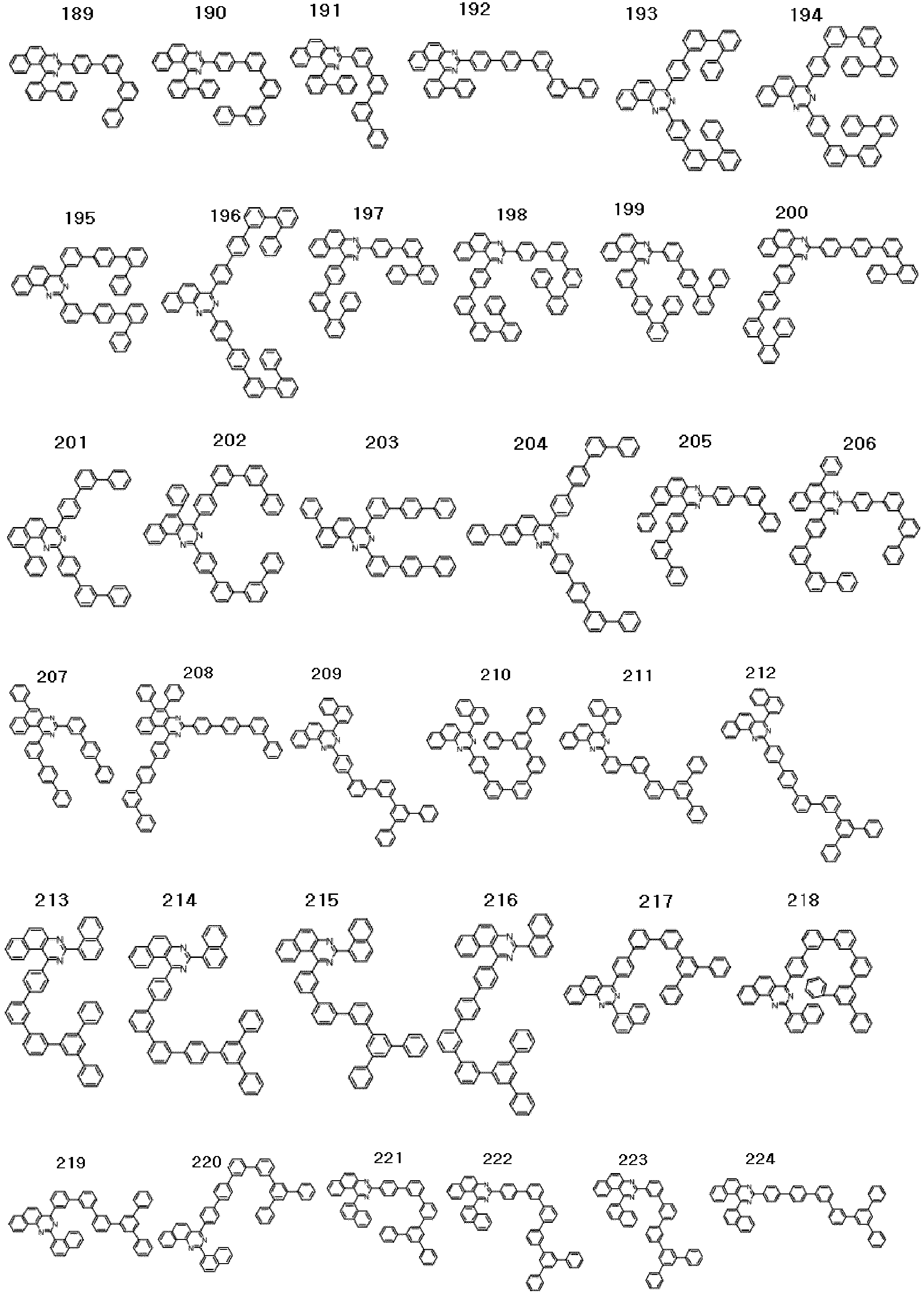

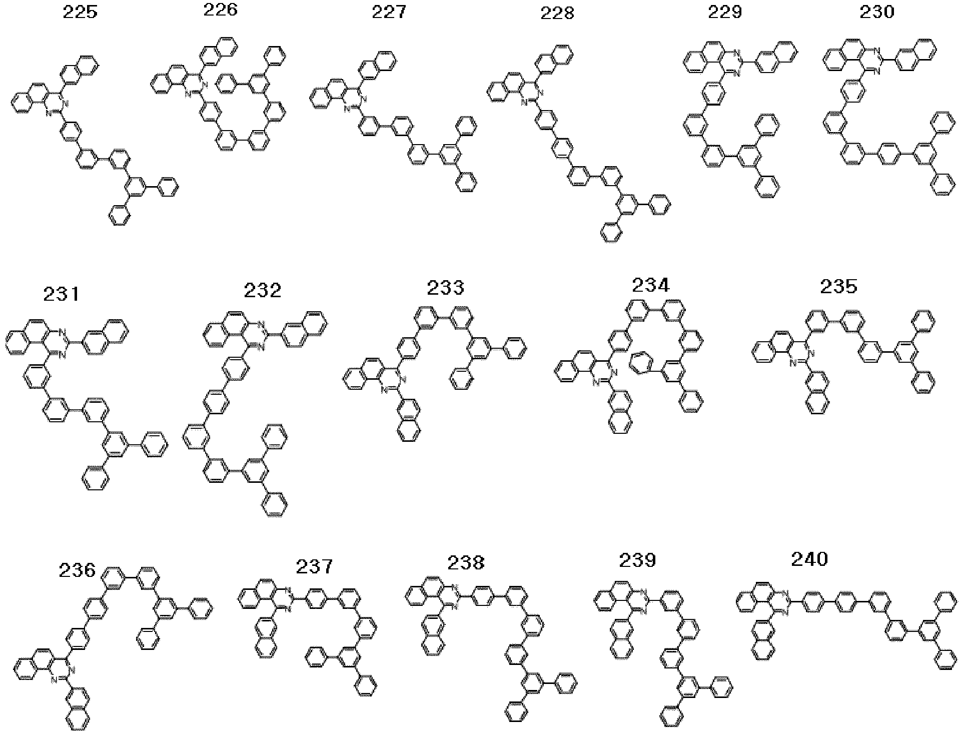

The organic compound may be applied to an organic optoelectric device.

The organic compound may be applied to an organic optoelectric device at alone or with another organic compound. The organic compound may be applied as a composition with another organic compound.

Hereinafter, examples of a composition for an organic optoelectric device including the organic compound are described.

The composition for an organic optoelectric device may be, for example a composition including the organic compound and at least one organic compound having a carbazole moiety. Hereinafter, the organic compound is referred to as 'a first organic compound' and at least one organic compound having a carbazole moiety is referred to as 'a second organic compound'.

The second organic compound may be, for example a compound represented by the following Chemical Formula 4. 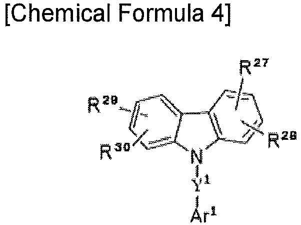 In the Chemical Formula 4,
Y¹ is a single bond, unsubstituted C1 to C20 alkylene group, or unsubstituted C2 to C20 alkenylene group, unsubstituted C6 to C30 arylene group, unsubstituted C2 to C30 heterocycle, or a combination thereof,
Ar¹ is a substituted or unsubstituted C6 to C30 aryl group, a substituted or unsubstituted C2 to C30 heterocycle, or a combination thereof,
R²⁷ to R³⁰ are independently hydrogen, deuterium, unsubstituted C1 to C20 alkyl group, unsubstituted C6 to C50 aryl group, unsubstituted C2 to C50 heterocycle, or a combination thereof, and
at least one of R²⁷ to R³⁰ and Ar¹ includes a triphenylene group or a carbazole group.

The second organic compound represented by the Chemical Formula 4 may be, for example at least one of the following Chemical Formulae 4-I to 4-III: 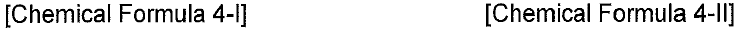 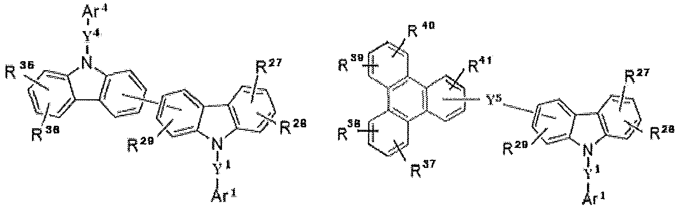 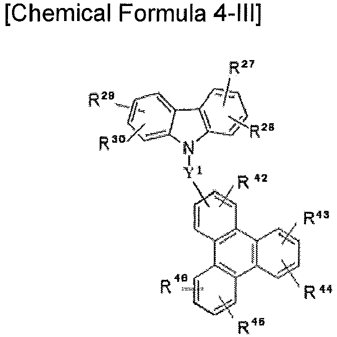 in Chemical Formulae 4-I to 4-III,
Y¹, Y⁴ and Y⁵ are independently a single bond, unsubstituted C1 to C20 alkylene group unsubstituted C2 to C20 alkenylene group, unsubstituted C6 to C30 arylene group, unsubstituted C2 to C30 heterocycle, or a combination thereof,
Ar¹ and Ar⁴ are independently substituted or unsubstituted C6 to C30 aryl group, a substituted or unsubstituted C2 to C30 heterocycle, or a combination thereof, and
R²⁷ to R³¹ and R³⁵ to R⁴⁶ are independently hydrogen, deuterium, unsubstituted C1 to C20 alkyl group, unsubstituted C6 to C50 aryl group, unsubstituted C2 to C50 heterocyclic or a combination thereof.

The second organic compound represented by the Chemical Formula 4 may be, for example one of compounds listed in Group 3.

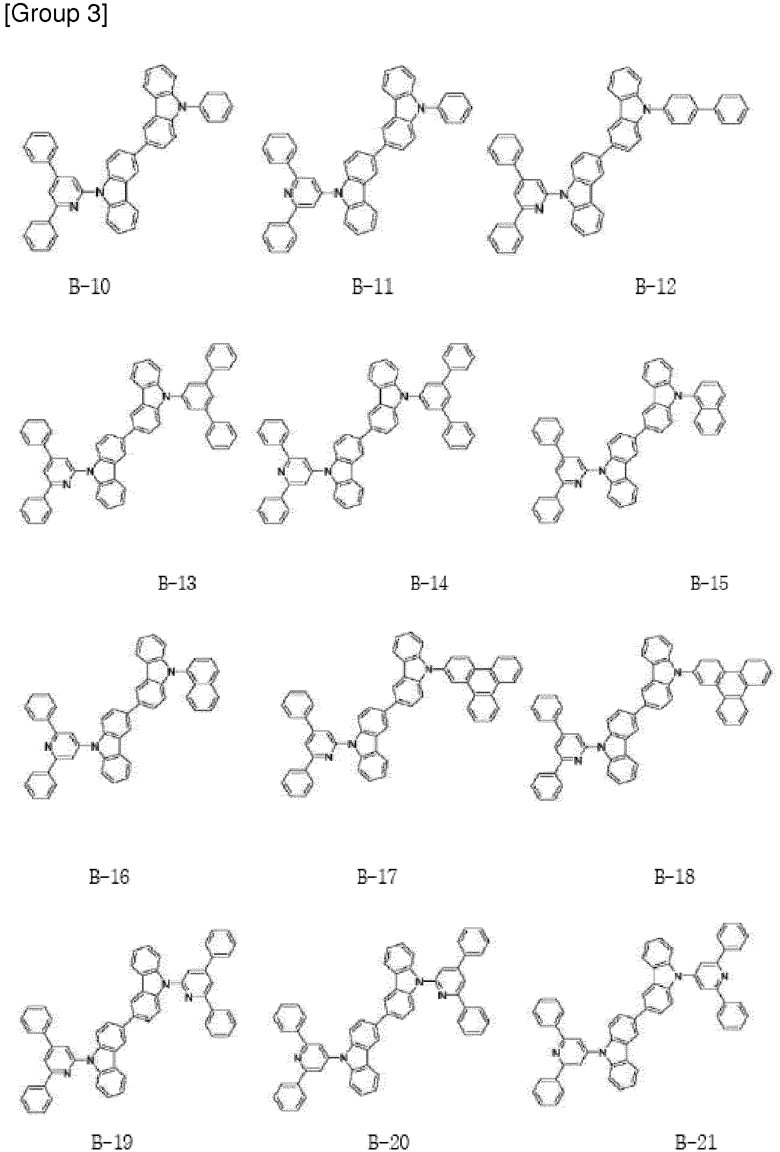

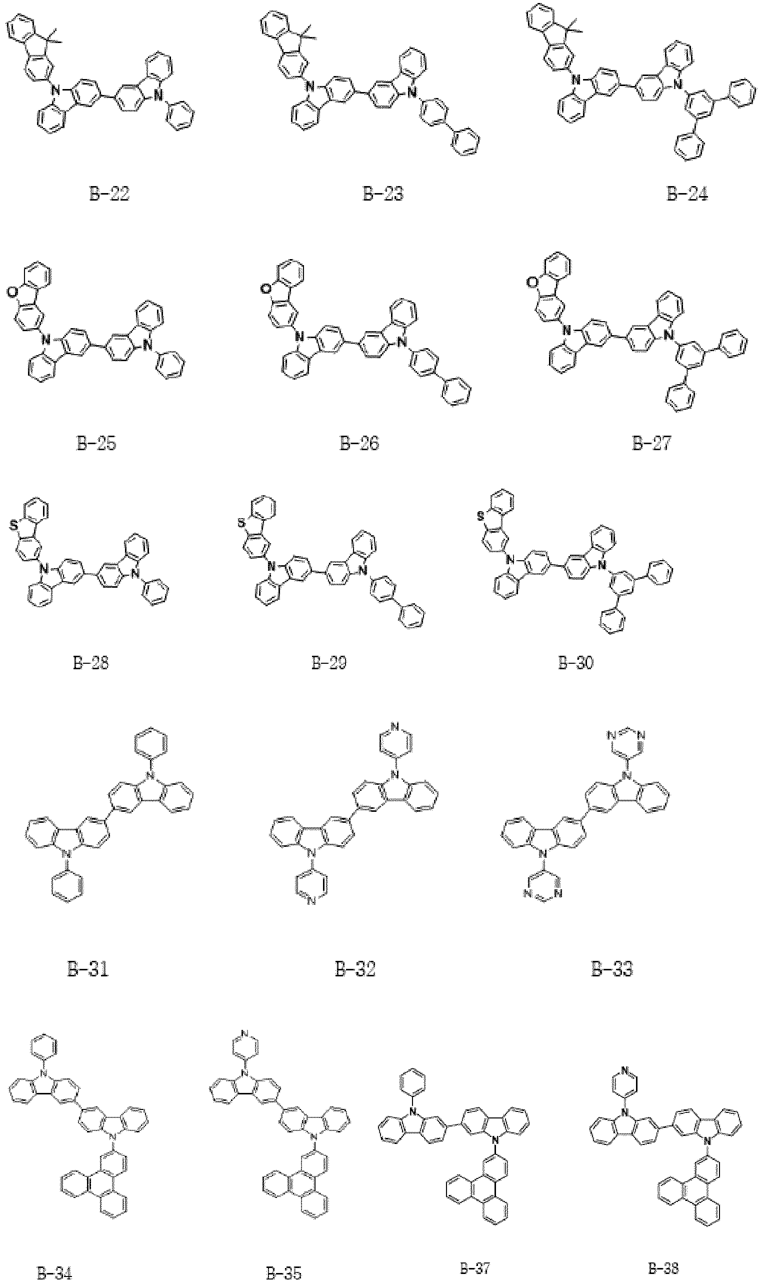

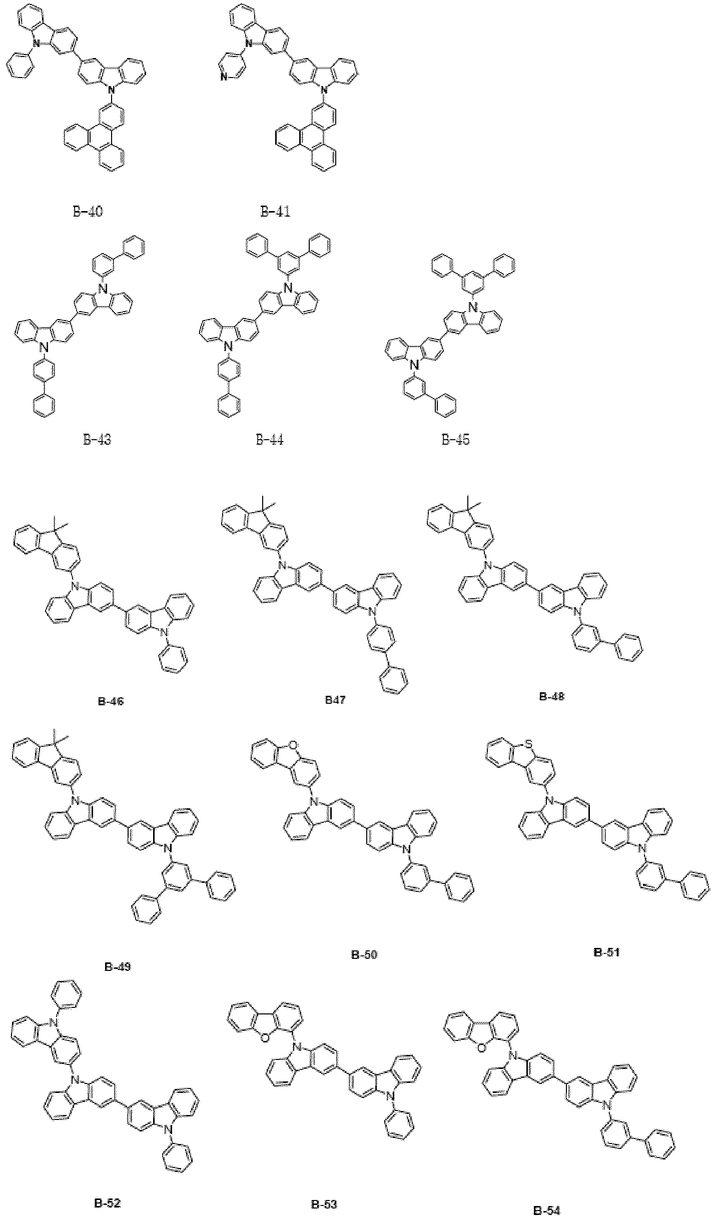

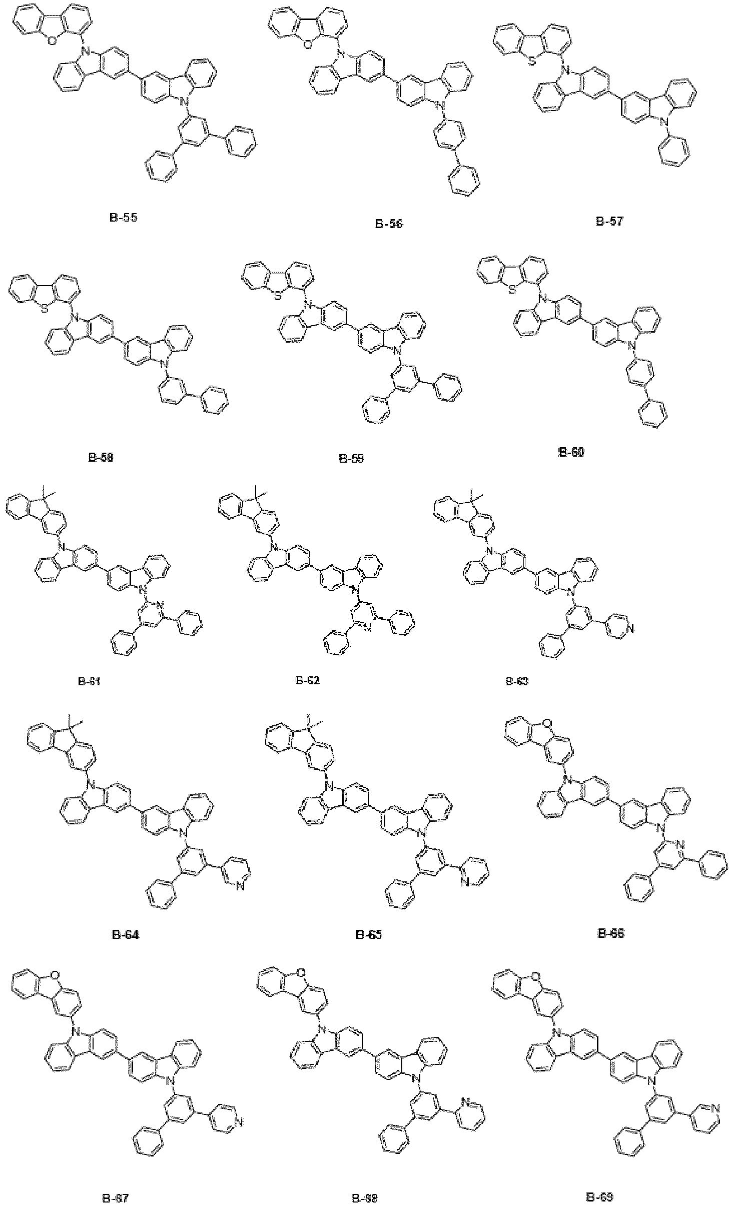

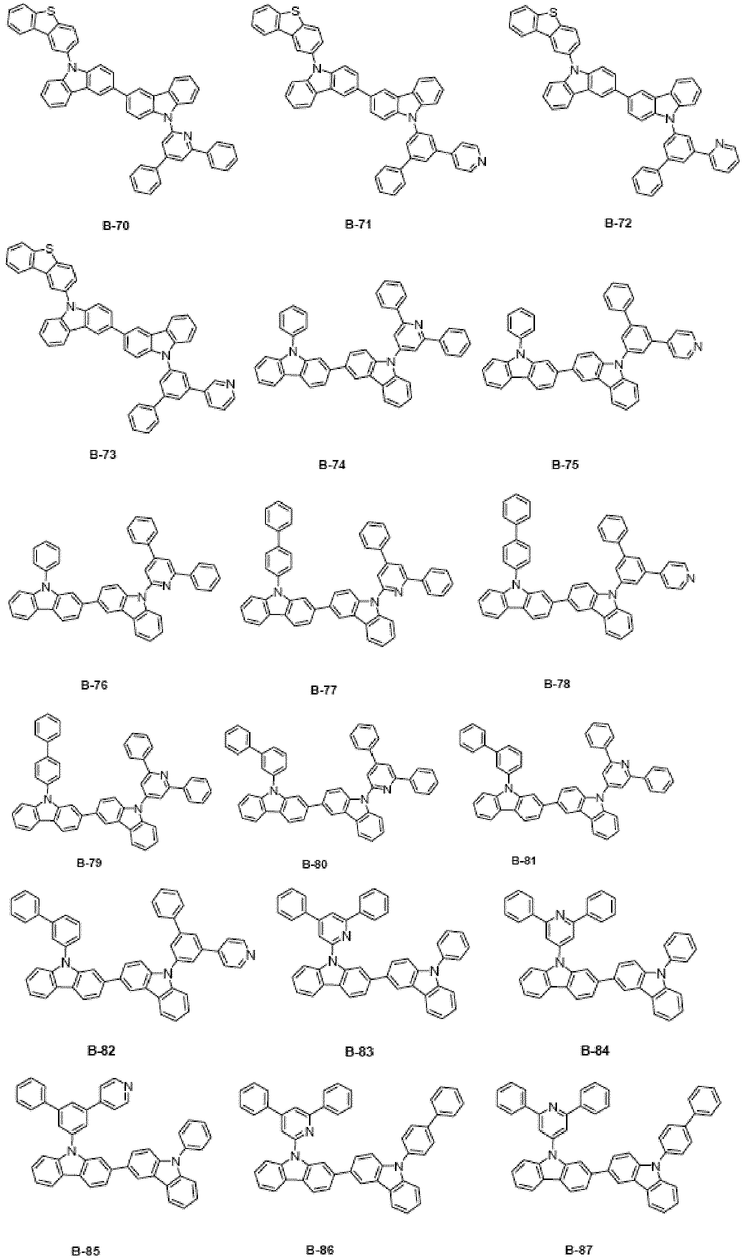

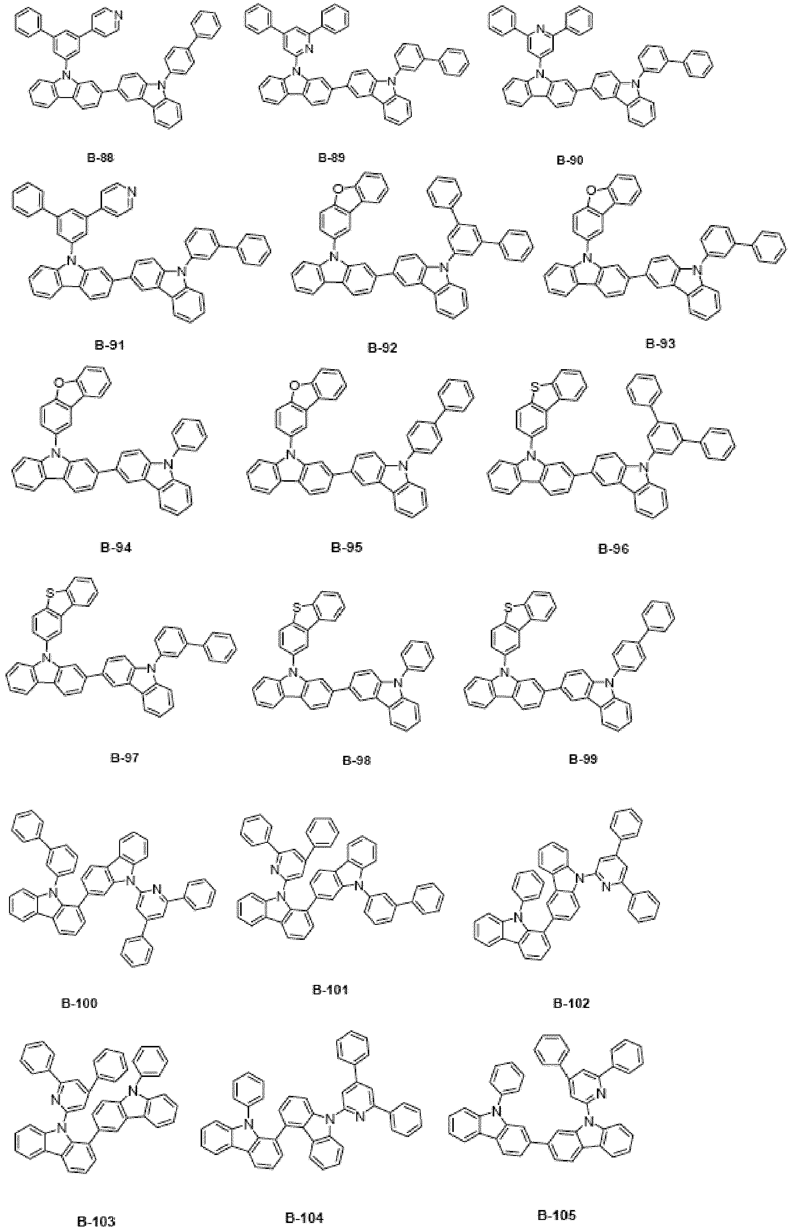

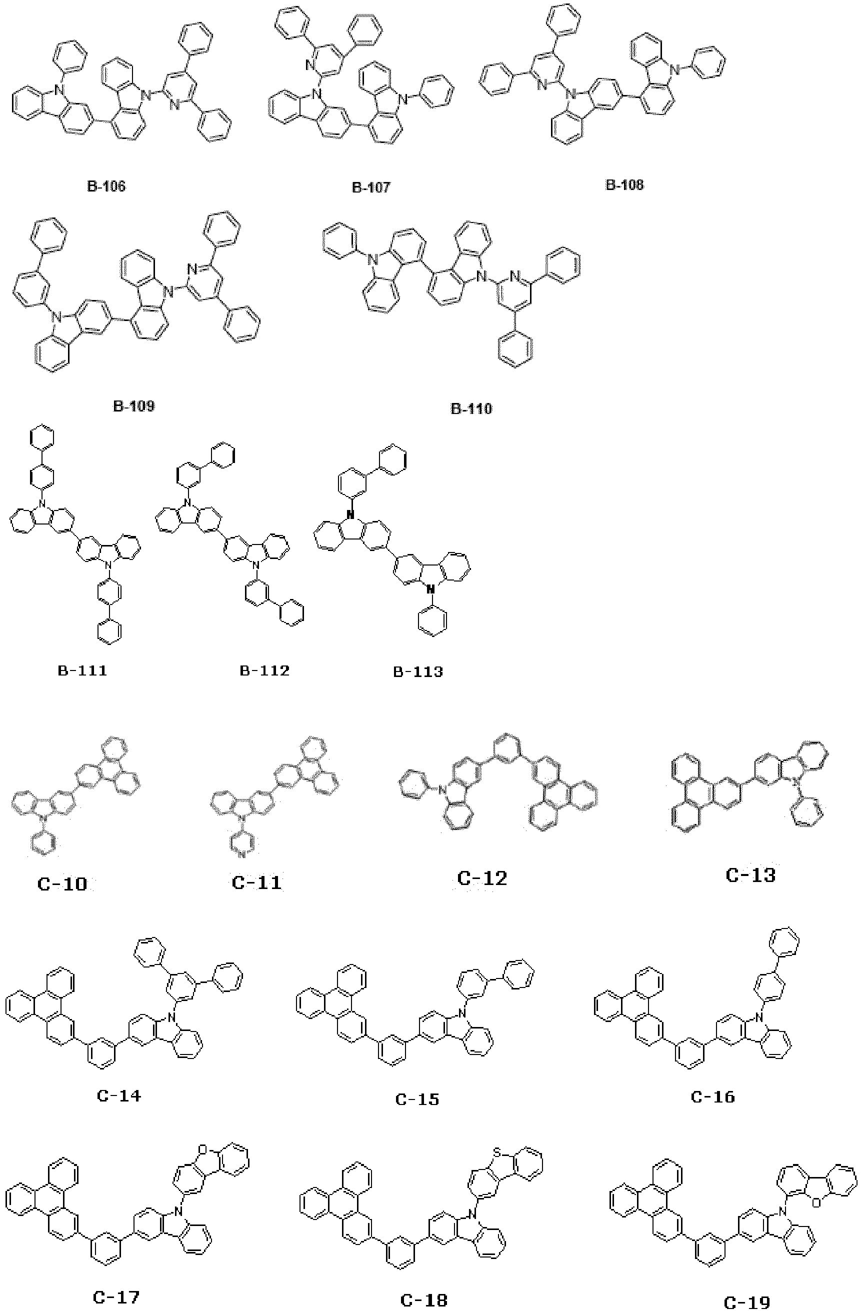

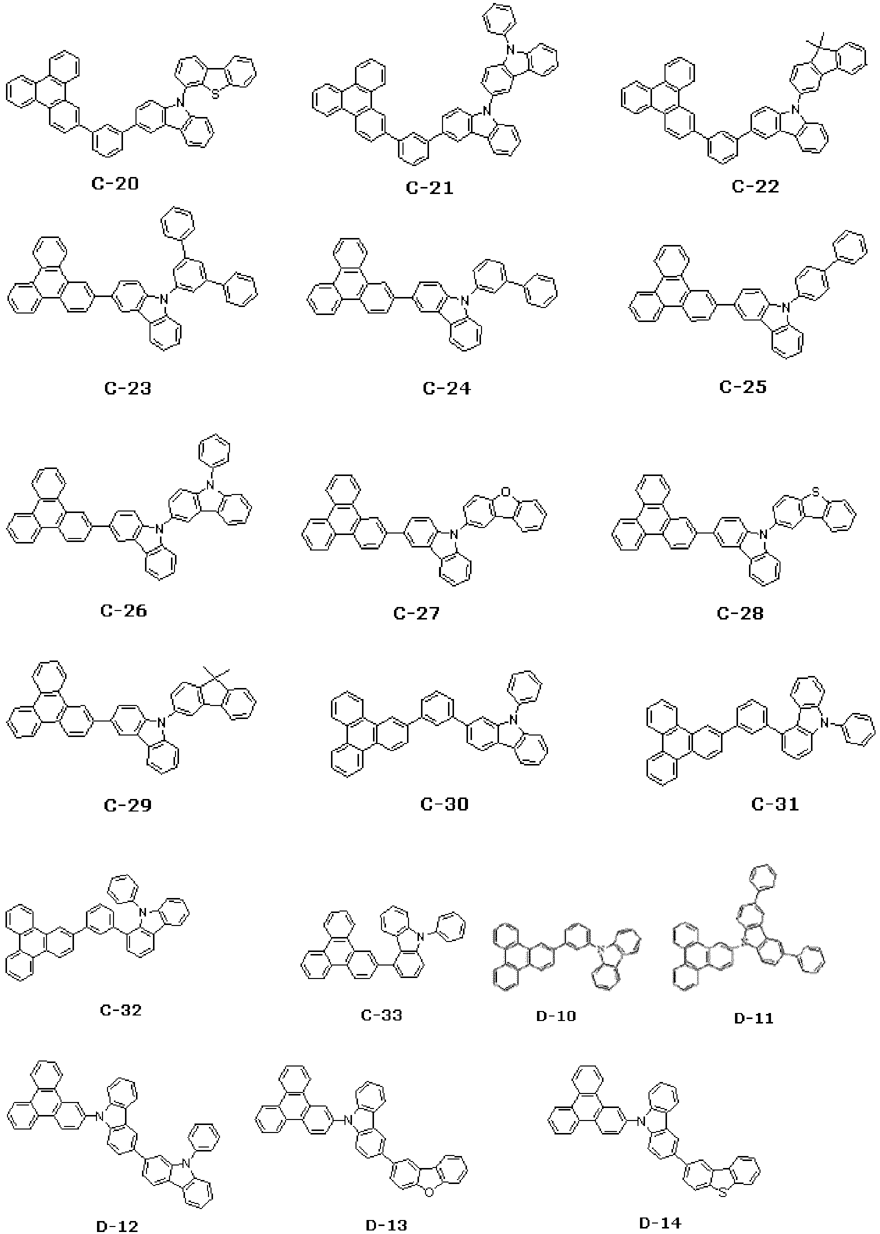

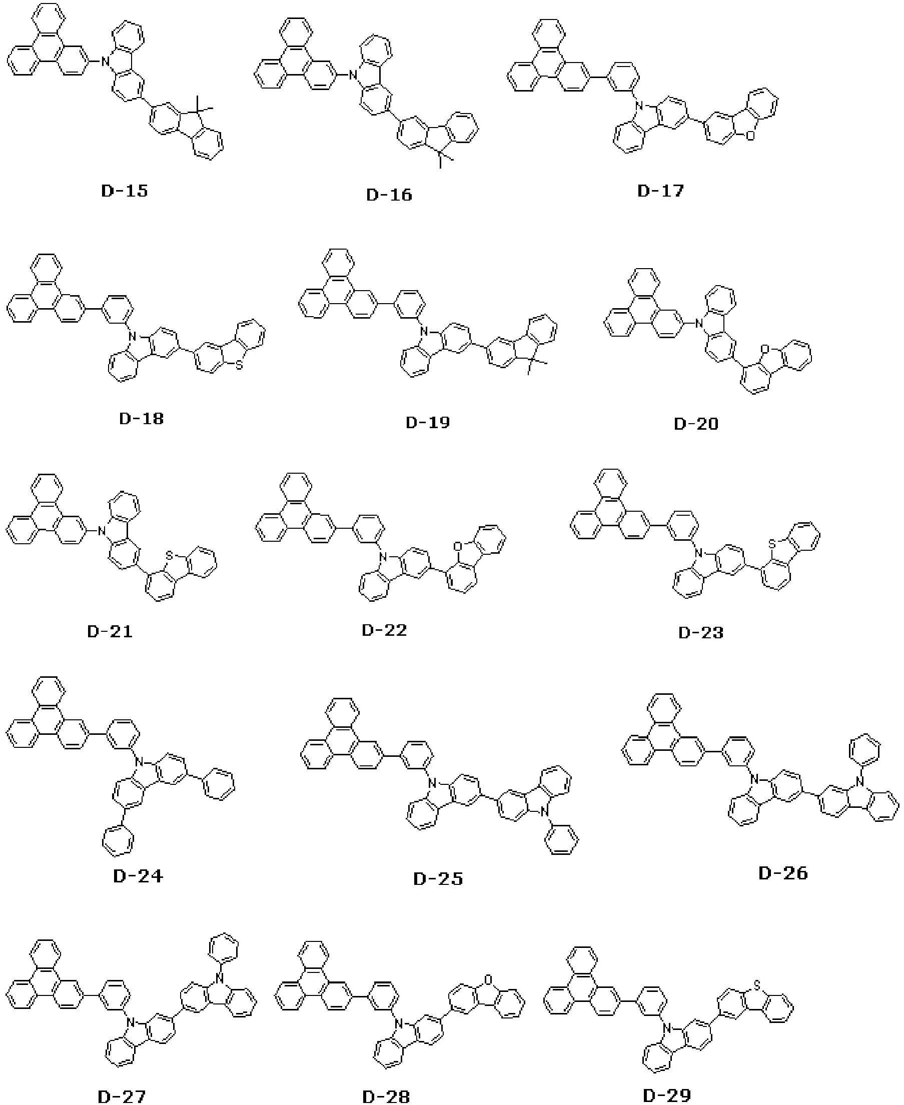

The second organic compound may be, for example a compound consisting of a combination of a moiety represented by the following Chemical Formula 5 and a moiety represented by the following Chemical Formula 6. 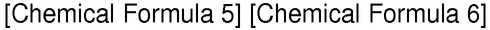 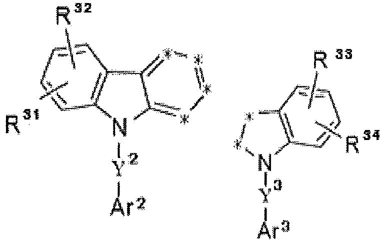 In Chemical Formula 5 or 6,
Y² and Y³ are independently a single bond, unsubstituted C1 to C20 alkylene group, unsubstituted C2 to C20 alkenylene group, unsubstituted C6 to C30 arylene group, unsubstituted C2 to C30 heterocycle, or a combination thereof,
Ar² and Ar³ are independently substituted or unsubstituted C6 to C30 aryl group, a substituted or unsubstituted C2 to C30 heterocycle, or a combination thereof,
R³¹ to R³⁴ are independently hydrogen, deuterium, unsubstituted C1 to C20 alkyl group, unsubstituted C6 to C50 aryl group, unsubstituted C2 to C50 heterocycle, or a combination thereof,
adjacent two *'s of the Chemical Formula 5 are combined with the two *'s of the Chemical Formula 6 to form a fused ring, and * that does not form a fused ring in the Chemical Formula 5 is are independently CR^{a}, and
R^{a} is hydrogen, deuterium, unsubstituted C1 to C10 alkyl group, unsubstituted C6 to C12 aryl group, unsubstituted C3 to C12 heterocyclic or a combination thereof.

The organic compound consisting of a combination of the moiety represented by the Chemical Formula 5 and the moiety represented by the Chemical Formula 6 may be one of compounds listed in the following Group 4.

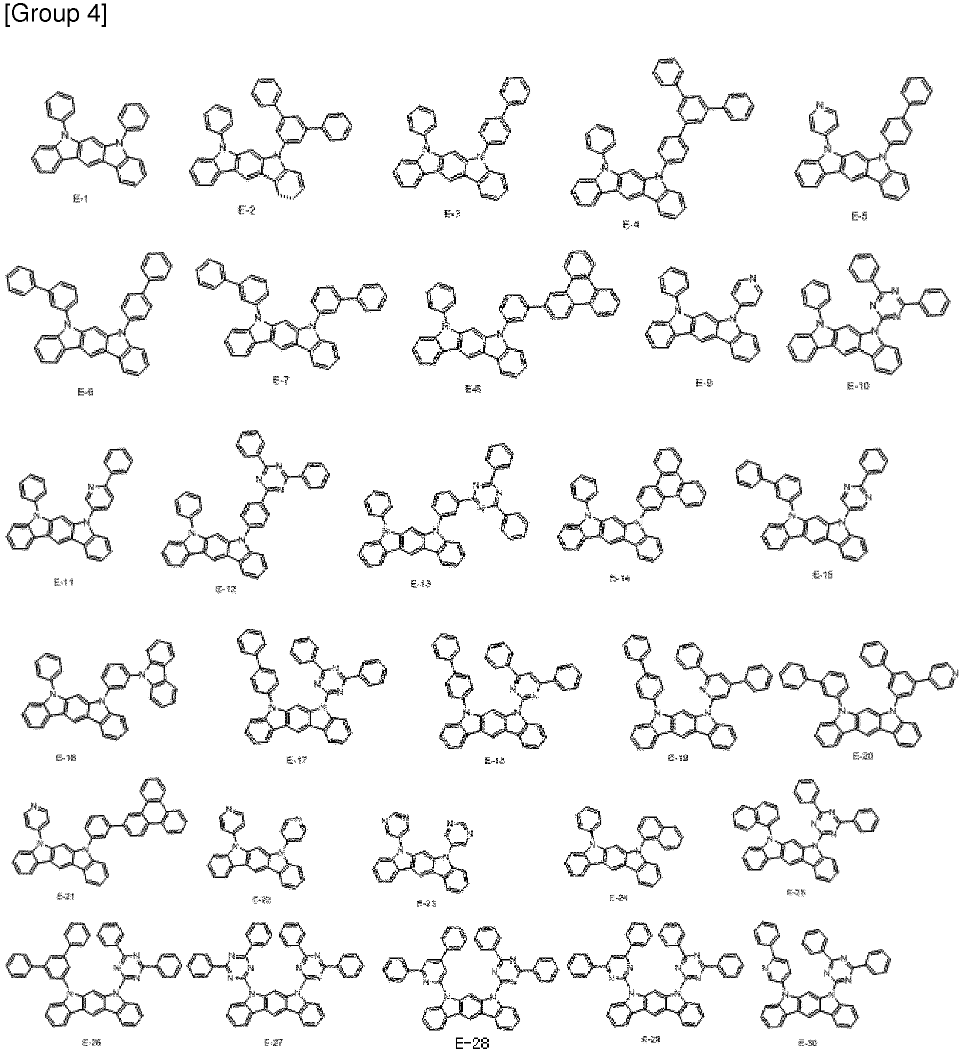

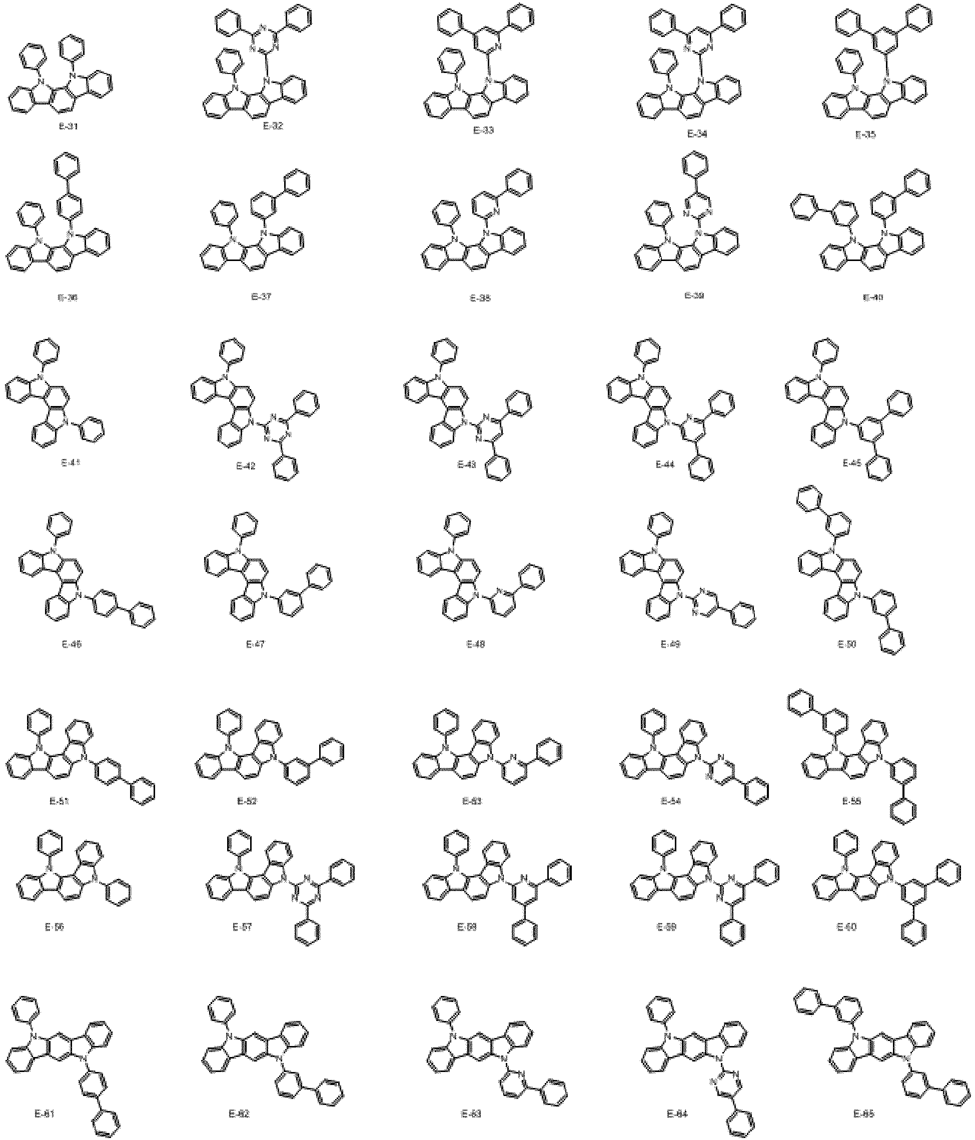

The second organic compound may include at least one of the compound represented by the above Chemical Formula 4 and a compound consisting of a combination of a moiety represented by the following Chemical Formula 5 and a moiety represented by the following Chemical Formula 6.

The composition may include the first organic compound and the second organic compound in a weight ratio of about 1:10 to about 10:1, preferably about 1:5 to about 5:1, most preferred about 1:4 to about 4:1.

The composition may be applied to an organic layer of an organic optoelectric device, and the first organic compound and the second organic compound may play a role of a host. Herein, since the first organic compound has bipolar characteristics in which electron characteristics are relatively strong, while the second organic compound has bipolar characteristics in which hole characteristic is relatively strong, the first and second organic compounds may be used together to increase charge mobility and stability and thus, to remarkably improve luminous efficiency and life-span characteristics.

The composition may further include at least one organic compound as well as the above first organic compound and second organic compound.

The composition may further include a dopant. The dopant may be a red, green, or blue dopant, for example a phosphorescent dopant.

The dopant is mixed with the host compound in a small amount to cause light emission, and may be generally a material such as a metal complex that emits light by multiple excitation into a triplet or more. The dopant may be, for example an inorganic, organic, or organic/inorganic compound, and one or more kinds thereof may be used.

Examples of the phosphorescent dopant may be an organic metal compound including Ir, Pt, Os, Ti, Zr, Hf, Eu, Tb, Tm, Fe, Co, Ni, Ru, Rh, Pd, or a combination thereof. The phosphorescent dopant may be, for example a compound represented by the following Chemical Formula Z.

[Chemical Formula Z] L₂MX

In the Chemical Formula Z, M is a metal, and L and X are the same or different, and are a ligand to form a complex compound with M.

The M may be, for example Ir, Pt, Os, Ti, Zr, Hf, Eu, Tb, Tm, Fe, Co, Ni, Ru, Rh, Pd or a combination thereof, and the L and X may be, for example a bidendate ligand.

The composition may form a film using a dry film-forming method such as chemical vapor deposition.

Hereinafter, an organic optoelectric device to which the organic compound or the composition is applied is described.

The organic optoelectric device may be any device to convert electrical energy into photoenergy and vice versa without particular limitation, and may be, for example an organic photoelectric device, an organic light emitting diode, an organic solar cell, and an organic photo-conductor drum.

The organic optoelectric device includes an anode and a cathode facing each other, and at least one organic layer interposed between the anode and the cathode, wherein the organic layer includes the above organic compound or composition.

Herein, an organic light emitting diode as one example of an organic optoelectric device is described referring to drawings.

FIGS. 1 and 2 are cross-sectional views of each organic light emitting diode according to one embodiment.

Referring to FIG. 1, an organic optoelectric device 100 according to one embodiment includes an anode 120 and a cathode 110 facing each other and an organic layer 105 interposed between the anode 120 and cathode 110.

The anode 120 may be made of a conductor having a large work function to help hole injection, and may be for example metal, metal oxide and/or a conductive polymer. The anode 120 may be, for example a metal or an alloy thereof such as nickel, platinum, vanadium, chromium, copper, zinc, gold, and the like; metal oxide such as zinc oxide, indium oxide, indium tin oxide (ITO), indium zinc oxide (IZO), and the like; a combination of metal and oxide such as ZnO and Al or SnO₂ and Sb; a conductive polymer such as poly(3-methylthiophene), poly(3,4-(ethylene-1,2-dioxy)thiophene) (PEDT), polypyrrole, and polyaniline.

The cathode 110 may be made of a conductor having a small work function to help electron injection, and may be for example metal, metal oxide and/or a conductive polymer. The cathode 110 may be for example a metal or an alloy thereof such as magnesium, calcium, sodium, potassium, titanium, indium, yttrium, lithium, gadolinium, aluminum silver, tin, lead, cesium, barium, and the like; a multi-layer structure material such as LiF/Al, LiO₂/Al, LiF/Ca, LiF/AI and BaF₂/Ca.

The organic layer 105 may include an emission layer 130 including the organic compound or the composition.

The emission layer 130 may include for example the above organic compound at alone, a mixture of at least two kinds of the above organic compound, or the composition.

Referring to FIG. 2, an organic light emitting diode 200 further includes a hole auxiliary layer 140 as well as an emission layer 130. The hole auxiliary layer 140 may further increase hole injection and/or hole mobility between the anode 120 and emission layer 130 and block electrons. The hole auxiliary layer 140 may be, for example a hole transport layer (HTL), a hole injection layer (HIL), and/or an electron blocking layer, and may include at least one layer.

In one embodiment of the present invention, an organic light emitting diode may further include an electron transport layer (ETL), an electron injection layer (EIL), a hole injection layer (HIL), and the like, as an organic layer 105 in FIG. 1 or FIG. 2.

The organic light emitting diodes 100 and 200 may be manufactured by forming an anode or a cathode on a substrate, forming an organic layer in accordance with a dry coating method such as evaporation, sputtering, plasma plating, and ion plating; and forming a cathode or an anode thereon.

The organic light emitting diode may be applied to an organic light emitting diode (OLED) display.

Hereinafter, the embodiments are illustrated in more detail with reference to examples. These examples, however, are not in any sense to be interpreted as limiting the scope of the invention.

### Representative Synthesis Method

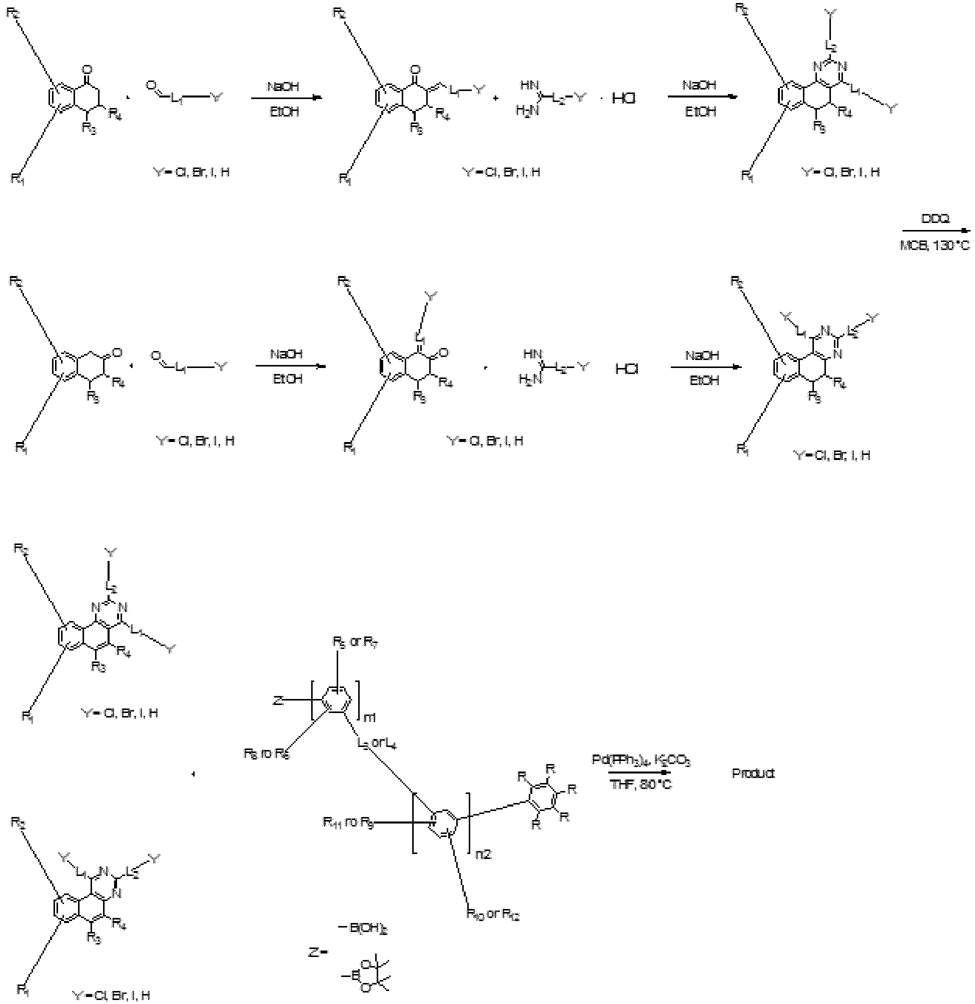

### Synthesis of Intermediate

### Synthesis Example 1: Synthesis of Intermediate I-1

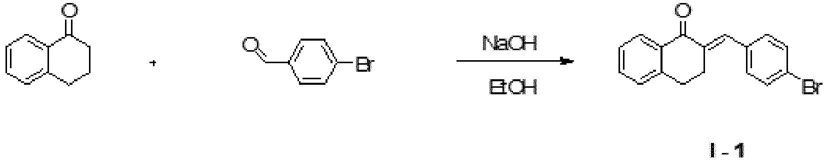

100 g (684 mmol) of α-tetralone was dissolved in 1 L of ethanol under a nitrogen environment, 127 g (684 mmol) of 4-bromobenzaldehyde and 41.0 g (1026 mmol) of sodium hydroxide were added thereto, and the mixture was agitated at room temperature for 2 hours. When the reaction was terminated, the reaction solution was filtered and then, washed with a small amount of ethanol. In this way, 179 g of an intermediate I-1 (a yield: 83 %) was obtained.

HRMS (70 eV, EI+): m/z calcd for C17H13BrO: 312.0150, found: 312.

Elemental Analysis: C, 65 %; H, 4 %

### Synthesis Example 2: Synthesis of Intermediate I-2

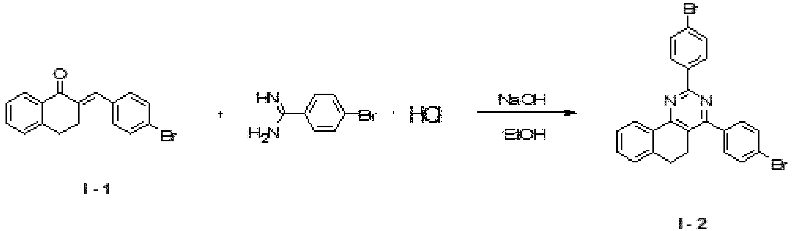

170 g (543 mmol) of the intermediate I-1 was dissolved in 1.5 L of ethanol under a nitrogen environment, 128 g (543 mmol) of 4-bromobenzimidamide hydrochloride and 65.2 g (1,629 mmol) of sodium hydroxide were added thereto, and the mixture was agitated at room temperature for 17 hours. When the reaction was terminated, the reaction solution was filtered and then, washed with a small amount of ethanol. In this way, 120 g of an intermediate I-2 (a yield: 45 %) was obtained.

HRMS (70 eV, EI+): m/z calcd for C24H16Br2N2: 489.9680, found: 490.

Elemental Analysis: C, 59 %; H, 3 %

### Synthesis Example 3: Synthesis of Intermediate I-3

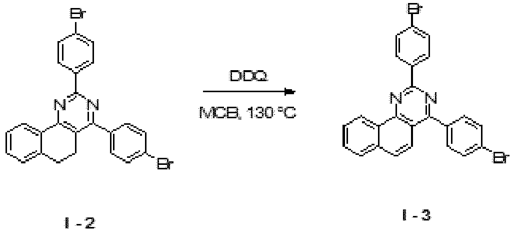

110 g (223 mmol) of the intermediate I-2 was dissolved in 1 L of monochlorobenzene (MCB) under a nitrogen environment, 101 g (446 mmol) of 2,3-dichloro-5,6-dicyano-1,4-benzoquinone (DDQ) was added thereto, and the mixture was heated and refluxed at 130° C for 15 hours. When the reaction was terminated, water was added to the reaction solution, and the mixture was extracted with dichloromethane (DCM), treated with anhydrous MgSO4 to remove moisture and then, filtered and concentrated under a reduced pressure. The obtained residue was separated and purified through flash column chromatography, obtaining 76.5 g of an intermediate I-3 (a yield: 70 %).

HRMS (70 eV, EI+): m/z calcd for C24H14Br2N2: 487.9524, found: 488.

Elemental Analysis: C, 59 %; H, 3 %

### Synthesis Example 4: Synthesis of Intermediate I-4

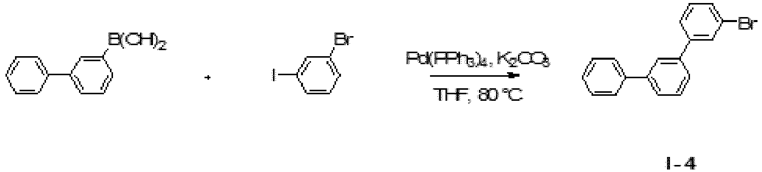

100 g (505 mmol) of biphenyl-3-ylboronic acid was dissolved in 1.4 L of tetrahydrofuran (THF) under a nitrogen environment, 171 g (606 mmol) of 1-bromo-3-iodobenzene and 5.84 g (5.05 mmol) of tetrakis(triphenylphosphine)palladium were added thereto, and the mixture was agitated. 174 g (1,263 mmol) of potassium carbonate saturated in water was added thereto, and the resulting mixture was heated and refluxed at 80° C for 8 hours. When the reaction was terminated, water was added to the reaction solution, and the mixture was extracted with dichloromethane (DCM), treated with anhydrous MgSO4 to remove moisture and then, filtered and concentrated under a reduced pressure. The obtained residue was separated and purified through flash column chromatography, obtaining 141 g of an intermediate I-4 (a yield: 90 %).

HRMS (70 eV, EI+): m/z calcd for C18H13Br: 308.0201, found: 308.

Elemental Analysis: C, 70 %; H, 4 %

### Synthesis Example 5: Synthesis of Intermediate I-5

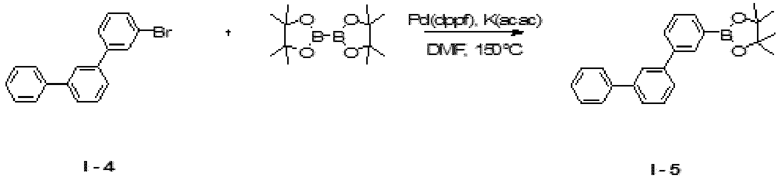

130 g (420 mmol) of the intermediate I-4 was dissolved in 1.3 L of dimethylforamide (DMF) under a nitrogen environment, 128 g (505 mmol) of bis(pinacolato)diboron, 3.43 g (4.2 mmol) of (1,1'-bis(diphenylphosphine)ferrocene)dichloropalladium (II) and 124 g (1,260 mmol) of potassium acetate were added thereto, and the mixture was heated and refluxed at 150° C for 6 hours. When the reaction was terminated, water was added to the reaction solution, and the mixture was filtered and dried in a vacuum oven. Then, the obtained residue was separated and purified through flash column chromatography, obtaining 106 g of an intermediate I-5 (a yield: 71 %).

HRMS (70 eV, EI+): m/z calcd for C24H25BO2: 356.1948, found: 356.

Elemental Analysis: C, 81 %; H, 7 %

### Synthesis Example 6: Synthesis of Intermediate I-6

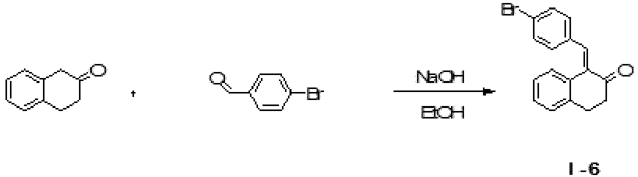

100 g (684 mmol) of β-tetralone was dissolved in 1 L of ethanol under a nitrogen environment, 127 g (684 mmol) of 4-bromobenzaldehyde and 41.0 g (1026 mmol) of sodium hydroxide were added thereto, and the mixture was agitated at room temperature for 2 hours. When the reaction was terminated, the reaction solution was filtered and washed with a small amount of ethanol. In this way, 161 g of an intermediate I-6 (a yield: 75 %) was obtained.

HRMS (70 eV, EI+): m/z calcd for C17H13BrO: 312.0150, found: 312.

Elemental Analysis: C, 65 %; H, 4 %

### Synthesis Example 7: Synthesis of Intermediate I-7

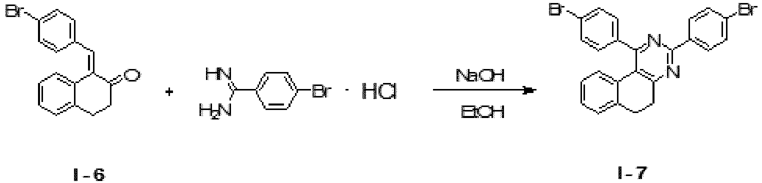

150 g (479 mmol) of the intermediate I-6 was dissolved in 1.5 L of ethanol in a nitrogen environment, 95.3 g (479 mmol) of 4-bromobenzimidamide hydrochloride and 65.2 g (1,437 mmol) of sodium hydroxide were added thereto, and the mixture was agitated at room temperature for 15 hours. When the reaction was terminated, the reaction solution was filtered and washed with a small amount of ethanol. In this way, 91.9 g of an intermediate I-7 (a yield: 39 %) was obtained.

HRMS (70 eV, EI+): m/z calcd for C24H16Br2N2: 489.9680, found: 490.

Elemental Analysis: C, 59 %; H, 3 %

### Synthesis Example 8: Synthesis of Intermediate I-8

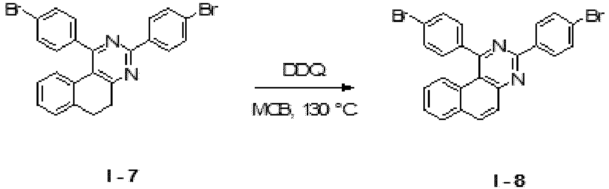

85 g (173 mmol) of the intermediate I-7 was dissolved in 0.8 L of monochlorobenzene (MCB) in a nitrogen environment, 78.4 g (345 mmol) of 2,3-dichloro-5,6-dicyano-1,4-benzoquinone (DDQ) was added thereto, and the mixture was heated and refluxed at 130° C for 15 hours. When the reaction was terminated, water was added to the reaction solution, and the mixture was extracted with dichloromethane (DCM), treated with anhydrous MgSO4 to remove moisture, and then, filtered and concentrated under a reduced pressure. Then, the obtained residue was separated and purified through flash column chromatography, obtaining 57.7 g of an intermediate I-8 (a yield: 68 %).

HRMS (70 eV, EI+): m/z calcd for C24H14Br2N2: 487.9524, found: 488.

Elemental Analysis: C, 59 %; H, 3 %

### Synthesis Example 9: Synthesis of Intermediate I-9

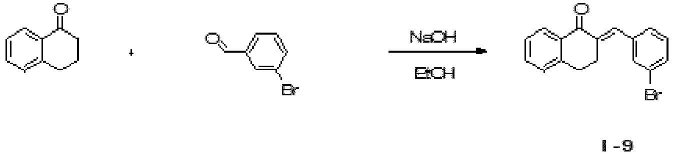

100 g (684 mmol) of α-tetralone was dissolved in 1 L of ethanol in a nitrogen environment, 127 g (684 mmol) of 3-bromobenzaldehyde and 41.0 g (1026 mmol) of sodium hydroxide were added thereto, and the mixture was agitated at room temperature for 2 hours. When the reaction was terminated, the reaction solution was filtered and then, washed with a small amount of ethanol. In this way, 171 g of an intermediate I-9 (a yield: 80 %) was obtained.

HRMS (70 eV, EI+): m/z calcd for C17H13BrO: 312.0150, found: 312.

Elemental Analysis: C, 65 %; H, 4 %

### Synthesis Example 10: Synthesis of Intermediate I-10

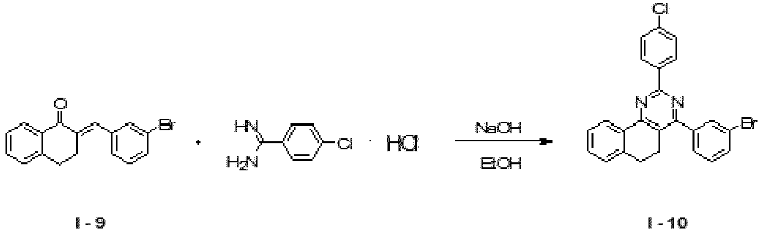

165 g (527 mmol) of the intermediate I-9 was dissolved in 1.5 L of ethanol in a nitrogen environment, 101 g (527 mmol) of 4-chlorobenzimidamide hydrochloride and 63.2 g (1,581 mmol) of sodium hydroxide were added thereto, and the mixture was agitated at room temperature for 15 hours. When the reaction was terminated, the reaction solution was filtered and then, washed with a small amount of ethanol. In this way, 99.1 g of an intermediate 1-10 (a yield: 42 %) was obtained.

HRMS (70 eV, EI+): m/z calcd for C24H16BrCIN2: 446.0185, found: 446.

Elemental Analysis: C, 64 %; H, 4 %

### Synthesis Example 11: Synthesis of Intermediate I-11

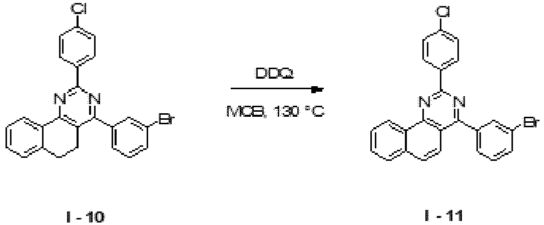

90 g (201 mmol) of the intermediate 1-10 was dissolved in 1 L of monochlorobenzene (MCB) in a nitrogen environment, 91.3 g (402 mmol) of 2,3-dichloro-5,6-dicyano-1,4-benzoquinone (DDQ) was added thereto, and the mixture was refluxed and heated at 130° C for 15 hours. When the reaction was terminated, water was added to the reaction solution, and the mixture was extracted with dichloromethane (DCM), treated with anhydrous MgSO4 to remove moisture and then, filtered and concentrated under a reduced pressure. Then, the obtained residue was separated and purified through flash column chromatography, obtaining 58.2 g of an intermediate 1-11 (a yield: 65 %).

HRMS (70 eV, EI+): m/z calcd for C24H14BrCIN2: 444.0029, found: 444.

Elemental Analysis: C, 65 %; H, 3 %

### Synthesis Example 12: Synthesis of Intermediate I-12

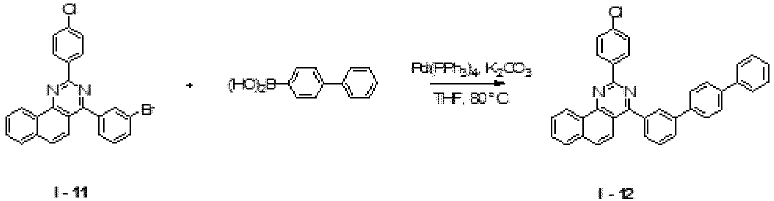

50 g (112 mmol) of the intermediate 1-11 was dissolved in 0.4 L of tetrahydrofuran (THF) in a nitrogen environment, 24.4 g (123 mmol) of biphenyl-4-ylboronic acid and 1.29 g (1.12 mmol) of tetrakis(triphenylphosphine)palladium were added thereto, and the mixture was agitated. 38.7 g (280 mmol) of potassium carbonate saturated in water was added thereto, and the mixture was heated and refluxed at 80° C for 18 hours. When the reaction was terminated, water was added to the reaction solution, and the mixture was extracted with dichloromethane (DCM) and treated with anhydrous MgSO4 to remove moisture and then, filtered and concentrated under a reduced pressure. Then, the obtained residue was separated and purified through flash column chromatography, obtaining 48.2 g of an intermediate 1-12 (a yield: 83 %).

HRMS (70 eV, EI+): m/z calcd for C36H23CIN2: 518.1550, found: 518.

Elemental Analysis: C, 83 %; H, 4 %

### Synthesis Example 13: Synthesis of Intermediate I-13

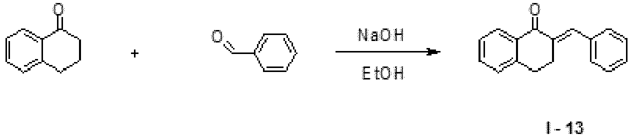

100 g (684 mmol) of α-tetralone was dissolved in 1 L of ethanol in a nitrogen environment, 72.6 g (684 mmol) of benzaldehyde and 41.0 g (1026 mmol) of sodium hydroxide were added thereto, and the mixture was agitated at room temperature for 2 hours. When the reaction was terminated, the reaction solution was filtered and washed with a small amount of ethanol. In this way, 139 g of an intermediate I-13 (a yield: 87 %) was obtained.

HRMS (70 eV, EI+): m/z calcd for C17H14O: 234.1045, found: 234.

Elemental Analysis: C, 87 %; H, 6 %

### Synthesis Example 14: Synthesis of Intermediate I-14

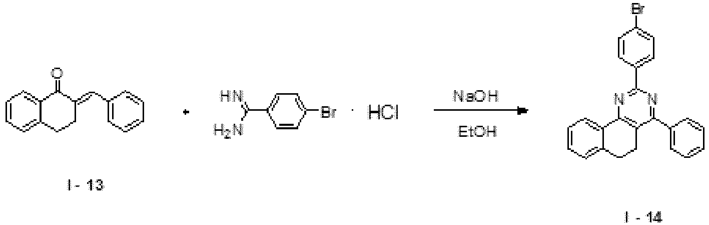

130 g (555 mmol) of the intermediate I-13 was dissolved in 1.5 L of ethanol in a nitrogen environment, 131 g (555 mmol) of 4-bromobenzimidamide hydrochloride and 66.6 g (1,665 mmol) of sodium hydroxide were added thereto, and the mixture was agitated at room temperature for 15 hours. When the reaction was terminated, the reaction solution was filtered and washed with a small amount of ethanol. In this way, 115 g of an intermediate I-14 (a yield: 50 %) was obtained.

HRMS (70 eV, EI+): m/z calcd for C24H17BrN2: 412.0575, found: 412.

Elemental Analysis: C, 70 %; H, 4 %

### Synthesis Example 15: Synthesis of Intermediate I-15

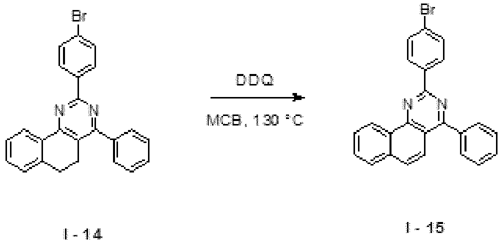

110 g (266 mmol) of the intermediate I-14 was dissolved in 1.2 L of monochlorobenzene (MCB) in a nitrogen environment, 121 g (532 mmol) of 2,3-dichloro-5,6-dicyano-1,4-benzoquinone (DDQ) was added thereto, and the mixture was heated and refluxed at 130° C for 15 hours. When the reaction was terminated, water was added to the reaction solution, and the mixture was extracted with dichloromethane (DCM) and treated with anhydrous MgSO4 to remove moisture and then, filtered and concentrated under a reduced pressure. Then, the obtained residue was separated and purified through flash column chromatography, obtaining 72.2 g (66 %) of an intermediate I-15.

HRMS (70 eV, EI+): m/z calcd for C24H15BrN2: 410.0419, found: 410.

Elemental Analysis: C, 70 %; H, 4 %

### Synthesis Example 16: Synthesis of Intermediate I-16

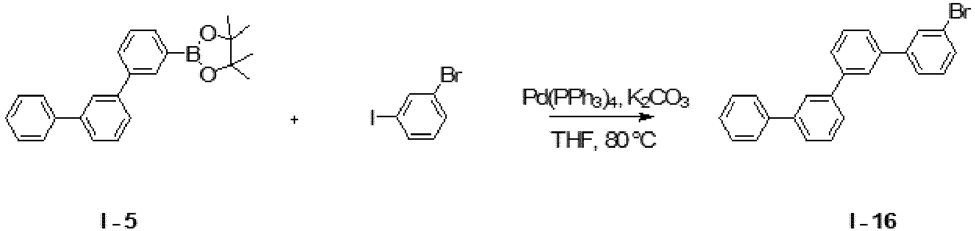

100 g (281 mmol) of the intermediate I-5 was dissolved in 1 L of tetrahydrofuran (THF) in a nitrogen environment, 95.3 g (337 mmol) of 1-bromo-3-iodobenzene and 3.25 g (2.81 mmol) of tetrakis(triphenylphosphine)palladium were added thereto, and the mixture was agitated. 97.1 g (703 mmol) of potassium carbonate saturated in water was added thereto, and the resulting mixture was heated and refluxed at 80° C for 11 hours. When the reaction was terminated, water was added to the reaction solution, and the mixture was extracted with dichloromethane (DCM), treated with anhydrous MgSO4 to remove moisture and then, filtered and concentrated under a reduced pressure.

Then, the obtained residue was separated and purified through flash column chromatography, obtaining 92.0 g of an intermediate I-16 (a yield: 85 %).

HRMS (70 eV, EI+): m/z calcd for C24H17Br: 384.0514, found: 384.

Elemental Analysis: C, 75 %; H, 4 %

### Synthesis Example 17: Synthesis of Intermediate I-17

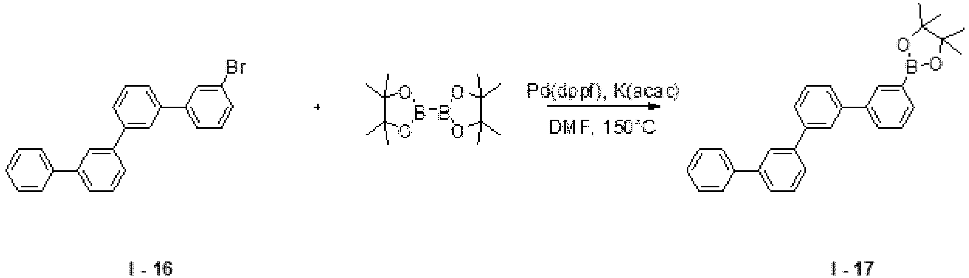

85 g (221 mmol) of the intermediate I-16 was dissolved in 0.8 L of dimethylforamide (DMF) in a nitrogen environment, 67.2 g (265 mmol) of bis(pinacolato)diboron, 1.80 g (2.21 mmol) of (1,1'-bis(diphenylphosphine)ferrocene)dichloropalladium (II) and 65.1 g (663 mmol) of potassium acetate were added thereto, and the mixture was heated and refluxed at 150° C for 5 hours. When the reaction was terminated, water was added to the reaction solution, and the mixture was filtered and then, dried in a vacuum oven. Then, the obtained residue was separated and purified through flash column chromatography, obtaining 74.5 g of an intermediate I-17 (a yield: 78 %).

HRMS (70 eV, EI+): m/z calcd for C30H29BO2: 432.2261, found: 432.

Elemental Analysis: C, 83 %; H, 7 %

### Synthesis Example 18: Synthesis of Intermediate I-18

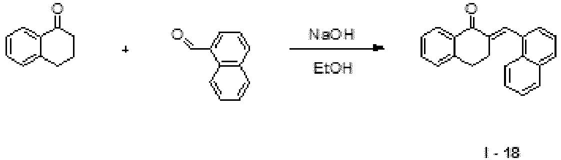

100 g (684 mmol) of α-tetralone was dissolved in 1 L of ethanol in a nitrogen environment, 107 g (684 mmol) of 1-naphthaldehyde and 41.0 g (1026 mmol) of sodium hydroxide were added thereto, and the mixture was agitated at room temperature for 2 hours. When the reaction was terminated, the reaction solution was filtered and washed with a small amount of ethanol. In this way, 173 g of an intermediate I-18 (a yield: 89 %) was obtained.

HRMS (70 eV, EI+): m/z calcd for C21H6O: 284.1201, found: 284.

Elemental Analysis: C, 89 %; H, 6 %

### Synthesis Example 19: Synthesis of Intermediate I-19

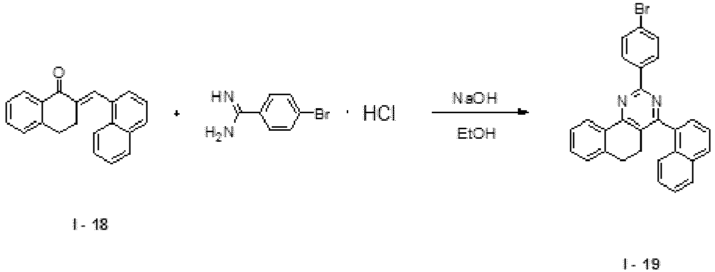

170 g (598 mmol) of the intermediate I-18 was dissolved in 1.5 L of ethanol in a nitrogen environment, 141 g (598 mmol) of 4-bromobenzimidamide hydrochloride and 71.8 g (1,794 mmol) of sodium hydroxide were added thereto, and the mixture was agitated at room temperature for 15 hours. When the reaction was terminated, the reaction solution was filtered and then, washed with a small amount of ethanol. In this way, 114 g of an intermediate I-19 (a yield: 41 %) was obtained.

HRMS (70 eV, EI+): m/z calcd for C28H19BrN2: 462.0732, found: 462.

Elemental Analysis: C, 73 %; H, 4 %

### Synthesis Example 20: Synthesis of Intermediate I-20

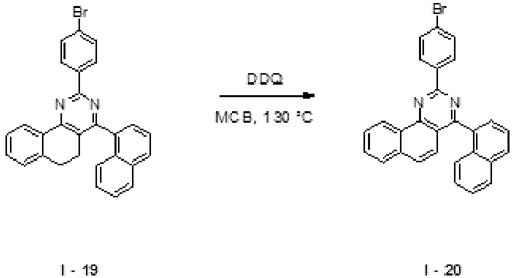

105 g (227 mmol) of the intermediate I-19 was dissolved in 1 L of monochlorobenzene (MCB) in a nitrogen environment, 103 g (453 mmol) of 2,3-dichloro-5,6-dicyano-1,4-benzoquinone (DDQ) was added thereto, and the mixture was heated and refluxed at 130° C for 15 hours. When the reaction was terminated, water was added to the reaction solution, and the mixture was extracted with dichloromethane (DCM) and treated with anhydrous MgSO4 to remove moisture and then, filtered and concentrated under a reduced pressure. Then, the obtained residue was separated and purified through flash column chromatography, obtaining 68.1 g of an intermediate I-20 (a yield: 65 %).

HRMS (70 eV, EI+): m/z calcd for C28H17BrN2: 460.0575, found: 460.

Elemental Analysis: C, 73 %; H, 4 %

### Synthesis of Final Compound

### Synthesis Example 21: Synthesis of Compound 1

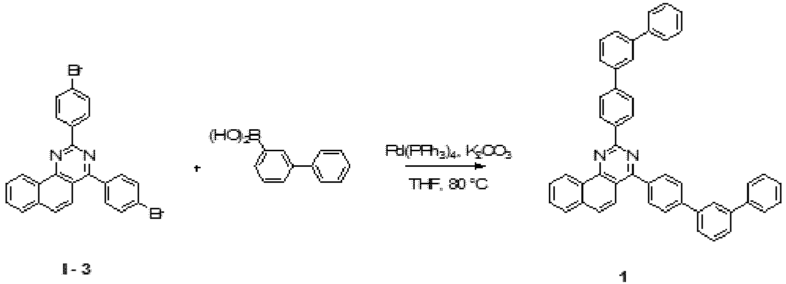

20 g (40.8 mmol) of the intermediate I-3 was dissolved in 0.2 L of tetrahydrofuran (THF) in a nitrogen environment, 16.2 g (81.6 mmol) of biphenyl-3-ylboronic acid and 0.94 g (0.82 mmol) of tetrakis(triphenylphosphine)palladium were added thereto, and the mixture was agitated. 28.2 g (204 mmol) of potassium carbonate saturated in water was added thereto, and the mixture was heated and refluxed at 80° C for 12 hours. When the reaction was terminated, water was added to the reaction solution, and the mixture was extracted with dichloromethane (DCM) and treated with anhydrous MgSO4 to remove moisture and then, filtered and concentrated under a reduced pressure. Then, the obtained residue was separated and purified through flash column chromatography, obtaining 24.9 g of a compound 1 (a yield: 96 %).

HRMS (70 eV, EI+): m/z calcd for C48H32N2: 636.2565, found: 636.

Elemental Analysis: C, 91 %; H, 5 %

### Synthesis Example 22: Synthesis of Compound 2

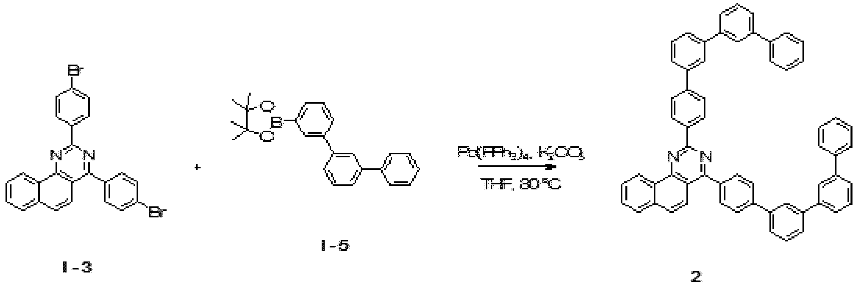

20 g (40.8 mmol) of the intermediate I-3 was dissolved in 0.2 L of tetrahydrofuran (THF) in a nitrogen environment, 29.1 g (81.6 mmol) of the intermediate I-5 and 0.94 g (0.82 mmol) of tetrakis(triphenylphosphine)palladium were added thereto, and the mixture was agitated. 28.2 g (204 mmol) of potassium carbonate saturated in water was added thereto, and the mixture was heated and refluxed at 80° C for 15 hours. When the reaction was terminated, water was added to the reaction solution, and the mixture was extracted with dichloromethane (DCM) and treated with anhydrous MgSO4 to remove moisture and then, filtered and concentrated under a reduced pressure. Then, the obtained residue was separated and purified through flash column chromatography, obtaining 29.6 g of a compound 2 (a yield: 92 %).

HRMS (70 eV, EI+): m/z calcd for C60H40N2: 788.3191, found: 788.

Elemental Analysis: C, 91 %; H, 5 %

### Synthesis Example 23: Synthesis of Compound 5

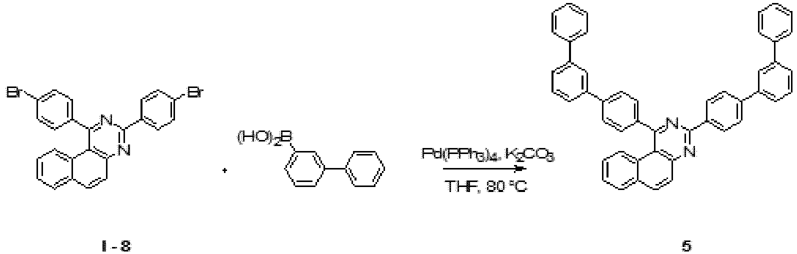

20 g (40.8 mmol) of the intermediate I-8 was dissolved in 0.2 L of tetrahydrofuran (THF) in a nitrogen environment, 16.2 g (81.6 mmol) of biphenyl-3-yl boronic acid and 0.94 g (0.82 mmol) of tetrakis(triphenylphosphine)palladium were added thereto, and the mixture was agitated. 28.2 g (204 mmol) of potassium carbonate saturated in water was added thereto, and the mixture was heated and refluxed at 80° C for 13 hours. When the reaction was terminated, water was added to the reaction solution, and the mixture was extracted with dichloromethane (DCM) and treated with anhydrous MgSO4 and then, filtered and concentrated under a reduced pressure. Then, the obtained residue was separated and purified through flash column chromatography, obtaining 24.7 g of a compound 5 (a yield: 95 %).

HRMS (70 eV, EI+): m/z calcd for C48H32N2: 636.2565, found: 636.

Elemental Analysis: C, 91 %; H, 5 %

### Synthesis Example 24: Synthesis of Compound 6

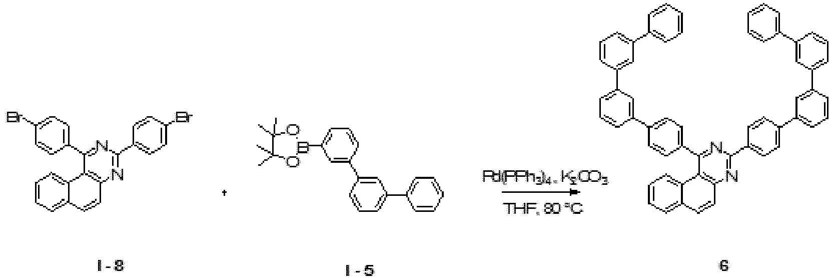

20 g (40.8 mmol) of the intermediate I-8 was dissolved in 0.2 L of tetrahydrofuran (THF) in a nitrogen environment, 29.1 g (81.6 mmol) of the intermediate I-5 and 0.94 g (0.82 mmol) of tetrakis(triphenylphosphine)palladium were added thereto, and the mixture was agitated. 28.2 g (204 mmol) of potassium carbonate saturated in water was heated and refluxed at 80° C for 15 hours. When the reaction was terminated, water was added to the reaction solution, and the mixture was extracted with dichloromethane (DCM) and treated with anhydrous MgSO4 to remove moisture and then, filtered and concentrated under a reduced pressure. Then, the obtained residue was separated and purified through flash column chromatography, obtaining 29.0 g of a compound 6 (a yield: 90 %).

HRMS (70 eV, EI+): m/z calcd for C60H40N2: 788.3191, found: 788.

Elemental Analysis: C, 91 %; H, 5 %

### Synthesis Example 25: Synthesis of Compound 19

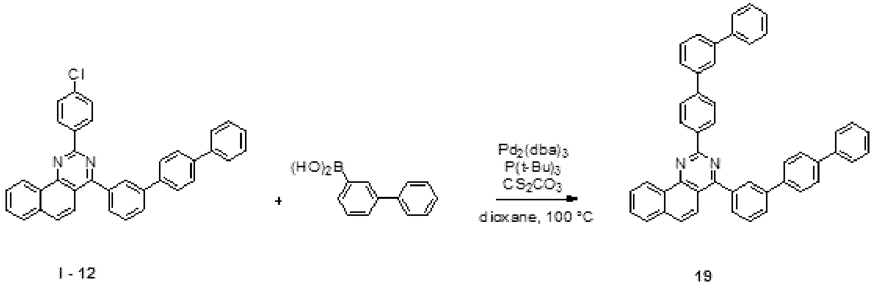

20 g (38.5 mmol) of the intermediate I-12 was dissolved in 0.15 L of dioxane in a nitrogen environment, 7.63 g (38.5 mmol) of biphenyl-3-ylboronic acid, 0.36 g (0.39 mmol) of tris(diphenylideneacetone)dipalladium (0), 0.39 g (1.95 mmol) of tris-tert butylphosphine and 31.4 g (96.3 mmol) of caesium carbonate were sequentially added thereto, and the mixture was heated and refluxed at 100° C for 18 hours. When the reaction was terminated, water was added to the reaction solution, and the mixture was extracted with dichloromethane (DCM) and treated with anhydrous MgSO4 and then, filtered and concentrated under a reduced pressure. Then, the obtained residue was separated and purified through flash column chromatography, obtaining 22.1 g of a compound 19 (a yield: 90 %).

HRMS (70 eV, EI+): m/z calcd for C48H32N2: 636.2565, found: 636.

Elemental Analysis: C, 91 %; H, 5 %

### Synthesis Example 26: Synthesis of Compound 66

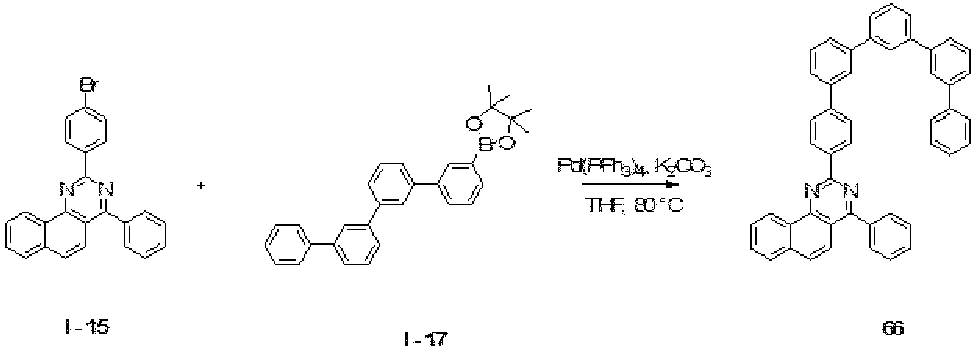

20 g (48.6 mmol) of the intermediate I-15 was dissolved in 0.2 L of tetrahydrofuran (THF) in a nitrogen environment, 21.0 g (48.6 mmol) of the intermediate I-17 and 0.57 g (0.49 mmol) of tetrakis(triphenylphosphine)palladium were added thereto, and the mixture was agitated. 16.8 g (122 mmol) of potassium carbonate saturated in water was added thereto, and the mixture was heated and refluxed at 80° C for 18 hours. When the reaction was terminated, water was added to the reaction solution, and the mixture was extracted with dichloromethane (DCM) and treated with anhydrous MgSO4 to remove moisture and then, filtered and concentrated under a reduced pressure. Then, the obtained residue was separated and purified through flash column chromatography, obtaining 27.2 g of a compound 66 (a yield: 88 %).

HRMS (70 eV, EI+): m/z calcd for C48H32N2: 636.2565, found: 636.

Elemental Analysis: C, 91 %; H, 5 %

### Synthesis Example 27: Synthesis of Compound 114

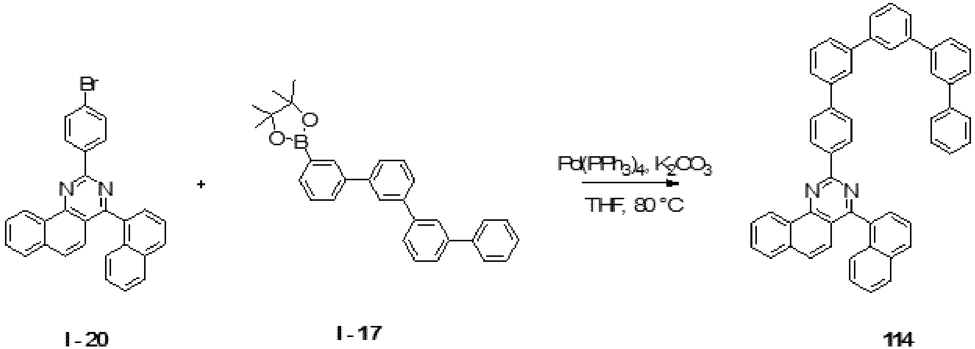

20 g (43.4 mmol) of the intermediate I-20 was dissolved in 0.2 L of tetrahydrofuran (THF) in a nitrogen environment, 18.7 g (43.4 mmol) of the intermediate I-17 and 0.50 g (0.43 mmol) of tetrakis(triphenylphosphine)palladium were added thereto, and the mixture was agitated. 15.0 g (109 mmol) of potassium carbonate saturated in water was added thereto, and the mixture was heated and refluxed at 80° C for 16 hours. When the reaction was terminated, water was added to the reaction solution, and the mixture was extracted with dichloromethane (DCM) and treated with anhydrous MgSO4 to remove moisture and then, filtered and concentrated under a reduced pressure. Then, the obtained residue was separated and purified through flash column chromatography, obtaining 23.8 g of a compound 114 (a yield: 80 %).

HRMS (70 eV, EI+): m/z calcd for C52H34N2: 686.2722, found: 686.

Elemental Analysis: C, 91 %; H, 5 %

### Manufacture of Organic Light Emitting Diode

### Example 1

The compound 1 obtained in Synthesis Example 21 was used as a host, and acetylacetonatobis (2-phenylquinolinato)iridium (Ir(pq)2acac) was used as a dopant to manufacture an organic light emitting diode.

A 1500 Å-thick ITO was used as an anode, and a 1000 Å-thick aluminum (Al) was used as a cathode. Specifically, an organic light emitting diode was manufactured by manufacturing the anode by cutting an ITO glass substrate having 15 Ω/cm² of sheet resistance into a size of 50 mm × 50 mm × 0.7 mm and cleaning with a ultrasonic wave in acetone, isopropyl alcohol, and pure water respectively for 15 minutes and with UV ozone for 30 minutes.

On the upper side of a substrate, a 600 Å-thick hole injection layer (HIL) was formed by vacuum-depositing 4,4' -bis[N-[4-{N,N-bis (3-methylphenyl)amino}-phenyl]-N-phenylamino]biphenyl [DNTPD] under a vacuum degree of 650 × 10⁻⁷ Pa at a deposition rate ranging from 0.1 to 0.3 nm/s. Subsequently, a 300 Å-thick hole transport layer (HTL) was formed by vacuum-depositing HT-1 under the same vacuum deposition condition. Then, a 300 Å-thick emission layer was formed by vacuum-depositing the compound 1 of Synthesis Example 21 under the same vacuum deposition condition, and a phosphorescent dopant, acetylacetonatobis (2-phenylquinolinato)iridium (Ir(pq)2acac) was simultaneously deposited. Herein, 7 wt% of the phosphorescent dopant was deposited by adjusting a deposition rate of the phosphorescent dopant based on 100 wt% of the total amount of the emission layer.

On the emission layer, a 50 Å-thick hole blocking layer was formed by depositing bis (2-methyl-8-quinolinolate)-4-(phenylphenolato)aluminium (BAlq) under the same vacuum deposition condition. Subsequently, a 250 Å-thick electron transport layer (ETL) was formed by depositing tris (8-hydroxyquinolinato)aluminium (Alq3) under the same vacuum deposition condition. LiF and Al were sequentially deposited to form a cathode on the electron transport layer (ETL), manufacturing an organic light emitting diode .

The organic light emitting diode has a structure of ITO/DNTPD 60nm/HT-1 30 nm/EML (a compound 1 (93 wt%) + Ir(pq)2acac (7 wt%), 30nm)/Balq (5 nm)/Alq3 25 nm/LiF (1 nm)/AI (100 nm).

### Example 2

An organic light emitting diode was manufactured according to the same method as Example 1 except for using the compound 2 according to Synthesis Example 22 instead of the compound 1 according to Synthesis Example 21.

### Example 3

An organic light emitting diode was manufactured according to the same method as Example 1 except for using the compound 5 according to Synthesis Example 23 instead of the compound 1 according to Synthesis Example 21.

### Example 4

An organic light emitting diode was manufactured according to the same method as Example 1 except for using the compound 6 according to Synthesis Example 24 instead of the compound 1 according to Synthesis Example 21.

### Example 5

An organic light emitting diode was manufactured according to the same method as Example 1 except for using the compound 19 according to Synthesis Example 25 instead of the compound 1 according to Synthesis Example 21.

### Example 6

An organic light emitting diode was manufactured according to the same method as Example 1 except for using the compound 66 according to Synthesis Example 26 instead of the compound 1 according to Synthesis Example 21.

### Example 7

An organic light emitting diode was manufactured according to the same method as Example 1 except for using the compound 114 according to Synthesis Example 27 instead of the compound 1 according to Synthesis Example 21.

### Comparative Example 1

An organic light emitting diode was manufactured according to the same method as Example 1 except for using 4,4'-di (9H-carbazol-9-yl)biphenyl (CBP) instead of the compound 1 according to Synthesis Example 21.

The structures of DNTPD, BAlq, HT-1, CBP, Ir(pq)2acac, and Alq3 used to manufacture the organic light emitting diode are as follows.

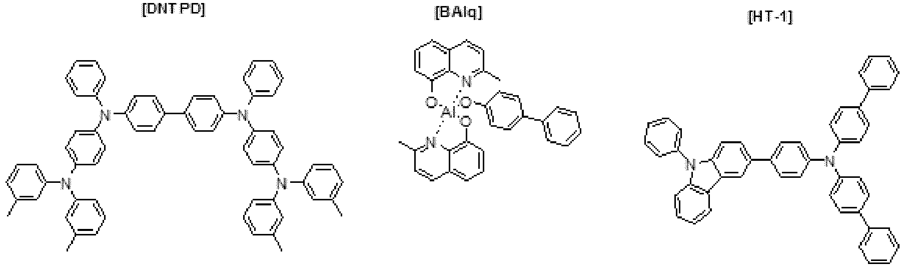

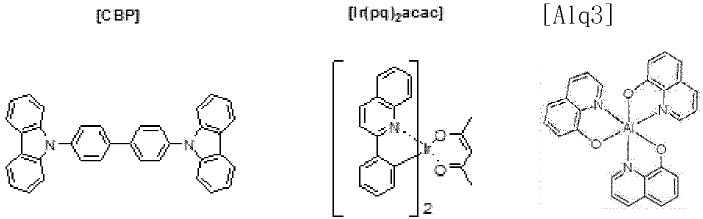

### Evaluation

Current density and luminance changes depending on a voltage and luminous efficiency of each organic light emitting diode according to Examples 1 to 7 and Comparative Example 1 were measured.

The measurements were specifically performed in the following method, and the results were provided in the following Table 1.
(1) Measurement of Current Density Change Depending on Voltage Change
   Current values flowing in the unit device of the manufactured organic light emitting diodes were measured for, while increasing the voltage from 0V to 10V using a current-voltage meter (Keithley 2400), and the measured current values were divided by an area to provide the results.
(2) Measurement of Luminance Change Depending on Voltage Change
   Luminance of the manufactured organic light emitting diodes was measured for luminance, while increasing the voltage from 0V to 10V using a luminance meter (Minolta Cs-1000A).
(3) Measurement of Luminous Efficiency
   Current efficiency (cd/A) at the same current density (10 mA/cm²) were calculated by using the luminance, current density, and voltages (V) from the items (1) and (2).
(4) Measurement of Life-span
   Life-span of the manufactured organic light emitting diodes was obtained by emitting light with 3000 cd/m² in the initial luminance (cd/m²), measuring luminance decrease as time goes and measuring time taken until the luminance decreased by 90 % relative to the initial luminance.

**[Table 1]**

| Nos. | Compound | Driving voltage (V) | Color (EL color) | Efficiency (cd/A) | 90 % life-span (h) at 3000 cd/m² |
|---|---|---|---|---|---|
| Example 1 | compound 1 | 7.0 | Red | 48.4 | 75 |
| Example 2 | compound 2 | 6.9 | Red | 46.5 | 100 |
| Example 3 | compound 5 | 7.1 | Red | 47.2 | 72 |
| Example 4 | compound 6 | 7.0 | Red | 47.0 | 97 |
| Example 5 | compound 19 | 6.9 | Red | 49.1 | 76 |
| Example 6 | compound 66 | 7.0 | Red | 47.9 | 60 |
| Example 7 | compound 114 | 7.1 | Red | 45.5 | 81 |
| Comparative Example 1 | CBP | 7.4 | Red | 37.2 | 50 |

Referring to Table 1, the organic light emitting diodes according to Examples 1 to 7 showed remarkably improved luminous efficiency and life-span characteristics compared with the organic light emitting diode according to Comparative Example 1.

### Synthesis Examples of Second Host Compound

### Synthesis Example 1 of Second Host Compound: Synthesis of Intermediate I-30

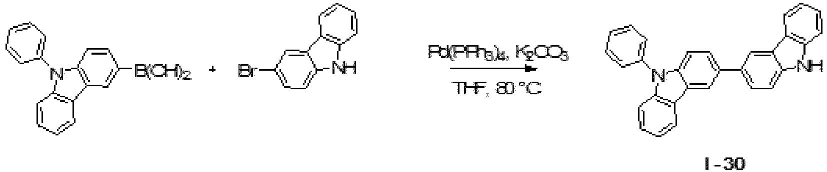

100 g (348 mmol) of 9-phenyl-9H-carbazol-3-ylboronic acid was dissolved in 0.93 L of tetrahydrofuran (THF) in a nitrogen environment, 85.6 g (348 mmol) of 3-bromo-9H-carbazole and 4.02 g (3.48 mmol) of tetrakis(triphenylphosphine)palladium were added thereto, and the mixture was agitated. Then, 120 g (870 mmol) of potassium carbonate was added thereto, and the mixture was heated and refluxed at 80° C for 10 hours. When the reaction was terminated, water was added to the reaction solution, and the mixture was extracted with dichloromethane (DCM) and treated with anhydrous MgSO4 to remove moisture and then, filtered and concentrated under a reduced pressure. Then, the obtained residue was separated and purified through flash column chromatography, obtaining 85.3 g of a compound I-30 (a yield: 60 %).

HRMS (70 eV, EI+): m/z calcd for C30H20N2: 408.1626, found: 408.

Elemental Analysis: C, 88 %; H, 5 %

### Synthesis Example 2 of Second Host Compound: Synthesis of Intermediate I-31

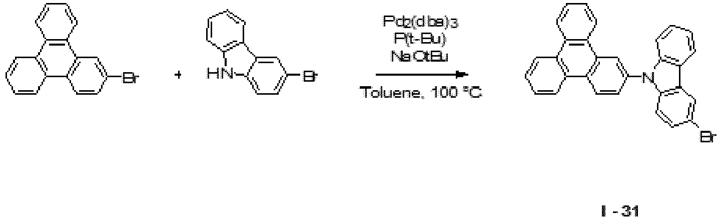

100 g (326 mmol) of 2-bromotriphenylene was dissolved in 0.18 L of toluene in a nitrogen environment, 80.1 g (326 mmol) of 3-bromo-9H-carbazole, 2.99 g (3.26 mmol) of tris(diphenylideneacetone)dipalladium (0), 2.64 g (13.0 mmol) of tris-tert butylphosphine and 37.6 g (391 mmol) of sodium tert-butoxide were sequentially added thereto, and the mixture was heated and refluxed at 100° C for 15 hours. When the reaction was terminated, water was added to the reaction solution, and the mixture was extracted with dichloromethane (DCM) and treated with anhydrous MgSO4 to remove moisture and then, filtered and concentrated under a reduced pressure. Then, the obtained residue was separated and purified through flash column chromatography, obtaining 109 g of a compound I-31 (a yield: 71 %).

HRMS (70 eV, EI+): m/z calcd for C30H18BrN: 471.0623, found: 471.

Elemental Analysis: C, 76 %; H, 4 %

### Synthesis Example 3 of Second Host Compound: Synthesis of Intermediate I-32

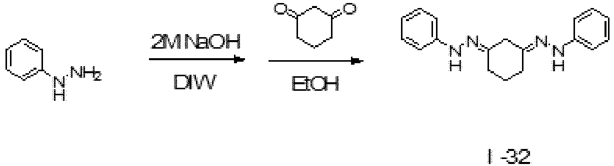

100 g (925 mmol) of phenylhydrazine hydrochloride was dissolved in 0.5 L of distilled water in a nitrogen environment, and a 2M NaOH aqueous solution was added thereto. A solid produced therein was filtered, obtaining phenylhydrazine. Then, the obtained phenylhydrazine was slowly added to the solution that 51.8 g (462 mmol) of cyclohexane-1,3-dione was dissolved in 1.0 L of ethanol, and the mixture was reacted for 20 minutes. When the reaction was terminated, ice water was added thereto. Then, a solid produced therein was filtered while washed with ethanol. The filtered product was dried under a reduced pressure, obtaining 108 g of a compound I-32 (a yield: 40 %).

HRMS (70 eV, EI+): m/z calcd for C18H20N4: 292.1688, found: 292

Elemental Analysis: C, 74 %; H, 7%

### Synthesis Example 4 of Second Host Compound: Synthesis of Intermediate I-33

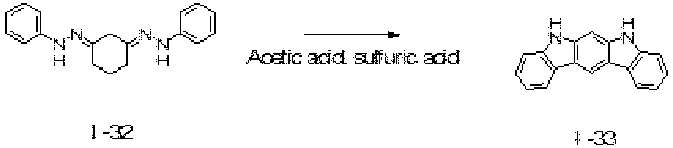

100 g (342 mmol) of the compound I-32 was slowly added to 0.5 L of a mixed solution of acetic acid and sulfuric acid in a ratio of 1:4 under a nitrogen environment at 0° C. The mixture was agitated for 5 minutes and then, rapidly heated up to 50° C and slowly heated up to 110° C. Twenty minutes later, the resultant was cooled down to room temperature and agitated for 12 hours. 0.5 L of ethanol was added thereto, and a solid produced one hour later was filtered under a reduced pressure and neutralized. The solid was dried under a reduced pressure, obtaining 43.8 g of the compound I-33 (a yield: 50 %).

HRMS (70 eV, EI+): m/z calcd for C18H12N2: 256.1000, found: 256

Elemental Analysis: C, 84 %; H, 5 %

### Synthesis Example 5 of Second Host Compound: Synthesis of Compound C-10

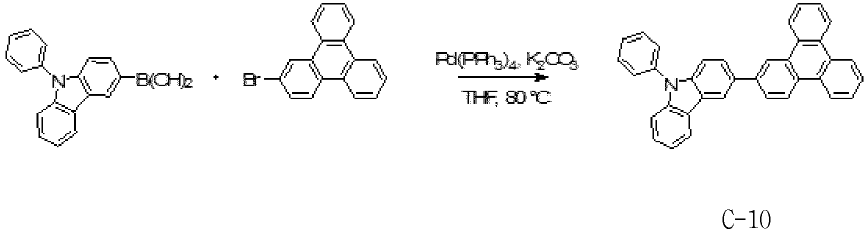

20 g (69.7 mmol) of 9-phenyl-9H-carbazol-3-ylboronic acid was dissolved in 0.21 L of tetrahydrofuran (THF) in a nitrogen environment, 21.4 g (69.7 mmol) of 2-bromotriphenylene and 0.81 g (0.70 mmol) of tetrakis(triphenylphosphine)palladium were added thereto, and the mixture was agitated. Then, 24.1 g (174 mmol) of potassium carbonate saturated in water was added thereto, and the mixture was heated and refluxed at 80° C for 8 hours. When the reaction was terminated, water was added to the reaction solution, and the mixture was extracted with dichloromethane (DCM) and treated with anhydrous MgSO4 to remove moisture and then, filtered and concentrated under a reduced pressure. Then, the obtained residue was separated and purified through flash column chromatography, obtaining 29.5 g of a compound C-10 (a yield: 90 %).

HRMS (70 eV, EI+): m/z calcd for C36H23N: 469.1830, found: 469.

Elemental Analysis: C, 92 %; H, 5 %

### Synthesis Example 6 of Second Host Compound: Synthesis of Compound B-10

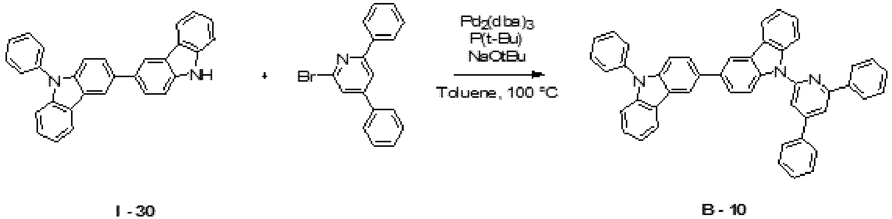

20 g (49.0 mmol) of the intermediate I-30 was dissolved in 0.18 L of toluene in a nitrogen environment, 15.1 g (49.0mmol) of 2-bromo-4,6-diphenylpyridine made by Amadis Chemical (http://www.amadischem.com/), 0.45 g (0.49 mmol) of tris(diphenylideneacetone)dipalladium (0), 0.40 g (1.96 mmol) of tris-tert butylphosphine and 5.65 g (58.8 mmol) of sodium tert-butoxide were sequentially added thereto, and the mixture was heated and refluxed at 100° C for 18 hours. When the reaction was terminated, water was added to the reaction solution, and the mixture was extracted with dichloromethane (DCM) and treated with anhydrous MgSO4 to remove moisture and then, filtered and concentrated under a reduced pressure. Then, the obtained residue was separated and purified through flash column chromatography, obtaining 31.3 g (77 %) of a compound B-10.

HRMS (70 eV, EI+): m/z calcd for C47H31N3: 637.2518, found: 637.

Elemental Analysis: C, 89 %; H, 5 %

### Synthesis Example 7 of Second Host Compound: Synthesis of Compound B-31

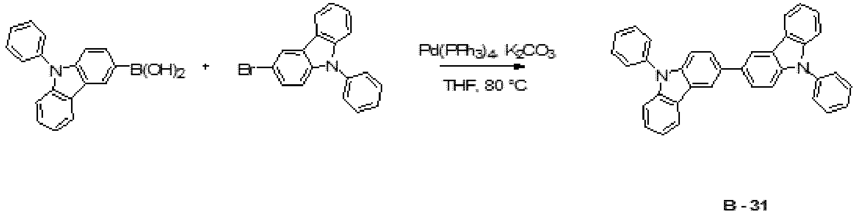

20 g (69.7 mmol) of 9-phenyl-9H-carbazol-3-ylboronic acid was dissolved in 0.21 L of tetrahydrofuran (THF) in a nitrogen environment, 22.5 g (69.7 mmol) of 3-bromo-9-phenyl-9H-carbazole and 0.81 g (0.70 mmol) of tetrakis(triphenylphosphine)palladium were added thereto, and the mixture was agitated. Then, 24.1 g (174 mmol) of potassium carbonate saturated in water was heated and refluxed at 80° C for 12 hours. When the reaction was terminated, water was added to the reaction solution, and the mixture was extracted with dichloromethane (DCM) and treated with anhydrous MgSO4 to remove moisture and then, filtered and concentrated under a reduced pressure. Then, the obtained residue was separated and purified through flash column chromatography, obtaining 31.1 g of a compound B-31 (a yield: 92 %).

HRMS (70 eV, EI+): m/z calcd for C36H24N2: 484.1939, found: 484.

Elemental Analysis: C, 89 %; H, 5 %

### Synthesis Example 8 of Second Host Compound: Synthesis of Compound B-34

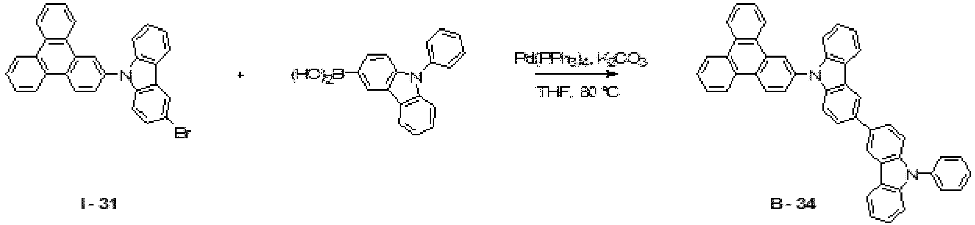

20 g (42.3 mmol) of the intermediate I-31 was dissolved in 0.16 L of tetrahydrofuran (THF) in a nitrogen environment, 12.1 g (42.3 mmol) of 9-phenyl-9H-carbazol-3-ylboronic acid and 0.49 g (0.42 mmol) of tetrakis(triphenylphosphine)palladium were added thereto, and the mixture was agitated. Then, 14.7 g (106 mmol) of potassium carbonate saturated in water was added thereto, and the mixture was heated and refluxed at 80° C for 16 hours. When the reaction was terminated, water was added to the reaction solution, and the mixture was extracted with dichloromethane (DCM) and treated with anhydrous MgSO4 to remove moisture and then, filtered and concentrated under a reduced pressure. Then, the obtained residue was separated and purified through flash column chromatography, obtaining 22.8 g (85 %) of a compound B-34.

HRMS (70 eV, EI+): m/z calcd for C48H30N2: 634.2409, found: 634.

Elemental Analysis: C, 91 %; H, 5 %

### Synthesis Example 9 of Second Host Compound: Synthesis of Compound B-43

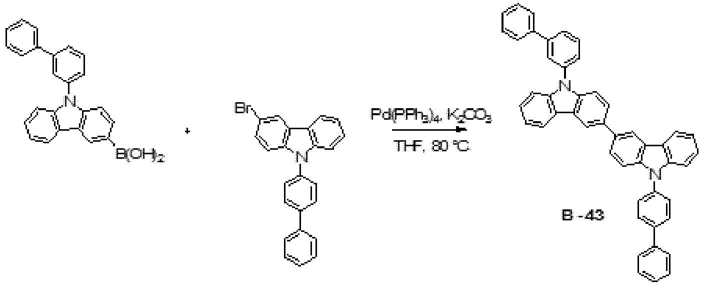

20 g (55.1 mmol) of 9-(biphenyl-3-yl)-9H-carbazol-3-ylboronic acid made by Amadis Chemical (http://www.amadischem.com/) was dissolved in 0.21 L of tetrahydrofuran (THF) in a nitrogen environment, 21.9 g (55.1 mmol) of 9-(biphenyl-4-yl)-3-bromo-9H-carbazole made by Amadis Chemical and 0.64 g (0.55 mmol) of tetrakis(triphenylphosphine)palladium were added thereto, and the mixture was agitated. Then, 19.0 g (138 mmol) of potassium carbonate saturated in water was added thereto, and the mixture was heated and refluxed at 80° C for 18 hours. When the reaction was terminated, water was added to the reaction solution, and the mixture was extracted with dichloromethane (DCM) and treated with anhydrous MgSO4 to remove moisture and then, filtered and concentrated under a reduced pressure. Then, the obtained residue was separated and purified through flash column chromatography, obtaining 33.7 g of a compound B-43 (a yield: 96 %).

HRMS (70 eV, EI+): m/z calcd for C48H32N2: 636.2565, found: 636.

Elemental Analysis: C, 91 %; H, 5 %

### Synthesis Example 10 of Second Host Compound: Synthesis of Compound E-1

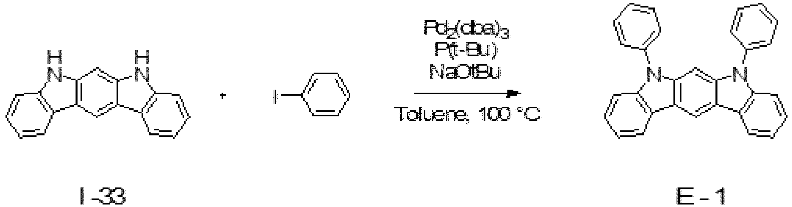

20 g (78.0 mmol) of the intermediate I-33 was dissolved in 0.26 L of toluene in a nitrogen environment, 31.8 g (156 mmol) of iodobenzene, 0.71 g (0.78 mmol) of tris(diphenylideneacetone)dipalladium (0), 0.63 g (3.12 mmol) of tris-tert butylphosphine and 8.99 g (93.6 mmol) of sodium tert-butoxide were sequentially added thereto, and the mixture was heated and refluxed at 100° C for 10 hours. When the reaction was terminated, water was added to the reaction solution, and the mixture was extracted with dichloromethane (DCM) and treated with anhydrous MgSO4 to remove moisture and then, filtered and concentrated under a reduced pressure. Then, the obtained residue was separated and purified through flash column chromatography, obtaining 25.5 g of a compound E-1 (a yield: 80 %).

HRMS (70 eV, EI+): m/z calcd for C30H20N2: 408.1626, found: 408.

Elemental Analysis: C, 88 %; H, 5 %

### Manufacture of Organic Light Emitting Diode Using Second Host Example 8

A 1500 Å-thick ITO was used as an anode, and a 1000 Å-thick aluminum (Al) was used as a cathode. Specifically, an organic light emitting diode was manufactured by manufacturing the anode by cutting an ITO glass substrate having 15 Ω/cm² of sheet resistance into a size of 50 mm ×50 mm × 0.7 mm and cleaning with a ultrasonic wave in acetone, isopropyl alcohol, and pure water respectively for 15 minutes and with UV ozone for 30 minutes.

On the upper side of a substrate, a 600 Å-thick hole injection layer (HIL) was formed by vacuum-depositing 4,4'-bis[N-[4-{N,N-bis(3-methylphenyl)amino}-phenyl]-N-phenylamino]biphenyl [DNTPD] under a vacuum degree of 650 × 10⁻⁷ Pa at a deposition rate ranging from 0.1 to 0.3 nm/s. Subsequently, a 300 Å-thick hole transport layer (HTL) was formed by vacuum-depositing HT-1 under the same vacuum deposition condition. Then, a 300 Å-thick emission layer was formed by vacuum-depositing the compound 1 of Synthesis Example 21 and a second host, the compound C-10 obtained in Synthesis Example 5 under the same vacuum deposition condition. The compound 1 and the compound C-10 were used in a weight ratio of 4:1. When depositing the hosts, a phosphorescent dopant, acetylacetonatobis(2-phenylquinolinato)iridium (Ir(pq)2acac) was simultaneously deposited. Herein, 7 wt% of the phosphorescent dopant was deposited by adjusting a deposition rate of the phosphorescent dopant based on 100 wt% of the total amount of the emission layer.

On the emission layer, a 50 Å-thick hole blocking layer was formed by depositing bis(2-methyl-8-quinolinolate)-4-(phenylphenolato)aluminium (BAlq) under the same vacuum deposition condition. Subsequently, a 250 Å-thick electron transport layer (ETL) was formed by depositing tris(8-hydroxyquinolinato)aluminium (Alq3) under the same vacuum deposition condition. LiF and Al were sequentially deposited to form a cathode on the electron transport layer (ETL), manufacturing an organic light emitting diode.

The organic light emitting diode has a structure of ITO/ DNTPD (60 nm)/ HT-1 (30 nm)/ EML (compound1: C-10=4:1, 93 wt%) + Ir(pq)2acac (7 wt%), 30 nm)/ Balq (5 nm)/ Alq3 (25 nm)/ LiF (1 nm) / Al (100 nm).

### Example 9

An organic light emitting diode was manufactured according to the same method as Example 8 except for using the compounds 1 and C-10 in a ratio of 1:1.

### Example 10

An organic light emitting diode was manufactured according to the same method as Example 8 except for using the compounds 1 and C-10 in a ratio of 1:4.

### Example 11

An organic light emitting diode was manufactured according to the same method as Example 8 except for using the compound B-10 instead of the compound C-10.

### Example 12

An organic light emitting diode was manufactured according to the same method as Example 11 except for using the compounds 1 and B-10 in a ratio of 1:1.

### Example 13

An organic light emitting diode was manufactured according to the same method as Example 11 except for using the compounds 1 and B-10 in a ratio of 1:4.

### Example 14

An organic light emitting diode was manufactured according to the same method as Example 8 except for using the compound B-31 instead of the compound C-10.

### Example 15

An organic light emitting diode was manufactured according to the same method as Example 14 except for using the compounds 1 and B-31 in a ratio of 1:1.

### Example 16

An organic light emitting diode was manufactured according to the same method as Example 8 except for using the compound B-34 instead of the compound C-10.

### Example 17

An organic light emitting diode was manufactured according to the same method as Example 16 except for using the compounds 1 and B-34 in a ratio of 1:1.

### Example 18

An organic light emitting diode was manufactured according to the same method as Example 8 except for using the compound B-43 instead of the compound C-10.

### Example 19

An organic light emitting diode was manufactured according to the same method as Example 8 except for using the compound E-1 instead of the compound C-10.

### Example 20

An organic light emitting diode was manufactured according to the same method as Example 8 except for using the compound 1 as a single host instead of the compound C-10.

### Comparative Example 2

An organic light emitting diode was manufactured according to the same method as Example 8 except for using CBP as a host instead of two hosts of the compounds 1 and C-10.

### Comparative Example 3

An organic light emitting diode was manufactured according to the same method as Example 8 except for using the compound C-10 as a single host instead of two hosts of the compounds 1 and C-10.

### Comparative Example 4

An organic light emitting diode was manufactured according to the same method as Example 8 except for using the compound B-10 as a single host instead of two hosts of the compounds 1 and C-10.

### Comparative Example 5

An organic light emitting diode was manufactured according to the same method as Example 8 except for using the compound B-31 as a single host instead of two hosts of the compounds 1 and C-10.

### Comparative Example 6

An organic light emitting diode was manufactured according to the same method as Example 8 except for using the compound B-34 as a single host instead of two hosts of the compounds 1 and C-10.

### Comparative Example 7

An organic light emitting diode was manufactured according to the same method as Example 8 except for using the compound B-43 as a single host instead of two hosts of the compounds 1 and C-10.

### Comparative Example 8

An organic light emitting diode was manufactured according to the same method as Example 8 except for using the compound E-1 as a single host instead of two hosts of the compounds 1 and C-10.

### Evaluation

Current density and luminance changes depending on a voltage and luminous efficiency of each organic light emitting diode according to Examples 8 to 20 and Comparative Examples 2 to 8 were measured.

The measurements were specifically performed in the following method, and the results were provided in the following Table 2.
(1) Measurement of Current Density Change Depending on Voltage Change
   Current values flowing in the unit device of the manufactured organic light emitting diodes were measured for, while increasing the voltage from 0V to 10V using a current-voltage meter (Keithley 2400), and the measured current values were divided by an area to provide the results.
(2) Measurement of Luminance Change Depending on Voltage Change
   Luminance of the manufactured organic light emitting diodes was measured for luminance, while increasing the voltage from 0V to 10V using a luminance meter (Minolta Cs-1000A).
(3) Measurement of Luminous Efficiency
   Current efficiency (cd/A) at the same current density (10 mA/cm²) were calculated by using the luminance, current density, and voltages (V) from the items (1) and (2).
(4) Measurement of Life-span
   Life-span of the organic light emitting diodes was obtained by emitting light with 3000 cd/m2 in the initial luminance (cd/m²) and measuring time taken until luminance decreased by 50 % relative to the initial luminance as time goes.

**[Table 2]**

| | First host | Second host | First host: second host | Efficiency (cd/A) | 50% life-span (h) at 3000 cd/m² |
|---|---|---|---|---|---|
| Example8 | compound 1 | C-10 | 4:1 | 49.5 | 480 |
| Example9 | compound 1 | C-10 | 1:1 | 50.0 | 510 |
| Example10 | compound 1 | C-10 | 1:4 | 43.6 | 470 |
| Example11 | compound 1 | B-10 | 4:1 | 48.4 | 280 |
| Example12 | compound 1 | B-10 | 1:1 | 48.1 | 330 |
| Example13 | compound 1 | B-10 | 1:4 | 45.6 | 250 |
| Example14 | compound 1 | B-31 | 4:1 | 48.2 | 285 |
| Example15 | compound 1 | B-31 | 1:1 | 49.2 | 310 |
| Example16 | compound 1 | B-34 | 4:1 | 47.5 | 465 |
| Example17 | compound 1 | B-34 | 1:1 | 48.7 | 500 |
| Example18 | compound 1 | B-43 | 4:1 | 49.0 | 300 |
| Example19 | compound 1 | E-1 | 4:1 | 49.1 | 320 |
| Example20 | compound 1 | - | - | 48.4 | 250 |
| Comparative Example2 | CBP | | - | 37.2 | 220 |
| Comparative Example3 | C-10 | | - | 10 | 30 |
| Comparative Example4 | B-10 | | - | 10 | 30 |
| Comparative Example5 | B-31 | | - | 5 | 0 |
| Comparative Example6 | B-34 | | - | 20.1 | 10 |
| Comparative Example7 | B-43 | | - | 10 | 30 |
| Comparative Example8 | E-1 | | - | 5 | 50 |

Referring to Table 2, the organic light emitting diodes according to Examples 8 to 20 showed remarkably improved luminous efficiency and life-span characteristics compared with the organic light emitting diodes according to Comparative Examples 2 to 8.

### <Description of Symbols>

100, 200: organic light emitting diode
105: organic layer
110: cathode
120: anode
130: emission layer
140: hole auxiliary layer

## Claims

1. An organic compound represented by the following Chemical Formula 1: 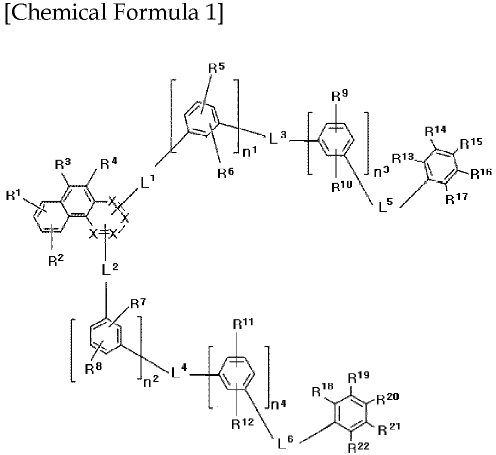 wherein, in the Chemical Formula 1,
two of X's are N and two of X's are C,
R¹ to R⁴ are independently hydrogen, deuterium, unsubstituted C1 to C10 alkyl group, unsubstituted C6 to C12 aryl group, unsubstituted C3 to C12 heterocycle, or a combination thereof,
R⁵ to R¹² are independently hydrogen, deuterium, unsubstituted C1 to C10 alkyl group, unsubstituted C6 to C12 aryl group, or a combination thereof,
R¹³ to R²² are independently hydrogen, deuterium, unsubstituted C1 to C10 alkyl group, unsubstituted C6 to C12 aryl group, unsubstituted C3 to C12 heterocycle, or a combination thereof,
R¹³ to R²² are independently present or adjacent two are linked to each other to form a fused ring,
L¹ to L⁶ are independently a single bond, unsubstituted phenylene group, unsubstituted biphenylene group, unsubstituted terphenylene group or unsubstituted quaterphenylene group,
n¹ to n⁴ are independently integers ranging from 0 to 5, and
the sum of n¹ to n⁴ is an integer of 2 or more.

2. The organic compound of claim 1, which is represented by the following Chemical Formula 2 or 3: 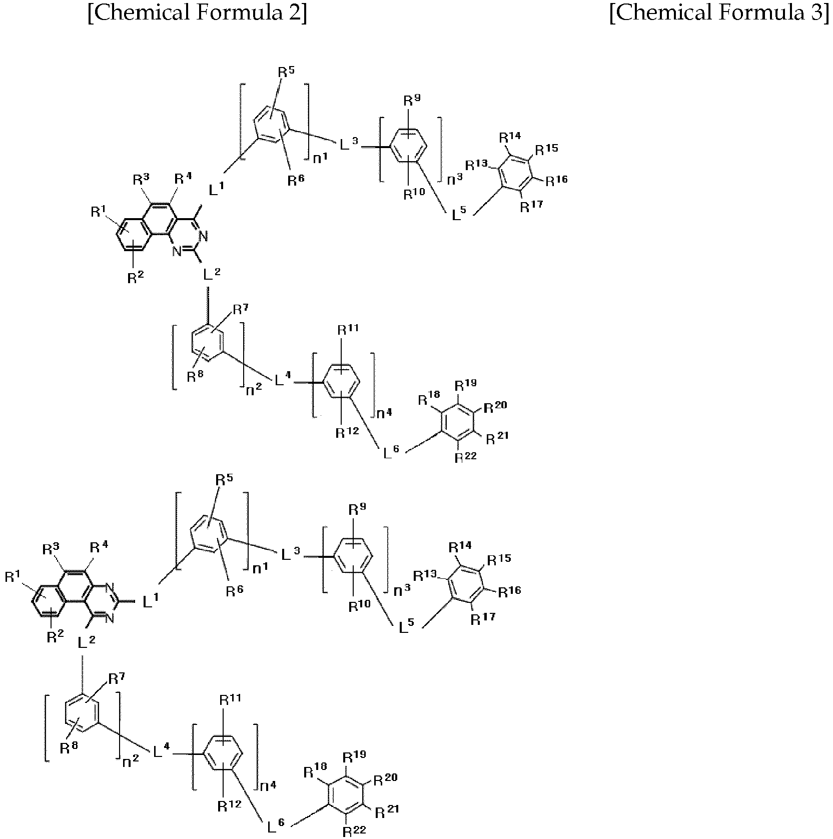 wherein, in the Chemical Formula 2 or 3,
R¹ to R⁴ are independently hydrogen, deuterium, unsubstituted C1 to C10 alkyl group, unsubstituted C6 to C12 aryl group, unsubstituted C3 to C12 heterocycle, or a combination thereof,
R⁵ to R¹² are independently hydrogen, deuterium, unsubstituted C1 to C10 alkyl group, unsubstituted C6 to C12 aryl group, or a combination thereof,
R¹³ to R²² are independently hydrogen, deuterium, unsubstituted C1 to C10 alkyl group, unsubstituted C6 to C12 aryl group, unsubstituted C3 to C12 heterocycle, or a combination thereof,
R¹³ to R²² are independently present or adjacent two are linked to each other to form a fused ring,
L¹ to L⁶ are independently a single bond, unsubstituted phenylene group, unsubstituted biphenylene group, unsubstituted terphenylene group or unsubstituted quaterphenylene group,
n¹ to n⁴ are independently integers ranging from 0 to 5, and
the sum of n¹ to n⁴ is an integer of 2 or more.

3. The organic compound of claim 2, which is represented by the following Chemical Formula 2A or 3A: 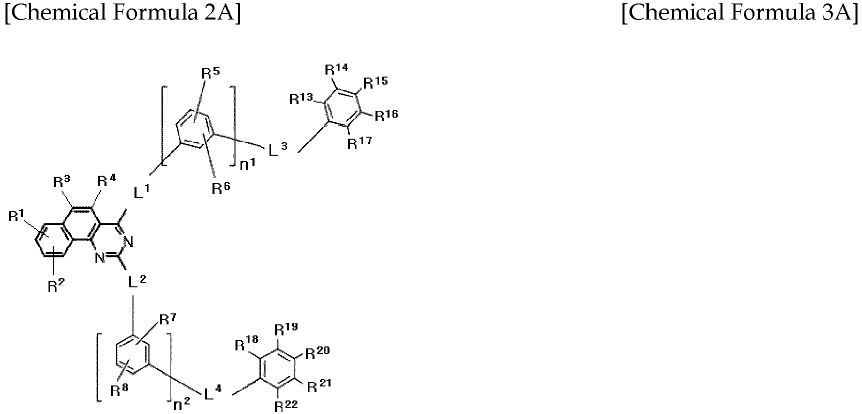 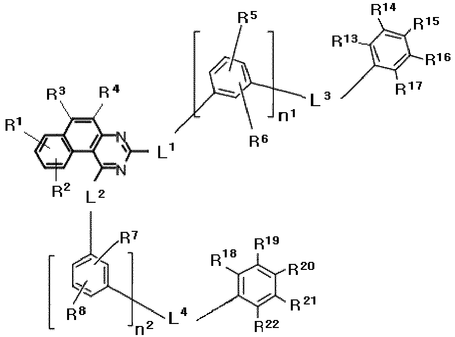 wherein, in the Chemical Formula 2A or 3A,
R¹ to R⁴ are independently hydrogen, deuterium, unsubstituted C1 to C10 alkyl group, unsubstituted C6 to C12 aryl group, unsubstituted C3 to C12 heterocycle, or a combination thereof,
R⁵ to R⁸ are independently hydrogen, deuterium, unsubstituted C1 to C10 alkyl group, unsubstituted C6 to C12 aryl group, or a combination thereof,
R¹³ to R²² are independently hydrogen, deuterium, unsubstituted C1 to C10 alkyl group, unsubstituted C6 to C12 aryl group, unsubstituted C3 to C12 heterocycle, or a combination thereof,
R¹³ to R²² are independently present or adjacent two are linked to each other to form a fused ring,
L¹ to L⁴ are independently a single bond, unsubstituted phenylene group, unsubstituted biphenylene group, unsubstituted terphenylene group or unsubstituted quaterphenylene group,
n¹ and n² are independently integers ranging from 0 to 5, and
the sum of n¹ and n² is an integer of 2 or more.

4. The organic compound of claim 1, wherein n¹ and n² of the Chemical Formula 1 are independently 1 to 5.

5. The organic compound of claim 1, wherein R³ and R⁴ of the Chemical Formula 1 are hydrogen.

6. The organic compound of claim 1, wherein L¹ to L⁶ of the Chemical Formula 1 are independently a single bond or groups listed in the following Group 1: 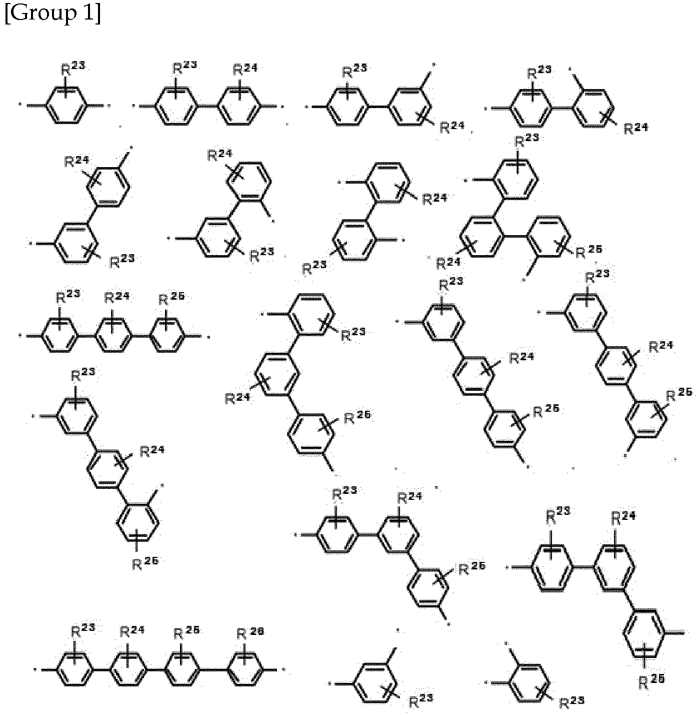 wherein, in the Group 1,
R²³ to R²⁶ are independently hydrogen, and
* is a linking point.

7. The organic compound of claim 6, wherein the R²³ to R²⁶ are hydrogen.

8. A composition for an organic optoelectric device, comprising the first organic compound of claim 1, and
at least one second organic compound,
wherein the second organic compound comprises at least one of a compound represented by the following Chemical Formula 4 and a compound consisting of a combination of a moiety represented by the following Chemical Formula 5 and a moiety represented by the following Chemical Formula 6: 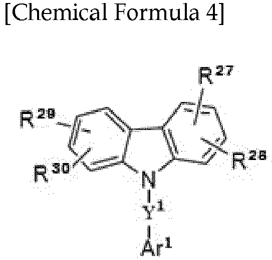 wherein, in the Chemical Formula 4,
Y¹ is a single bond, unsubstituted C1 to C20 alkylene group, unsubstituted C2 to C20 alkenylene group, unsubstituted C6 to C30 arylene group, unsubstituted C2 to C30 heterocycle, or a combination thereof,
Ar¹ is a substituted or unsubstituted C6 to C30 aryl group, a substituted or unsubstituted C2 to C30 heterocycle, or a combination thereof,
R²⁷ to R³⁰ are independently hydrogen, deuterium, unsubstituted C1 to C20 alkyl group, unsubstituted C6 to C50 aryl group, unsubstituted C2 to C50 heterocycle, or a combination thereof, and
at least one of R²⁷ to R³⁰ and Ar¹ includes a triphenylene group or a carbazole group, 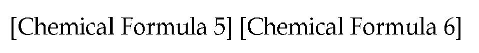 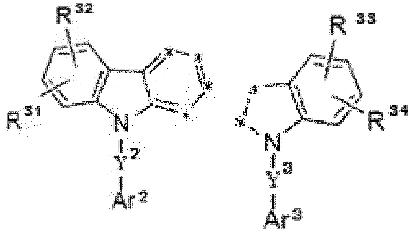
wherein, in the Chemical Formula 5 or 6,
Y² and Y³ are independently a single bond, unsubstituted C1 to C20 alkylene group, unsubstituted C2 to C20 alkenylene group, unsubstituted C6 to C30 arylene group, unsubstituted C2 to C30 heterocycle, or a combination thereof,
Ar² and Ar³ are independently substituted or unsubstituted C6 to C30 aryl group, a substituted or unsubstituted C2 to C30 heterocycle, or a combination thereof,
R³¹ to R³⁴ are independently hydrogen, deuterium, unsubstituted C1 to C20 alkyl group, unsubstituted C6 to C50 aryl group, unsubstituted C2 to C50 heterocycle, or a combination thereof,
adjacent two *'s of the Chemical Formula 5 are combined with the two *'s of the Chemical Formula 6 to form a fused ring, and * that does not form a fused ring in the Chemical Formula 5 is are independently CRa, and
R^{a} is hydrogen, deuterium, unsubstituted C1 to C10 alkyl group, unsubstituted C6 to C12 aryl group, unsubstituted C3 to C12 heterocyclic or a combination thereof,
herein, the term "substituted" of Ar¹ to Ar³ refers to one substituted with a C6 to C30 aryl group or a C6 to C30 heterocyclic group, instead of at least one hydrogen of a substituent.

9. The composition for an organic optoelectric device of claim 8, wherein the compound represented by the Chemical Formula 4 is represented by one of the following Chemical Formulae 4-I to 4-III: 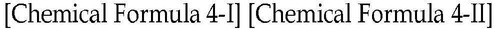 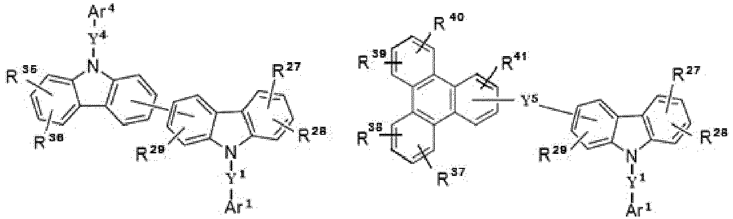 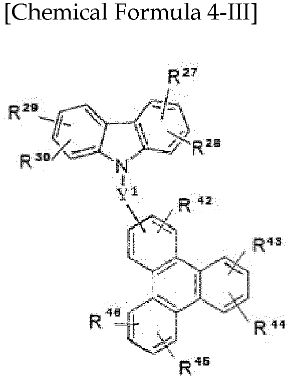 wherein, in Chemical Formulae 4-1 to 4-III,
Y¹, Y⁴ and Y⁵ are independently a single bond, unsubstituted C1 to C20 alkylene group, unsubstituted C2 to C20 alkenylene group, unsubstituted C6 to C30 arylene group, unsubstituted C2 to C30 heterocycle, or a combination thereof,
Ar¹ and Ar⁴ are independently substituted or unsubstituted C6 to C30 aryl group, a substituted or unsubstituted C2 to C30 heterocycle, or a combination thereof, and
R²⁷ to R³⁰ and R³⁵ to R⁴⁶ are independently hydrogen, deuterium, unsubstituted C1 to C20 alkyl group, unsubstituted C6 to C50 aryl group, unsubstituted C2 to C50 heterocyclic or a combination thereof,
herein, the term "substituted" of Ar¹ and Ar⁴ refers to one substituted with a C6 to C30 aryl group or a C6 to C30 heterocyclic group, instead of at least one hydrogen of a substituent.

10. The composition for an organic optoelectric device of claim 8, wherein the first organic compound and the second organic compound are included in a weight ratio of about 1:10 to about 10:1.

11. The composition for an organic optoelectric device of claim 8, which further comprises a phosphorescent dopant.

12. An organic optoelectric device, comprising
an anode and a cathode facing each other, and
at least one organic layer interposed between the anode and the cathode,
wherein the organic layer comprises the organic compound of any one of claims 1 to 7 or the composition of any one of claims 8 to 11.

13. The organic optoelectric device of claim 12, wherein
the organic layer comprises an emission layer, and
the emission layer comprises the organic compound or the composition, preferably . as a host of the emission layer.

14. A display device comprising the organic optoelectric device of claim 12or 13.

## Patentansprüche

1. Organische Verbindung, die durch die folgende chemische Formel 1 dargestellt ist: 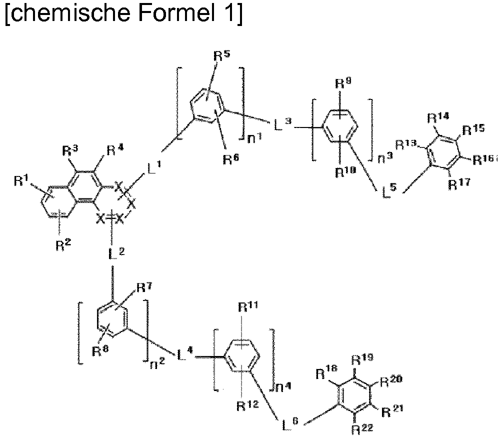 wobei in der chemischen Formel 1
zwei der Komponenten X N und zwei der Komponenten X C sind,
R¹ bis R⁴ unabhängig voneinander Wasserstoff, Deuterium, eine unsubstituierte C1- bis C10-Alkylgruppe, eine unsubstituierte C6- bis C12-Arylgruppe, ein unsubstituierter C3- bis C12-Heterocyclus oder eine Kombination davon sind.
R⁵ bis R¹² unabhängig voneinander Wasserstoff, Deuterium, eine unsubstituierte C1- bis C10-Alkylgruppe, eine unsubstituierte C6- bis C12-Arylgruppe oder eine Kombination davon sind,
R¹³ bis R²² unabhängig voneinander Wasserstoff, Deuterium, eine unsubstituierte C1- bis C10-Alkylgruppe, eine unsubstituierte C6- bis C12-Arylgruppe, ein unsubstituierter C3- bis C12-Heterocyclus oder eine Kombination davon sind,
R¹³ bis R²² unabhängig voneinander einen kondensierten Ring darstellen oder zwei benachbarte dieser Komponenten miteinander verbunden sind, um einen kondensierten Ring zu bilden,
L¹ bis L⁶ unabhängig voneinander eine Einfachbindung, eine unsubstituierte Phenylengruppe, eine unsubstituierte Biphenylengruppe, eine unsubstituierte Terphenylengruppe oder eine unsubstituierte Quaterphenylengruppe sind,
n¹ bis n⁴ unabhängig ganze Zahlen im Bereich von 0 bis 5 sind, und die Summe von n¹ bis n⁴ eine ganze Zahl von 2 oder mehr ist.

2. Organische Verbindung nach Anspruch 1, die durch die folgende chemische Formel 2 oder 3 dargestellt ist: 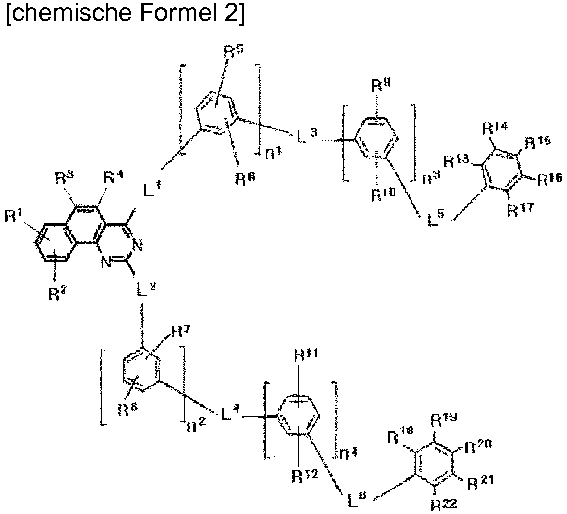 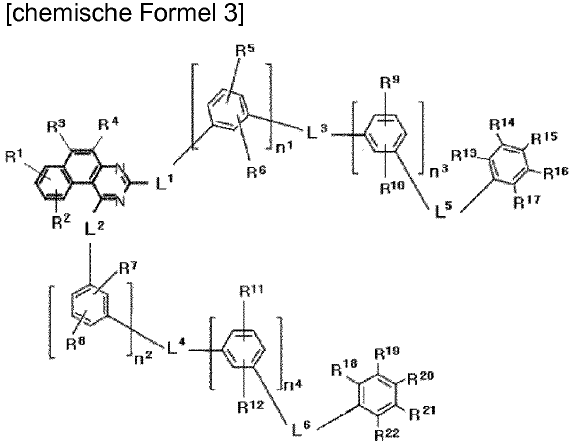 wobei in der chemischen Formel 2 oder 3
R¹ bis R⁴ unabhängig voneinander Wasserstoff, Deuterium, eine unsubstituierte C1- bis C10-Alkylgruppe, eine unsubstituierte C6- bis C12-Arylgruppe, ein unsubstituierter C3- bis C12-Heterocyclus oder eine Kombination davon sind,
R⁵ bis R¹² unabhängig voneinander Wasserstoff, Deuterium, eine unsubstituierte C1- bis C10-Alkylgruppe, eine unsubstituierte C6- bis C12-Arylgruppe oder eine Kombination davon sind,
R¹³ bis R²² unabhängig voneinander Wasserstoff, Deuterium, eine unsubstituierte C1- bis C10-Alkylgruppe, eine unsubstituierte C6- bis C12-Arylgruppe, ein unsubstituierter C3- bis C12-Heterocyclus oder eine Kombination davon sind,
R¹³ bis R²² unabhängig voneinander einen kondensierten Ring darstellen oder zwei benachbarte dieser Komponenten verbunden sind, um einen kondensierten Ring zu bilden,
L¹ bis L⁶ unabhängig voneinander eine Einfachbindung, eine unsubstituierte Phenylengruppe, eine unsubstituierte Biphenylengruppe, eine unsubstituierte Terphenylengruppe oder eine unsubstituierte Quaterphenylengruppe sind,
n¹ bis n⁴ unabhängig ganze Zahlen im Bereich von 0 bis 5 sind, und die Summe von n¹ bis n⁴ eine ganze Zahl von 2 oder mehr ist.

3. Organische Verbindung nach Anspruch 2, die durch die folgende chemische Formel 2A oder 3A dargestellt ist: 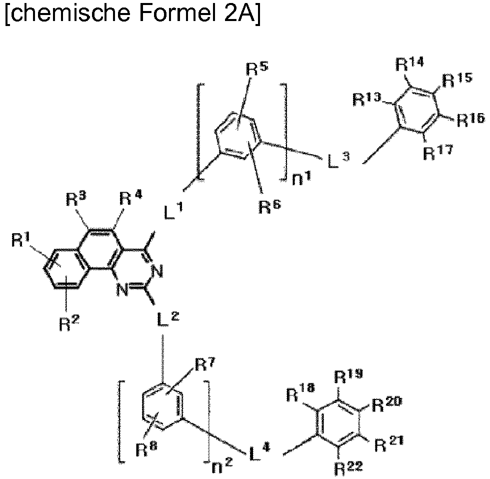 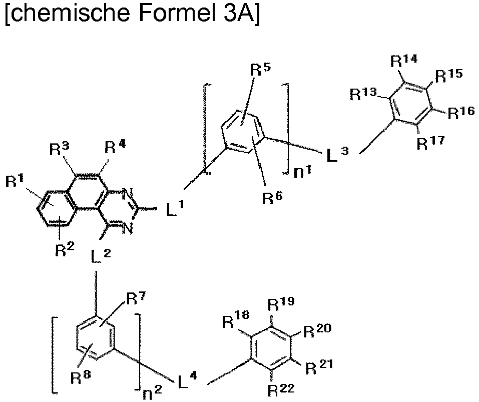 wobei in der chemischen Formel 2A oder 3A
R¹ bis R⁴ unabhängig voneinander Wasserstoff, Deuterium, eine unsubstituierte C1- bis C10-Alkylgruppe, eine unsubstituierte C6- bis C12-Arylgruppe, ein unsubstituierter C3- bis C12-Heterocyclus oder eine Kombination davon sind.
R⁵ bis R⁸ unabhängig voneinander Wasserstoff, Deuterium, eine unsubstituierte C1- bis C10-Alkylgruppe, eine unsubstituierte C6- bis C12-Arylgruppe oder eine Kombination davon sind,
R¹³ bis R²² unabhängig voneinander Wasserstoff, Deuterium, eine unsubstituierte C1- bis C10-Alkylgruppe, eine unsubstituierte C6- bis C12-Arylgruppe, ein unsubstituierter C3- bis C12-Heterocyclus oder eine Kombination davon sind,
R¹³ bis R²² unabhängig voneinander einen kondensierten Ring darstellen oder zwei benachbarte dieser Komponenten verbunden sind, um einen kondensierten Ring zu bilden,
L¹ bis L⁴ unabhängig voneinander eine Einfachbindung, eine unsubstituierte Phenylengruppe, eine unsubstituierte Biphenylengruppe, eine unsubstituierte Terphenylengruppe oder eine unsubstituierte Quaterphenylengruppe sind,
n¹ und n² unabhängig ganze Zahlen im Bereich von 0 bis 5 sind, und die Summe von n¹ und n² eine ganze Zahl von 2 oder mehr ist.

4. Organische Verbindung nach Anspruch 1, wobei n¹ und n² der chemischen Formel 1 unabhängig voneinander 1 bis 5 betragen.

5. Organische Verbindung nach Anspruch 1, wobei R³ und R⁴ der chemischen Formel 1 Wasserstoff sind.

6. Organische Verbindung nach Anspruch 1, wobei L¹ bis L⁶ der chemischen Formel 1 unabhängig voneinander eine Einfachbindung oder Gruppen sind, die in der folgenden Gruppe 1 aufgeführt sind: 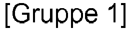 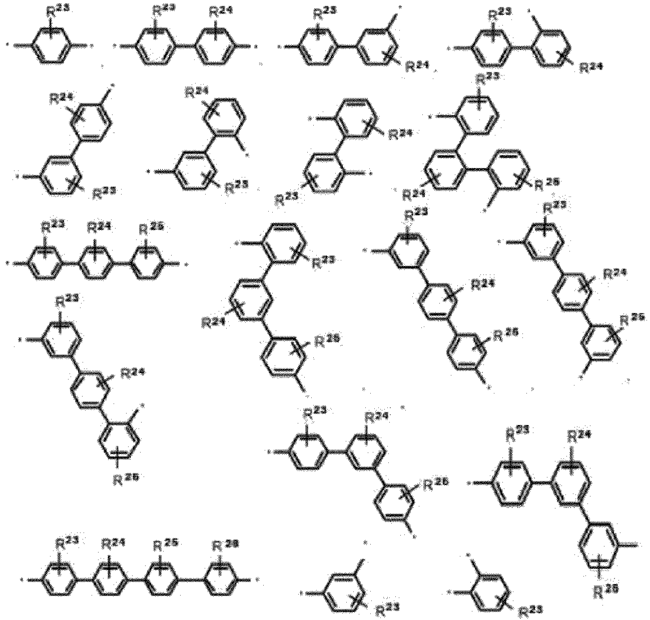 wobei in der Gruppe 1
R²³ bis R²⁶ unabhängig voneinander Wasserstoff sind, und
* eine Bindungsstelle ist.

7. Organische Verbindung nach Anspruch 6, wobei R²³ bis R²⁶ Wasserstoff sind.

8. Zusammensetzung für ein organisches optoelektrisches Bauelement, mit:
der ersten organischen Verbindung nach Anspruch 1; und
mindestens einer zweiten organischen Verbindung,
wobei die zweite organische Verbindung mindestens eine Verbindung unter einer durch die folgende chemische Formel 4 dargestellten Verbindung und einer Verbindung aufweist, die aus einer Kombination aus einer durch die folgende chemische Formel 5 dargestellten Einheit und einer durch die folgende chemische Formel 6 dargestellten Einheit besteht: 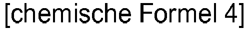 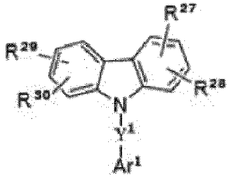
wobei in der chemischen Formel 4
Y¹ eine Einfachbindung, eine unsubstituierte C1- bis C20-Alkylengruppe, eine unsubstituierte C2- bis C20-Alkenylengruppe, eine unsubstituierte C6- bis C30-Arylengruppe, ein unsubstituierter C2- bis C30-Heterocyclus oder eine Kombination davon ist,
Ar¹ eine substituierte oder unsubstituierte C6- bis C30-Arylgruppe, ein substituierter oder unsubstituierter C2- bis C30-Heterocyclus oder eine Kombination davon ist,
R²⁷ bis R³⁰ unabhängig voneinander Wasserstoff, Deuterium, eine unsubstituierte C1- bis C20-Alkylgruppe, eine unsubstituierte C6- bis C50-Arylgruppe, ein unsubstituierter C2- bis C50-Heterocyclus oder eine Kombination davon sind, und
mindestens eine der Komponenten R²⁷ bis R³⁰ und Ar¹ eine Triphenylengruppe oder eine Carbazolgruppe aufweist, 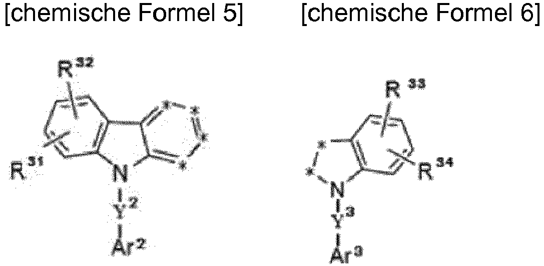
wobei in der chemischen Formel 5 oder 6
Y² und Y³ unabhängig voneinander eine Einfachbindung, eine unsubstituierte C1- bis C20-Alkylengruppe, eine unsubstituierte C2- bis C20-Alkenylengruppe, eine unsubstituierte C6- bis C30-Arylengruppe, ein unsubstituierter C2- bis C30-Heterocyclus oder eine Kombination davon sind,
Ar² und Ar³ unabhängig voneinander eine substituierte oder unsubstituierte C6- bis C30-Arylgruppe, ein substituierter oder unsubstituierter C2- bis C30-Heterocyclus oder eine Kombination davon sind,
R³¹ bis R³⁴ unabhängig voneinander Wasserstoff, Deuterium, eine unsubstituierte C1- bis C20-Alkylgruppe, eine unsubstituierte C6- bis C50-Arylgruppe, ein unsubstituierter C2- bis C50-Heterocyclus oder eine Kombination davon sind,
zwei benachbarte Komponenten * der chemischen Formel 5 mit den zwei Komponenten * der chemischen Formel 6 kombiniert sind, um einen kondensierten Ring zu bilden, und die Komponenten *, die in der chemischen Formel 5 keinen kondensierten Ring bilden, unabhängig voneinander CR^{a} sind, und
R^{a} Wasserstoff, Deuterium, eine unsubstituierte C1- bis C10-Alkylgruppe, eine unsubstituierte C6- bis C12-Arylgruppe, eine unsubstituierte C3- bis C12-Heterocyclusgruppe oder eine Kombination davon ist,
wobei der hierin verwendete Ausdruck "substituiert" von Ar¹ bis Ar³ bedeutet: substituiert mit einer C6- bis C30-Arylgruppe oder mit einer C6-bis C30-Heterocyklusgruppe an Stelle von mindestens einem Wasserstoff als Substituent.

9. Zusammensetzung nach Anspruch 8, wobei die durch die chemische Formel 4 dargestellte Verbindung durch eine der folgenden chemischen Formeln 4-I bis 4-III dargestellt ist: 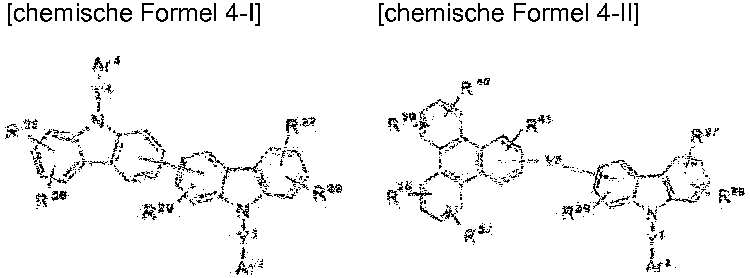 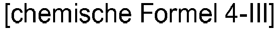 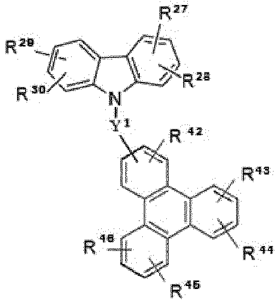 wobei in den chemischen Formeln 4-I bis 4-III
Y¹, Y⁴ und Y⁵ unabhängig voneinander eine Einfachbindung, eine unsubstituierte C1- bis C20-Alkylengruppe, eine unsubstituierte C2- bis C20-Alkenylengruppe, eine unsubstituierte C6- bis C30-Arylengruppe, ein unsubstituierter C2- bis C30-Heterocyclus, oder eine Kombination davon sind,
Ar¹ und Ar⁴ unabhängig voneinander eine substituierte oder unsubstituierte C6- bis C30-Arylgruppe, ein substituierter oder unsubstituierter C2- bis C30-Heterocyclus oder eine Kombination davon sind, und
R²⁷ bis R³⁰ und R³⁵ bis R⁴⁶ unabhängig voneinander Wasserstoff, Deuterium, eine unsubstituierte C1- bis C20-Alkylgruppe, eine unsubstituierte C6- bis C50-Arylgruppe, ein unsubstituierter C2- bis C50-Heterocyclus oder eine Kombination davon sind,
wobei der hierin verwendete Ausdruck "substituiert" von Ar¹ bis Ar⁴ bedeutet: substituiert mit einer C6- bis C30-Arylgruppe oder mit einer C6-bis C30-Heterocyklusgruppe an Stelle von mindestens einem Wasserstoff als Substituent.

10. Zusammensetzung nach Anspruch 8, wobei die erste organische Verbindung und die zweite organische Verbindung in einem Gewichtsverhältnis von etwa 1:10 bis etwa 10:1 enthalten sind.

11. Zusammensetzung nach Anspruch 8, ferner mit einem phosphoreszierenden Dotierstoff.

12. Organisches optoelektrisches Bauelement, mit
einer Anode und einer Kathode, die einander zugewandt sind; und mindestens einer zwischen der Anode und der Kathode angeordneten organischen Schicht,
wobei die organische Schicht die organische Verbindung nach einem der Ansprüche 1 bis 7 oder die Zusammensetzung nach einem der Ansprüche 8 bis 11 aufweist.

13. Organisches optoelektrisches Bauelement nach Anspruch 12, wobei
die organische Schicht eine Emissionsschicht aufweist, und
die Emissionsschicht die organische Verbindung oder die Zusammensetzung vorzugsweise als ein Wirtsmaterial der Emissionsschicht aufweist.

14. Anzeigevorrichtung mit dem organischen optoelektrischen Bauelement nach Anspruch 12 oder 13.

## Revendications

1. Composé organique représenté par la Formule Chimique 1 suivante : 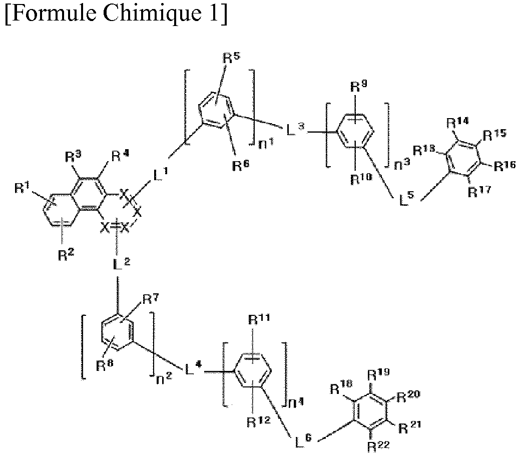 où, dans la Formule Chimique 1,
deux des Xs sont N et deux des Xs sont C,
R¹ à R⁴ sont indépendamment hydrogène, deutérium, groupement alkyle non-substitué en C1 à C10, groupement aryle non-substitué en C6 à C12, hétérocycle non-substitué en C3 à C12, ou une combinaison de ceux-ci,
R⁵ à R¹² sont indépendamment hydrogène, deutérium, groupement alkyle non-substitué en C1 à C10, groupement aryle non-substitué en C6 à C12, ou une combinaison de ceux-ci,
R¹³ à R²² sont indépendamment hydrogène, deutérium, groupement alkyle non-substitué en C1 à C10, groupement aryle non-substitué en C6 à C12, hétérocycle non-substitué en C3 à C12, ou une combinaison de ceux-ci,
R¹³ à R²² sont présents indépendamment ou reliés par paire adjacente pour former un cycle fusionné,
L¹ à L⁶ sont indépendamment une simple liaison, un groupement phénylène non-substitué, un groupement biphénylène non-substitué, un groupement terphénylène non-substitué ou un groupement quaterphénylène non-substitué,
n¹ à n⁴ sont des nombres entiers allant de 0 à 5, et
la somme de n¹ à n⁴ est un nombre entier supérieur ou égal à 2.

2. Composé organique selon la revendication 1, représenté par la Formule Chimique 2 ou 3 : 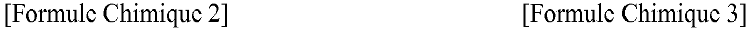 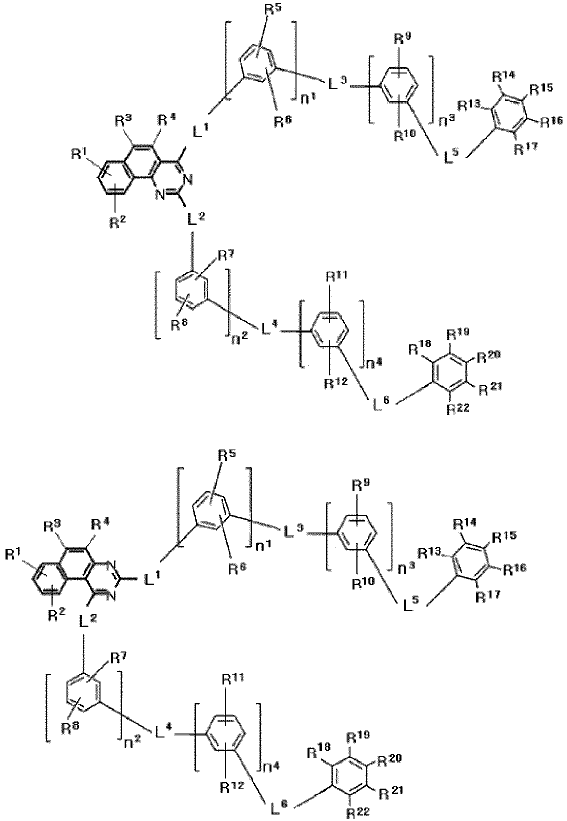 où, dans les Formules Chimique 2 et 3,
R¹ à R⁴ sont indépendamment hydrogène, deutérium, groupement alkyle non-substitué en C1 à C10, groupement aryle non-substitué en C6 à C12, hétérocycle non-substitué en C3 à C12, ou une combinaison de ceux-ci,
R⁵ à R¹² sont indépendamment hydrogène, deutérium, groupement alkyle non-substitué en C1 à C10, groupement aryle non-substitué en C6 à C12, ou une combinaison de ceux-ci,
R¹³ à R²² sont indépendamment hydrogène, deutérium, groupement alkyle non-substitué en C1 à C10, groupement aryle non-substitué en C6 à C12, hétérocycle non-substitué en C3 à C12, ou une combinaison de ceux-ci,
R¹³ à R²² sont présents indépendamment ou reliés par paire adjacente pour former un cycle fusionné,
L¹ à L⁶ sont indépendamment une simple liaison, un groupement phénylène non-substitué, un groupement biphénylène non-substitué, un groupement terphénylène non-substitué ou un groupement quaterphénylène non-substitué,
n¹ à n⁴ sont des nombres entiers allant de 0 à 5, et
la somme de n¹ à n⁴ est un nombre entier supérieur ou égal à 2.

3. Composé organique selon la revendication 1, représenté par les Formules Chimiques 2A ou 3A suivantes : 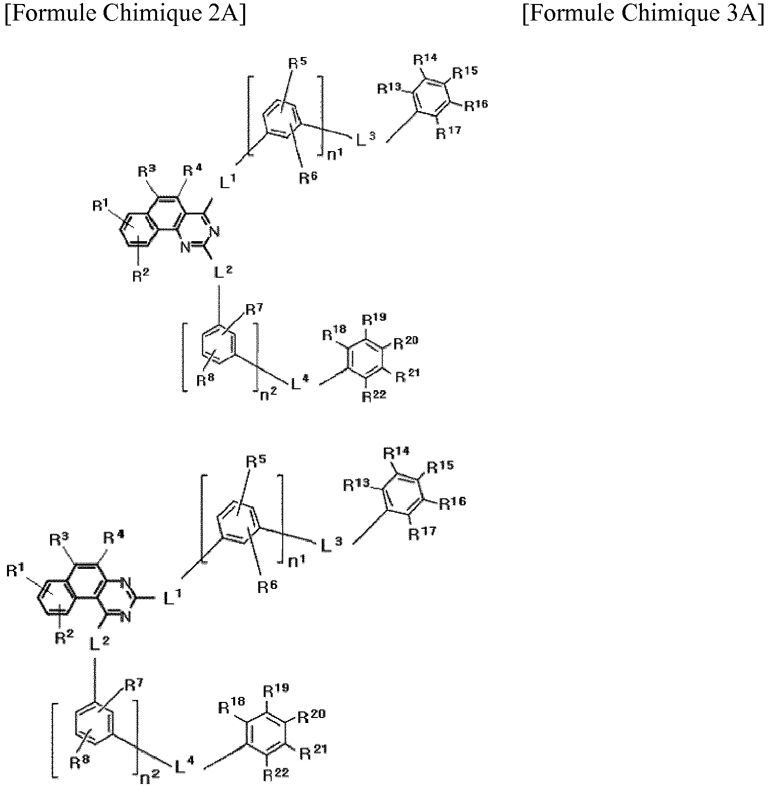 où, dans les Formules Chimique 2A et 3A,
R¹ à R⁴ sont indépendamment hydrogène, deutérium, groupement alkyle non-substitué en C1 à C10, groupement aryle non-substitué en C6 à C12, hétérocycle non-substitué en C3 à C12, ou une combinaison de ceux-ci,
R⁵ à R⁸ sont indépendamment hydrogène, deutérium, groupement alkyle non-substitué en C1 à C10, groupement aryle non-substitué en C6 à C12, ou une combinaison de ceux-ci,
R¹³ à R²² sont indépendamment hydrogène, deutérium, groupement alkyle non-substitué en C1 à C10, groupement aryle non-substitué en C6 à C12, hétérocycle non-substitué en C3 à C12, ou une combinaison de ceux-ci,
R¹³ à R²² sont présents indépendamment ou reliés par paire adjacente pour former un cycle fusionné,
L¹ à L⁴ sont indépendamment une simple liaison, un groupement phénylène non-substitué, un groupement biphénylène non-substitué, un groupement terphénylène non-substitué ou un groupement quaterphénylène non-substitué,
n¹ et n² sont des nombres entiers allant de 0 à 5, et
la somme de n¹ à n² est un nombre entier supérieur ou égal à 2.

4. Composé organique selon la revendication 1, dans lequel n¹ et n² de la Formule Chimique 1 sont indépendamment 1 à 5.

5. Composé organique selon la revendication 1, dans lequel R³ et R⁴ de la Formule Chimique 1 sont hydrogène.

6. Composé organique selon la revendication 1, dans lequel L¹ et L⁶ de la Formule Chimique 1 sont indépendamment une liaison simple ou des groupements listés dans le Groupe 1 suivant : 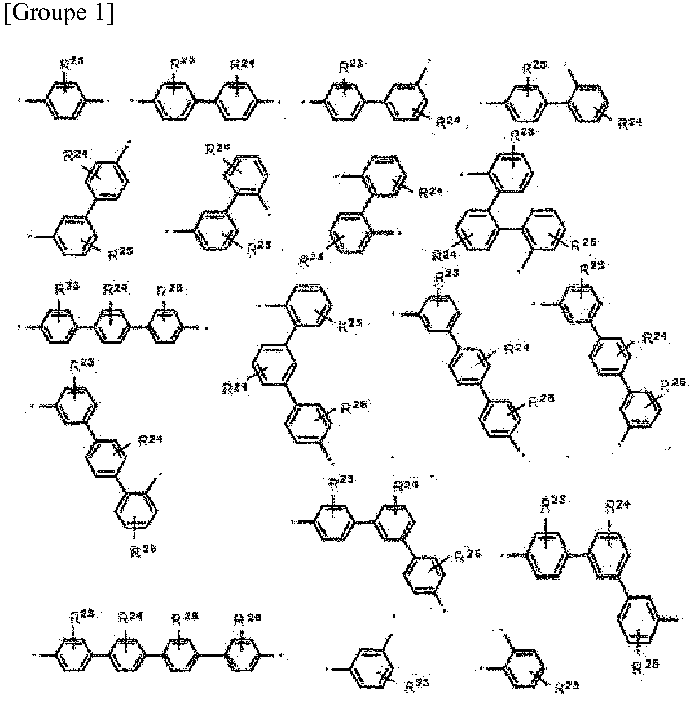 où, dans le Groupe 1,
R²³ à R²⁶ sont indépendamment hydrogène, et
* est un point de liaison

7. Composé organique selon la revendication 6, dans lequel les R²³ à R²⁶ sont hydrogène.

8. Composition pour un dispositif optoélectrique organique, comprenant le premier composé organique de la revendication 1, et
au moins un deuxième composé organique,
dans lequel le deuxième composé organique comprend au moins un composé représenté par la Formule Chimique 4 suivante et un composé consistant en une combinaison d'un fragment représenté par la Formule Chimique 5 suivante et d'un fragment représenté par la Formule Chimique 6 suivante : 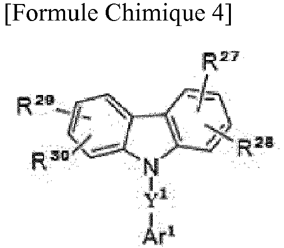 où, dans la Formule Chimique 4,
Y¹ est une liaison simple, un groupement alkylène non-substitué en C1 à C20, un groupement alcénylène non-substitué en C2 à C20, un groupement arylène non-substitué en C6 à C30, un hétérocycle non-substitué en C2 à C30, ou une combinaison de ceux-ci,
Ar¹ est un groupement aryle substitué ou non-substitué en C6 à C30, un hétérocycle substitué ou non-substitué en C2 à C30, ou une combinaison de ceux-ci,
R²⁷ à R³⁰ sont indépendamment hydrogène, deutérium, groupement alkyle non-substitué en C1 à C20, groupement aryle non-substitué en C6 à C50, hétérocycle non-substitué en C2 à C50, ou une combinaison de ceux-ci, et
au moins un de R²⁷ à R³⁰ et Ar¹ inclut un groupement triphénylène ou un groupement carbazole, 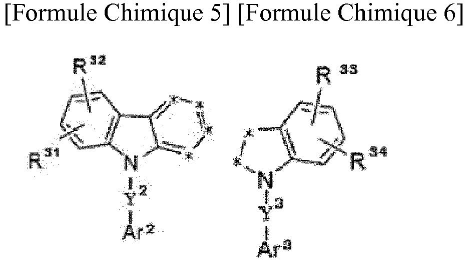
où, dans les Formules Chimiques 5 ou 6,
Y² et Y³ sont indépendamment une liaison simple, un groupement alkylène non-substitué en C1 à C20, un groupement alcénylène non-substitué en C2 à C20, un groupement arylène non-substitué en C6 à C30, un hétérocycle non-substitué en C2 à C30, ou une combinaison de ceux-ci,
Ar² et Ar³ sont indépendamment un groupement aryle substitué ou non-substitué en C6 à C30, un hétérocycle substitué ou non-substitué en C2 à C30, ou une combinaison de ceux-ci, et R³¹ à R³⁴ sont indépendamment hydrogène, deutérium, groupement alkyle non-substitué en C1 à C20, groupement aryle non-substitué en C6 à C50, hétérocycle non-substitué en C2 à C50, ou une combinaison de ceux-ci, et
deux *s adjacentes de la Formule 5 sont combinées avec les deux *s de la Formule 6 pour former un cycle fusionné, et *s qui ne forment pas un cycle fusionné dans la Formule 5 sont indépendamment CR^{a}, et
R^{a} est hydrogène, deutérium, groupement alkyle non-substitué en C1 à C10, groupement aryle non-substitué en C6 à C12, hétérocycle en C3 à C12 ou une combinaison de ceux-ci,
et où le terme « substitué » se réfère, pour Ar² et Ar³, à un fragment substitué avec un groupement aryle en C6 à C30 ou un groupement hétérocyclique en C6 à C30, au lieu d'un hydrogène d'un substituant.

9. Composition pour un dispositif optoélectrique organique selon la revendication 8, dans laquelle le composé représenté par la Formule Chimique 4 est représenté par une des Formules Chimiques 4-I à 4-III suivantes : 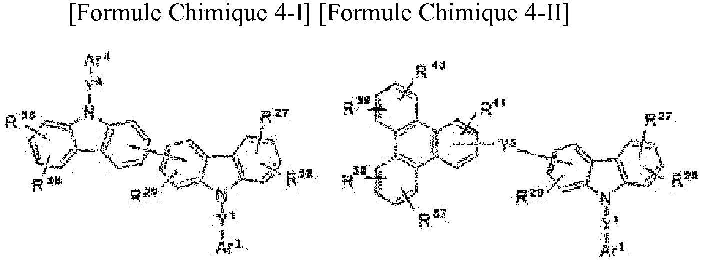 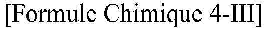 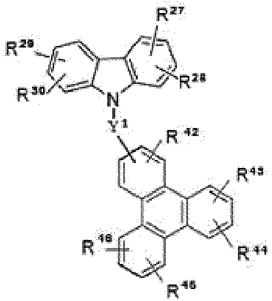 où, dans les Formules Chimiques 4-I à 4-III,
Y², Y⁴ et Y⁵ sont indépendamment une liaison simple, un groupement alkylène non-substitué en C1 à C20, un groupement alcénylène non-substitué en C2 à C20, un groupement arylène non-substitué en C6 à C30, un hétérocycle non-substitué en C2 à C30, ou une combinaison de ceux-ci,
Ar¹ et Ar⁴ sont indépendamment un groupement aryle substitué ou non-substitué en C6 à C30, un hétérocycle substitué ou non-substitué en C2 à C30, ou une combinaison de ceux-ci, et R²⁷ à R³⁰ et R³⁵ à R⁴⁶ sont indépendamment hydrogène, deutérium, groupement alkyle non-substitué en C1 à C20, groupement aryle non-substitué en C6 à C50, hétérocycle non-substitué en C2 à C50, ou une combinaison de ceux-ci, et
et où le terme « substitué » se réfère, pour Ar¹ et Ar⁴, à un fragment substitué avec un groupement aryle en C6 à C30 ou un groupement hétérocyclique en C6 à C30, au lieu d'un hydrogène d'un substituant.

10. Composition pour un dispositif optoélectrique organique selon la revendication 8, dans laquelle le premier composé organique et le deuxième composé organique sont inclus dans un ratio massique d'environ 1:10 à environ 10:1.

11. Composition pour un dispositif optoélectrique organique selon la revendication 8, comprenant en outre un agent dopant phosphorescent.

12. Dispositif optoélectrique organique, comprenant
une anode et une cathode se faisant face, et
au moins une couche organique interposée entre l'anode et la cathode,
dans laquelle la couche organique comprend le composé organique selon l'une quelconque des revendications 1 à 7 ou la composition selon l'une quelconque des revendications 8 à 11.

13. Dispositif optoélectrique organique selon la revendication 12, dans lequel la couche organique comprend une couche d'émission, et
la couche organique comprend le composé organique ou la composition, préférablement en tant qu'hôte de la couche d'émission.

14. Dispositif d'affichage comprenant le dispositif optoélectrique organique selon la revendication 12 ou la revendication 13.
